(19) Europäisches Patentamt
European Patent Office
Office européen des brevets

(11) EP 2 543 723 B1

(12) EUROPEAN PATENT SPECIFICATION

(45) Date of publication and mention of the grant of the patent:
**17.06.2015 Bulletin 2015/25**

(21) Application number: **11750425.8**

(22) Date of filing: **26.01.2011**

(51) Int Cl.:
***C12N 9/42*** *(2006.01)*
***C12N 15/09*** *(2006.01)*
***C12P 19/02*** *(2006.01)*
***C12N 1/19*** *(2006.01)*
***C12N 1/20*** *(2006.01)*

(86) International application number:
**PCT/JP2011/051406**

(87) International publication number:
**WO 2011/108312 (09.09.2011 Gazette 2011/36)**

(54) **METHOD FOR PRODUCING GLUCOSIDASE, ENZYME COMPOSITION, AND BIOMASS HYDROLYSIS METHOD**

VERFAHREN ZUR HERSTELLUNG VON GLUCOSIDASE UND ENZYMZUSAMMENSETZUNGEN SOWIE BIOMASSE-HYDROLYSIERUNGSVERFAHREN

MÉTHODE DE PRODUCTION DE GLUCOSIDASE, COMPOSITION ENZYMATIQUE ET MÉTHODE D'HYDROLYSE DE BIOMASSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.03.2010 JP 2010044242**

(43) Date of publication of application:
**09.01.2013 Bulletin 2013/02**

(73) Proprietor: **Toray Industries, Inc.**
**Tokyo, 103-8666 (JP)**

(72) Inventors:
- **KURIHARA, Hiroyuki**
**Kamakura-shi**
**Kanagawa 248-8555 (JP)**
- **WATANABE, Shiomi**
**Kamakura-shi**
**Kanagawa 248-8555 (JP)**
- **YAMADA, Katsushige**
**Kamakura-shi**
**Kanagawa 248-8555 (JP)**
- **ISHIKAWA, Kazuhiko**
**Higashihiroshima-shi**
**Hiroshima 739-0046 (JP)**
- **WADA, Yasunobu**
**Ikeda-shi**
**Osaka 563-8577 (JP)**
- **KADO, Yuji**
**Ikeda-shi**
**Osaka 563-8577 (JP)**

(74) Representative: **Kador & Partner**
**Corneliusstraße 15**
**80469 München (DE)**

(56) References cited:
**US-A1- 2010 015 662**

- **CLARK S E ET AL: "Effect of adding and removing N-glycosylation recognition sites on the thermostability of barley alpha-glucosidase", PROTEIN ENGINEERING, DESIGN AND SELECTION, OXFORD JOURNAL, LONDON, GB, vol. 17, no. 3, 1 March 2004 (2004-03-01), pages 245-249, XP008156901, ISSN: 1741-0126, DOI: 10.1093/PROTEIN/GZH028 [retrieved on 2004-03-29]**
- **WAKIYAMA M ET AL: "Purification and Properties of an Extracellular beta-Xylosidase from Aspergillus japonicus and Sequence Analysis of the Encoding Gene", JOURNAL OF BIOSCIENCE AND BIOENGINEERING, ELSEVIER, AMSTERDAM, NL, vol. 106, no. 4, 1 October 2008 (2008-10-01), pages 398-404, XP026149348, ISSN: 1389-1723, DOI: 10.1263/JBB.106.398 [retrieved on 2008-10-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- VOORHORST WG. ET AL.: 'Characterization of the celB gene coding for beta-glucosidase from the hyperthermophilic archaeon Pyrococcus furiosus and its expression and site-directed mutation in Escherichia coli.' J. BACTERIOL. vol. 177, no. 24, 1995, pages 7105 - 7111, XP002159355
- CLARK SE. ET AL.: 'Effect of adding and removing N-glycosylation recognition sites on the thermostability of barley alpha-glucosidase.' PROTEIN ENG. DES. SEL. vol. 17, no. 3, 2004, pages 245 - 249, XP008156901
- TAKASHIMA S. ET AL.: 'Molecular cloning and expression of the novel fungal beta-glucosidase genes from Humicola grisea and Trichoderma reesei.' J. BIOCHEM. vol. 125, no. 4, 1999, pages 728 - 736, XP002964031
- WATANABE T. ET AL.: 'Purification and properties of Aspergillus niger beta-glucosidase. ' EUR. J. BIOCHEM. vol. 209, no. 2, 1992, pages 651 - 659, XP008156900
- ORTEGA N. ET AL.: 'Optimisation of beta-glucosidase Entrapment in Alginate and Polyacrylamide Gels.' BIORESOURCE TECHNOLOGY vol. 64, 1998, pages 105 - 111, XP008157939

SEQUENCE LISTING

<110> Toray Industories, Inc. National Institute of Advanced Industrial Science and Technology

<120> Glycosylated glucosidase derived from thermophile, Production Method and Cellulase Composition

<130> PH-4632-PCT

<150> JP 2010-044242
<151> 2010-03-01

<160> 62

<170> PatentIn version 3.1

<210> 1
<211> 466
<212> PRT
<213> Trichoderma reesei

<400> 1

Met Leu Pro Lys Asp Phe Gln Trp Gly Phe Ala Thr Ala Ala Tyr Gln
1 5 10 15

Ile Glu Gly Ala Val Asp Gln Asp Gly Arg Gly Pro Ser Ile Trp Asp
20 25 30

Thr Phe Cys Ala Gln Pro Gly Lys Ile Ala Asp Gly Ser Ser Gly Val
35 40 45

Thr Ala Cys Asp Ser Tyr Asn Arg Thr Ala Glu Asp Ile Ala Leu Leu
50 55 60

Lys Ser Leu Gly Ala Lys Ser Tyr Arg Phe Ser Ile Ser Trp Ser Arg
65 70 75 80

Ile Ile Pro Glu Gly Gly Arg Gly Asp Ala Val Asn Gln Ala Gly Ile
85 90 95

Asp His Tyr Val Lys Phe Val Asp Asp Leu Leu Asp Ala Gly Ile Thr
100 105 110

Pro Phe Ile Thr Leu Phe His Trp Asp Leu Pro Glu Gly Leu His Gln
115 120 125

Arg Tyr Gly Gly Leu Leu Asn Arg Thr Glu Phe Pro Leu Asp Phe Glu
130 135 140

Asn Tyr Ala Arg Val Met Phe Arg Ala Leu Pro Lys Val Arg Asn Trp
145 150 155 160

Ile Thr Phe Asn Glu Pro Leu Cys Ser Ala Ile Pro Gly Tyr Gly Ser

165 170 175

Gly Thr Phe Ala Pro Gly Arg Gln Ser Thr Ser Glu Pro Trp Thr Val
180 185 190

Gly His Asn Ile Leu Val Ala His Gly Arg Ala Val Lys Ala Tyr Arg
195 200 205

Asp Asp Phe Lys Pro Ala Ser Gly Asp Gly Gln Ile Gly Ile Val Leu
210 215 220

Asn Gly Asp Phe Thr Tyr Pro Trp Asp Ala Ala Asp Pro Ala Asp Lys
225 230 235 240

Glu Ala Ala Glu Arg Arg Leu Glu Phe Phe Thr Ala Trp Phe Ala Asp
245 250 255

Pro Ile Tyr Leu Gly Asp Tyr Pro Ala Ser Met Arg Lys Gln Leu Gly
260 265 270

Asp Arg Leu Pro Thr Phe Thr Pro Glu Glu Arg Ala Leu Val His Gly
275 280 285

Ser Asn Asp Phe Tyr Gly Met Asn His Tyr Thr Ser Asn Tyr Ile Arg
290 295 300

His Arg Ser Ser Pro Ala Ser Ala Asp Asp Thr Val Gly Asn Val Asp
305 310 315 320

Val Leu Phe Thr Asn Lys Gln Gly Asn Cys Ile Gly Pro Glu Thr Gln
325 330 335

Ser Pro Trp Leu Arg Pro Cys Ala Ala Gly Phe Arg Asp Phe Leu Val
340 345 350

Trp Ile Ser Lys Arg Tyr Gly Tyr Pro Pro Ile Tyr Val Thr Glu Asn
355 360 365

Gly Thr Ser Ile Lys Gly Glu Ser Asp Leu Pro Lys Glu Lys Ile Leu
370 375 380

Glu Asp Asp Phe Arg Val Lys Tyr Tyr Asn Glu Tyr Ile Arg Ala Met
385 390 395 400

Val Thr Ala Val Glu Leu Asp Gly Val Asn Val Lys Gly Tyr Phe Ala
405 410 415

Trp Ser Leu Met Asp Asn Phe Glu Trp Ala Asp Gly Tyr Val Thr Arg
420 425 430

Phe Gly Val Thr Tyr Val Asp Tyr Glu Asn Gly Gln Lys Arg Phe Pro
435 440 445

Lys Lys Ser Ala Lys Ser Leu Lys Pro Leu Phe Asp Glu Leu Ile Ala
450 455 460

Ala Ala
465

<210> 2
<211> 434
<212> PRT
<213> Aspergillus niger

<400> 2

Met Leu Pro Lys Asp Leu Gln Trp Gly Phe Ala Lys Ala Ala Tyr Gln
1 5 10 15

Ile Glu Gly Ala Val Asp Gln Asp Gly Arg Gly Pro Ser Ile Trp Asp
20 25 30

Thr Phe Cys Ala Gln Pro Gly Lys Ile Ala Asp Gly Ser Ser Gly Val
35 40 45

Thr Ala Cys Asp Ser Tyr Asn Arg Thr Ala Glu Asp Ile Ala Leu Leu
50 55 60

Lys Ser Leu Gly Ala Lys Ser Tyr Arg Phe Ser Ile Ser Ser Arg Ile
65 70 75 80

Pro Glu Gly Gly Arg Gly Asp Ala Val Asn Gln Ala Gly Ile Asp His
85 90 95

Tyr Val Lys Phe Val Asp Asp Leu Leu Asp Ala Gly Ile Thr Pro Phe
100 105 110

Ile Thr Leu Phe His Trp Asp Leu Leu His Gln Arg Tyr Gly Gly Leu
115 120 125

Leu Asn Arg Thr Glu Phe Pro Leu Asp Phe Glu Asn Tyr Ala Arg Val
130 135 140

Met Phe Arg Ala Leu Pro Lys Val Arg Asn Trp Asn Glu Pro Leu Cys
145 150 155 160

Ser Ala Ile Pro Gly Tyr Gly Ser Gly Ser Phe Ala Pro Gly Arg Gln
165 170 175

Ser Thr Ser Glu Pro Trp Thr Val Gly His Asn Ile Leu Val Ala His
180 185 190

Gly Arg Ala Val Lys Ala Tyr Arg Asp Asp Phe Lys Pro Ala Ser Gly
195 200 205

Asp Gly Gln Ile Gly Ile Val Leu Asn Gly Asp Phe Thr Tyr Pro Trp
210 215 220

Asp Ala Ala Asp Pro Ala Asp Lys Glu Arg Leu Glu Phe Phe Thr Ala
225 230 235 240

Trp Phe Ala Asp Pro Ile Tyr Leu Gly Asp Tyr Pro Ala Ser Met Arg
245 250 255

Lys Gln Leu Gly Asp Arg Leu Pro Thr Phe Thr Pro Glu Glu Arg Ala
260 265 270

Leu Val His Gly Ser Asn Asp Phe Tyr Gly Met Asn His Tyr Thr Ser
275 280 285

Asn Tyr Ile Arg His Arg Ser Ser Pro Ala Ser Ala Asp Asp Thr Val
290 295 300

Gly Asn Val Asp Val Leu Phe Thr Asn Lys Gln Gly Asn Cys Ile Gly
305 310 315 320

Pro Glu Thr Gln Ser Pro Trp Leu Arg Pro Cys Ala Ala Gly Phe Arg
325 330 335

Asp Phe Leu Val Trp Thr Ser Lys Arg Tyr Gly Ser Pro Pro Ile Tyr
340 345 350

Val Thr Glu Asn Gly Thr Ser Ile Lys Gly Glu Ser Asp Leu Pro Asn
355 360 365

Glu Lys Ile Leu Glu Asp Asp Phe Arg Val Lys Tyr Tyr Asn Glu Tyr
370 375 380

Ile Arg Ala Met Val Thr Ala Val Glu Leu Asp Gly Val Asn Val Arg
385 390 395 400

Phe Gly Val Thr Tyr Val Asp Tyr Glu Asn Gly Gln Lys Arg Phe Pro
405 410 415

Lys Lys Ser Ala Lys Ser Leu Lys Pro Leu Phe Asp Glu Leu Ile Ala
420 425 430

Ala Ala

<210> 3
<211> 1419
<212> DNA
<213> Pyrococcus furiosus

<400> 3

```
atggcaaagt tcccaaaaaa cttcatgttt ggatattctt ggtctggttt ccagtttgag      60
atgggactgc caggaagtga agtggaaagc gactggtggg tgtgggttca cgacaaggag     120
aacatagcat caggtctagt aagtggagat ctaccagaga acggcccagc atattggcac     180
ctctataagc aagatcatga cattgcagaa aagctaggaa tggattgtat tagaggtggc     240
attgagtggg caagaatttt tccaaagcca acatttgacg ttaaagttga tgtggaaaag     300
gatgaagaag gcaacataat ttccgtagac gttccagaga gtacaataaa agagctagag     360
aaaattgcca acatggaggc ccttgaacat tatcgcaaga tttactcaga ctggaaggag     420
aggggcaaaa ccttcatatt aaacctctac cactggcctc ttccattatg gattcatgac     480
ccaattgcag taaggaaact tggcccggat agggctcctg caggatggtt agatgagaag     540
acagtggtag agtttgtgaa gtttgccgcc ttcgttgctt atcaccttga tgacctcgtt     600
gacatgtgga gcacaatgaa cgaaccaaac gtagtctaca atcaaggtta cattaatcta     660
cgttcaggat ttccaccagg atatctaagc tttgaagcag cagaaaaggc aaaattcaac     720
ttaattcagg ctcacatcgg agcatatgat gccataaaag agtattcaga aaaatccgtg     780
ggagtgatat acgcctttgc ttggcacgat cctctagcgg aggagtataa ggatgaagta     840
gaggaaatca gaaagaaaga ctatgagttt gtaacaattc tacactcaaa aggaaagcta     900
gactggatcg gcgtaaacta ctactccagg ctggtatatg gagccaaaga tggacaccta     960
gttcctttac ctggatatgg atttatgagt gagagaggag gatttgcaaa gtcaggaaga    1020
cctgctagtg actttggatg ggaaatgtac ccagagggcc ttgagaacct tcttaagtat    1080
ttaaacaatg cctacgagct accaatgata attacagaga acggtatggc cgatgcagca    1140
gatagataca ggccacacta tctcgtaagc catctaaagg cagtttacaa tgctatgaaa    1200
gaaggtgctg atgttagagg gtatctccac tggtctctaa cagacaacta cgaatgggcc    1260
caagggttca ggatgagatt tggattggtt tacgtggatt tcgagacaaa gaagagatat    1320
ttaaggccaa gcgccctggt attcagagaa atagccactc aaaaagaaat tccagaagaa    1380
ttagctcacc tcgcagacct caaatttgtt acaagaaag                           1419
```

<210> 4
<211> 473
<212> PRT
<213> Pyrococcus furiosus

<400> 4

Met Ala Lys Phe Pro Lys Asn Phe Met Phe Gly Tyr Ser Trp Ser Gly
1 5 10 15

Phe Gln Phe Glu Met Gly Leu Pro Gly Ser Glu Val Glu Ser Asp Trp
20 25 30

Trp Val Trp Val His Asp Lys Glu Asn Ile Ala Ser Gly Leu Val Ser
35 40 45

Gly Asp Leu Pro Glu Asn Gly Pro Ala Tyr Trp His Leu Tyr Lys Gln
50 55 60

Asp His Asp Ile Ala Glu Lys Leu Gly Met Asp Cys Ile Arg Gly Gly
65 70 75 80

Ile Glu Trp Ala Arg Ile Phe Pro Lys Pro Thr Phe Asp Val Lys Val
85 90 95

Asp Val Glu Lys Asp Glu Glu Gly Asn Ile Ile Ser Val Asp Val Pro
100 105 110

Glu Ser Thr Ile Lys Glu Leu Glu Lys Ile Ala Asn Met Glu Ala Leu
115 120 125

Glu His Tyr Arg Lys Ile Tyr Ser Asp Trp Lys Glu Arg Gly Lys Thr
130 135 140

Phe Ile Leu Asn Leu Tyr His Trp Pro Leu Pro Leu Trp Ile His Asp
145 150 155 160

Pro Ile Ala Val Arg Lys Leu Gly Pro Asp Arg Ala Pro Ala Gly Trp
165 170 175

Leu Asp Glu Lys Thr Val Val Glu Phe Val Lys Phe Ala Ala Phe Val
180 185 190

Ala Tyr His Leu Asp Asp Leu Val Asp Met Trp Ser Thr Met Asn Glu
195 200 205

Pro Asn Val Val Tyr Asn Gln Gly Tyr Ile Asn Leu Arg Ser Gly Phe
210 215 220

Pro Pro Gly Tyr Leu Ser Phe Glu Ala Ala Glu Lys Ala Lys Phe Asn
225 230 235 240

Leu Ile Gln Ala His Ile Gly Ala Tyr Asp Ala Ile Lys Glu Tyr Ser
245 250 255

Glu Lys Ser Val Gly Val Ile Tyr Ala Phe Ala Trp His Asp Pro Leu

260 265 270

Ala Glu Glu Tyr Lys Asp Glu Val Glu Glu Ile Arg Lys Lys Asp Tyr
275 280 285

Glu Phe Val Thr Ile Leu His Ser Lys Gly Lys Leu Asp Trp Ile Gly
290 295 300

Val Asn Tyr Tyr Ser Arg Leu Val Tyr Gly Ala Lys Asp Gly His Leu
305 310 315 320

Val Pro Leu Pro Gly Tyr Gly Phe Met Ser Glu Arg Gly Gly Phe Ala
325 330 335

Lys Ser Gly Arg Pro Ala Ser Asp Phe Gly Trp Glu Met Tyr Pro Glu
340 345 350

Gly Leu Glu Asn Leu Leu Lys Tyr Leu Asn Asn Ala Tyr Glu Leu Pro
355 360 365

Met Ile Ile Thr Glu Asn Gly Met Ala Asp Ala Ala Asp Arg Tyr Arg
370 375 380

Pro His Tyr Leu Val Ser His Leu Lys Ala Val Tyr Asn Ala Met Lys
385 390 395 400

Glu Gly Ala Asp Val Arg Gly Tyr Leu His Trp Ser Leu Thr Asp Asn
405 410 415

Tyr Glu Trp Ala Gln Gly Phe Arg Met Arg Phe Gly Leu Val Tyr Val
420 425 430

Asp Phe Glu Thr Lys Lys Arg Tyr Leu Arg Pro Ser Ala Leu Val Phe
435 440 445

Arg Glu Ile Ala Thr Gln Lys Glu Ile Pro Glu Glu Leu Ala His Leu
450 455 460

Ala Asp Leu Lys Phe Val Thr Arg Lys
465 470

<210> 5
<211> 1419
<212> DNA
<213> Artificial Sequence

<220>
<223> DNA encording mutant beta-glucosidase having glycosylation site.

<400> 5

```
atggcaaagt tcccaaaaaa cttcatgttt ggatattctt ggtctggttt ccagtttgag      60
atgggactgc caggaagtga agtggaaagc gactggtggg tgtgggttca cgacaaggag     120
aacatagcat caggtctagt aagtggagat ctaccagaga acggcccagc atattggaac     180
cgcactaagc aagatcatga cattgcagaa aagctaggaa tggattgtat tagaggtggc     240
attgagtggg caagaatttt tccaaagcca acatttgacg ttaaagttga tgtggaaaag     300
gatgaagaag gcaacataat ttccgtagac gttccagaga gtacaataaa agagctagag     360
aaaattgcca acatggaggc ccttgaacat tatcgcaaga tttactcaga ctggaaggag     420
aggggcaaaa ccttcatatt aaacctctac cactggcctc ttccattatg gattcatgac     480
ccaattgcag taaggaaact tggcccggat agggctcctg caggatggtt agatgagaag     540
acagtggtag agtttgtgaa gtttgccgcc ttcgttgctt atcaccttga tgacctcgtt     600
gacatgtgga gcacaatgaa cgaaccaaac gtagtctaca atcaaggtta cattaatcta     660
cgttcaggat ttccaccagg atatctaagc tttgaagcag cagaaaaggc aaaattcaac     720
ttaattcagg ctcacatcgg agcatatgat gccataaaag agtattcaga aaaatccgtg     780
ggagtgatat acgcctttgc ttggcacgat cctctagcgg aggagtataa ggatgaagta     840
gaggaaatca gaaagaaaga ctatgagttt gtaacaattc tacactcaaa aggaaagcta     900
gactggatcg gcgtaaacta ctactccagg ctggtatatg gagccaaaga tggacaccta     960
gttcctttac ctggatatgg atttatgagt gagagaggag gatttgcaaa gtcaggaaga    1020
cctgctagtg actttggatg ggaaatgtac ccagagggcc ttgagaacct tcttaagtat    1080
ttaaacaatg cctacgagct accaatgata attacagaga acggtatggc cgatgcagca    1140
gatagataca ggccacacta tctcgtaagc catctaaagg cagtttacaa tgctatgaaa    1200
gaaggtgctg atgttagagg gtatctccac tggtctctaa cagacaacta cgaatgggcc    1260
caagggttca ggatgagatt tggattggtt tacgtggatt tcgagacaaa gaagagatat    1320
ttaaggccaa gcgccctggt attcagagaa atagccactc aaaaagaaat tccagaagaa    1380
ttagctcacc tcgcagacct caaatttgtt acaagaaag                           1419
```

<210> 6
<211> 473
<212> PRT
<213> Artificial Sequence

<220>
<223> Mutant beta-glucosidase having glycosylation site.

<220>
<221> CARBOHYD

<222> (60)..(62)
<223>

<400> 6

Met Ala Lys Phe Pro Lys Asn Phe Met Phe Gly Tyr Ser Trp Ser Gly
1               5                   10                  15

Phe Gln Phe Glu Met Gly Leu Pro Gly Ser Glu Val Glu Ser Asp Trp
20 25 30

Trp Val Trp Val His Asp Lys Glu Asn Ile Ala Ser Gly Leu Val Ser
35 40 45

Gly Asp Leu Pro Glu Asn Gly Pro Ala Tyr Trp Asn Arg Thr Lys Gln
50 55 60

Asp His Asp Ile Ala Glu Lys Leu Gly Met Asp Cys Ile Arg Gly Gly
65 70 75 80

Ile Glu Trp Ala Arg Ile Phe Pro Lys Pro Thr Phe Asp Val Lys Val
85 90 95

Asp Val Glu Lys Asp Glu Glu Gly Asn Ile Ile Ser Val Asp Val Pro
100 105 110

Glu Ser Thr Ile Lys Glu Leu Glu Lys Ile Ala Asn Met Glu Ala Leu
115 120 125

Glu His Tyr Arg Lys Ile Tyr Ser Asp Trp Lys Glu Arg Gly Lys Thr
130 135 140

Phe Ile Leu Asn Leu Tyr His Trp Pro Leu Pro Leu Trp Ile His Asp
145 150 155 160

Pro Ile Ala Val Arg Lys Leu Gly Pro Asp Arg Ala Pro Ala Gly Trp
165 170 175

Leu Asp Glu Lys Thr Val Val Glu Phe Val Lys Phe Ala Ala Phe Val
180 185 190

Ala Tyr His Leu Asp Asp Leu Val Asp Met Trp Ser Thr Met Asn Glu
195 200 205

Pro Asn Val Val Tyr Asn Gln Gly Tyr Ile Asn Leu Arg Ser Gly Phe
210 215 220

Pro Pro Gly Tyr Leu Ser Phe Glu Ala Ala Glu Lys Ala Lys Phe Asn
225 230 235 240

Leu Ile Gln Ala His Ile Gly Ala Tyr Asp Ala Ile Lys Glu Tyr Ser
245 250 255

Glu Lys Ser Val Gly Val Ile Tyr Ala Phe Ala Trp His Asp Pro Leu
260 265 270

```
Ala Glu Glu Tyr Lys Asp Glu Val Glu Glu Ile Arg Lys Lys Asp Tyr
        275                 280                 285

Glu Phe Val Thr Ile Leu His Ser Lys Gly Lys Leu Asp Trp Ile Gly
    290                 295                 300

Val Asn Tyr Tyr Ser Arg Leu Val Tyr Gly Ala Lys Asp Gly His Leu
305                 310                 315                 320

Val Pro Leu Pro Gly Tyr Gly Phe Met Ser Glu Arg Gly Gly Phe Ala
                325                 330                 335

Lys Ser Gly Arg Pro Ala Ser Asp Phe Gly Trp Glu Met Tyr Pro Glu
            340                 345                 350

Gly Leu Glu Asn Leu Leu Lys Tyr Leu Asn Asn Ala Tyr Glu Leu Pro
        355                 360                 365

Met Ile Ile Thr Glu Asn Gly Met Ala Asp Ala Ala Asp Arg Tyr Arg
    370                 375                 380

Pro His Tyr Leu Val Ser His Leu Lys Ala Val Tyr Asn Ala Met Lys
385                 390                 395                 400

Glu Gly Ala Asp Val Arg Gly Tyr Leu His Trp Ser Leu Thr Asp Asn
                405                 410                 415

Tyr Glu Trp Ala Gln Gly Phe Arg Met Arg Phe Gly Leu Val Tyr Val
            420                 425                 430

Asp Phe Glu Thr Lys Lys Arg Tyr Leu Arg Pro Ser Ala Leu Val Phe
        435                 440                 445

Arg Glu Ile Ala Thr Gln Lys Glu Ile Pro Glu Glu Leu Ala His Leu
    450                 455                 460

Ala Asp Leu Lys Phe Val Thr Arg Lys
465                 470
```

<210> 7
<211> 1446
<212> DNA
<213> Thermosphaera aggregans

<400> 7

```
atgaaattcc ccaaagactt catgataggc tactcatctt caccgtttca atttgaagct      60
ggtattcccg ggtccgagga tccgaatagt gattggtggg tatgggtgca tgatccggag     120
aacacagcag ctggactagt cagcggcgat tttcccgaga acggcccagg ttactggaat     180
ttaaaccaaa atgaccacga cctggctgag aagctggggg ttaacactat tagagtaggc     240
gttgagtgga gtaggatttt tccaaagcca actttcaatg ttaaagtccc tgtagagaga     300
gatgagaacg gcagcattgt tcacgtagat gtcgatgata aagcggttga aagacttgat     360
gaattagcca acaaggaggc cgtaaaccat tacgtagaaa tgtataaaga ctgggttgaa     420
agaggtagaa aacttatact caatttatac cattggcccc tgcctctctg gcttcacaac     480
ccaatcatgg tgagaagaat gggcccggac agagcgccct caggctggct taacgaggag     540
tccgtggtgg agtttgccaa atacgccgca tacattgctt ggaaaatggg cgagctacct     600
gttatgtgga gcaccatgaa cgaacccaac gtcgtttatg agcaaggata catgttcgtt     660
aaagggggtt tcccacccgg ctacttgagt ttggaagctg ctgataaggc caggagaaat     720
atgatccagg ctcatgcacg ggcctatgac aatattaaac gcttcagtaa gaaacctgtt     780
ggactaatat acgctttcca atggttcgaa ctattagagg gtccagcaga agtatttgat     840
aagtttaaga gctctaagtt atactatttc acagacatag tatcgaaggg tagttcaatc     900
atcaatgttg aatacaggag agatcttgcc aataggctag actggttggg cgttaactac     960
tatagccgtt tagtctacaa aatcgtcgat gacaaaccta taatcctgca cgggtatgga    1020
ttcctttgta cacctggggg gatcagcccg gctgaaaatc cttgtagcga ttttgggtgg    1080
gaggtgtatc ctgaaggact ctacctactt ctaaaagaac tttacaaccg atacggggta    1140
gacttgatcg tgaccgagaa cggtgtttca gacagcaggg atgcgttgag accggcatac    1200
ctggtctcgc atgtttacag cgtatggaaa gccgctaacg agggcattcc cgtcaaaggc    1260
tacctccact ggagcttgac agacaattac gagtgggccc agggcttcag gcagaaattc    1320
ggtttagtca tggttgactt caaaactaag aaaaggtatc tccgcccaag cgccctagtg    1380
ttccgggaga tcgcaacgca taacggaata ccggatgagc tacagcatct tacactgatc    1440
cagtaa                                                               1446
```

<210> 8
<211> 481
<212> PRT
<213> Thermosphaera aggregans

<400> 8

```
Met Lys Phe Pro Lys Asp Phe Met Ile Gly Tyr Ser Ser Ser Pro Phe
1               5                   10                  15

Gln Phe Glu Ala Gly Ile Pro Gly Ser Glu Asp Pro Asn Ser Asp Trp
            20                  25                  30

Trp Val Trp Val His Asp Pro Glu Asn Thr Ala Ala Gly Leu Val Ser
        35                  40                  45

Gly Asp Phe Pro Glu Asn Gly Pro Gly Tyr Trp Asn Leu Asn Gln Asn
```

50 55 60

Asp His Asp Leu Ala Glu Lys Leu Gly Val Asn Thr Ile Arg Val Gly
65 70 75 80

Val Glu Trp Ser Arg Ile Phe Pro Lys Pro Thr Phe Asn Val Lys Val
85 90 95

Pro Val Glu Arg Asp Glu Asn Gly Ser Ile Val His Val Asp Val Asp
100 105 110

Asp Lys Ala Val Glu Arg Leu Asp Glu Leu Ala Asn Lys Glu Ala Val
115 120 125

Asn His Tyr Val Glu Met Tyr Lys Asp Trp Val Glu Arg Gly Arg Lys
130 135 140

Leu Ile Leu Asn Leu Tyr His Trp Pro Leu Pro Leu Trp Leu His Asn
145 150 155 160

Pro Ile Met Val Arg Arg Met Gly Pro Asp Arg Ala Pro Ser Gly Trp
165 170 175

Leu Asn Glu Glu Ser Val Val Glu Phe Ala Lys Tyr Ala Ala Tyr Ile
180 185 190

Ala Trp Lys Met Gly Glu Leu Pro Val Met Trp Ser Thr Met Asn Glu
195 200 205

Pro Asn Val Val Tyr Glu Gln Gly Tyr Met Phe Val Lys Gly Gly Phe
210 215 220

Pro Pro Gly Tyr Leu Ser Leu Glu Ala Ala Asp Lys Ala Arg Arg Asn
225 230 235 240

Met Ile Gln Ala His Ala Arg Ala Tyr Asp Asn Ile Lys Arg Phe Ser
245 250 255

Lys Lys Pro Val Gly Leu Ile Tyr Ala Phe Gln Trp Phe Glu Leu Leu
260 265 270

Glu Gly Pro Ala Glu Val Phe Asp Lys Phe Lys Ser Ser Lys Leu Tyr
275 280 285

Tyr Phe Thr Asp Ile Val Ser Lys Gly Ser Ser Ile Ile Asn Val Glu
290 295 300

Tyr Arg Arg Asp Leu Ala Asn Arg Leu Asp Trp Leu Gly Val Asn Tyr
305 310 315 320

Tyr Ser Arg Leu Val Tyr Lys Ile Val Asp Asp Lys Pro Ile Ile Leu
325 330 335

His Gly Tyr Gly Phe Leu Cys Thr Pro Gly Gly Ile Ser Pro Ala Glu
340 345 350

Asn Pro Cys Ser Asp Phe Gly Trp Glu Val Tyr Pro Glu Gly Leu Tyr
355 360 365

Leu Leu Leu Lys Glu Leu Tyr Asn Arg Tyr Gly Val Asp Leu Ile Val
370 375 380

Thr Glu Asn Gly Val Ser Asp Ser Arg Asp Ala Leu Arg Pro Ala Tyr
385 390 395 400

Leu Val Ser His Val Tyr Ser Val Trp Lys Ala Ala Asn Glu Gly Ile
405 410 415

Pro Val Lys Gly Tyr Leu His Trp Ser Leu Thr Asp Asn Tyr Glu Trp
420 425 430

Ala Gln Gly Phe Arg Gln Lys Phe Gly Leu Val Met Val Asp Phe Lys
435 440 445

Thr Lys Lys Arg Tyr Leu Arg Pro Ser Ala Leu Val Phe Arg Glu Ile
450 455 460

Ala Thr His Asn Gly Ile Pro Asp Glu Leu Gln His Leu Thr Leu Ile
465 470 475 480

Gln

<210> 9
<211> 1461
<212> DNA
<213> Caldivirga maquilingensis

<400> 9

atgattaagt tcccaagcga cttcagattc ggcttctcca cagtgggtac tcagcatgag 60
atgggtaccc ctggttctga attcgtaagt gactggtatg tgtggcttca tgaccctgag 120
aacattgctt cgggcttagt tagcggtgat ttacctgaac atgggccagg ttactgggac 180
ttgtataagc aggaccactc aatagctagg gatcttgggc ttgatgcagc atggataact 240
attgagtggg ctagggtgtt ccctaagccg acctttgacg ttaaggttaa ggttgatgag 300
gatgatggag gtaacgtggt tgacgttgag gttaatgaat cagcattaga ggagttacgc 360
aggctagctg acttaaatgc tgttaatcac tataggggga ttttaagtga ttggaaggag 420

aggggtggtt tactggtgat taacctttac cactgggcta tgcctacgtg gcttcatgac 480
ccaatagccg ttaggaagaa tggacctgat agagcccccct ccggttggct tgataagaga 540
tccgttattg agttcactaa gttcgcagcc ttcatagccc atgagttagg tgacttagct 600
gacatgtggt atacgatgaa tgaacctggg gtagtgataa ctgagggtta cctttacgtt 660
aagtcaggct tcccaccagg ttacctggac ttaaactccc tagccactgc gggtaagcat 720
ttaattgagg ctcatgccag agcctacgac gccattaaag cctactcaag gaaaccagtg 780
ggcctagtct actccttcgc agactatcag ccgcttaggc agggtgatga ggaggctgtt 840
aaggaggcta agggacttga ctactcattc ttcgacgctc caattaaggg tgaattaatg 900
gggggttacta gggatgactt gaagggtagg cttgactgga ttggggtaaa ctactacact 960
agggccgtat tgaggaggag gcaggatgct ggtcgggcat cagtagccgt ggtggatgga 1020
ttcggctact cctgtgaacc tggaggcgta tctaatgata ggagaccatg cagtgacttc 1080
ggctgggaaa tataccctga gggtgtttac aatgtcttaa tggacctatg gaggaggtat 1140
aggatgccca tgtacatcac tgagaacggt atagctgatg agcatgataa gtggaggtca 1200
tggttcatag tatcgcacct gtatcaaatt cacagggcca tggaggaggg ggtggatgtt 1260
agagggtact tccactggaa cctaatagat aacttggagt gggctgcagg atataggatg 1320
aggttcggcc tagtttacgt tgactatgca accaagagga ggtattttag gccaagcgcc 1380
ctggttatga gggaggtggc taaacagaag gctataccgg attacttaga gcattacatt 1440
aaaccaccta gaattgaatg a 1461

<210> 10
<211> 486
<212> PRT
<213> Caldivirga maquilingensis

<400> 10

```
Met Ile Lys Phe Pro Ser Asp Phe Arg Phe Gly Phe Ser Thr Val Gly
1               5                   10                  15

Thr Gln His Glu Met Gly Thr Pro Gly Ser Glu Phe Val Ser Asp Trp
            20                  25                  30

Tyr Val Trp Leu His Asp Pro Glu Asn Ile Ala Ser Gly Leu Val Ser
        35                  40                  45

Gly Asp Leu Pro Glu His Gly Pro Gly Tyr Trp Asp Leu Tyr Lys Gln
    50                  55                  60

Asp His Ser Ile Ala Arg Asp Leu Gly Leu Asp Ala Ala Trp Ile Thr
65                  70                  75                  80
```

Ile Glu Trp Ala Arg Val Phe Pro Lys Pro Thr Phe Asp Val Lys Val
85 90 95

Lys Val Asp Glu Asp Asp Gly Gly Asn Val Val Asp Val Glu Val Asn
100 105 110

Glu Ser Ala Leu Glu Glu Leu Arg Arg Leu Ala Asp Leu Asn Ala Val
115 120 125

Asn His Tyr Arg Gly Ile Leu Ser Asp Trp Lys Glu Arg Gly Gly Leu
130 135 140

Leu Val Ile Asn Leu Tyr His Trp Ala Met Pro Thr Trp Leu His Asp
145 150 155 160

Pro Ile Ala Val Arg Lys Asn Gly Pro Asp Arg Ala Pro Ser Gly Trp
165 170 175

Leu Asp Lys Arg Ser Val Ile Glu Phe Thr Lys Phe Ala Ala Phe Ile
180 185 190

Ala His Glu Leu Gly Asp Leu Ala Asp Met Trp Tyr Thr Met Asn Glu
195 200 205

Pro Gly Val Val Ile Thr Glu Gly Tyr Leu Tyr Val Lys Ser Gly Phe
210 215 220

Pro Pro Gly Tyr Leu Asp Leu Asn Ser Leu Ala Thr Ala Gly Lys His
225 230 235 240

Leu Ile Glu Ala His Ala Arg Ala Tyr Asp Ala Ile Lys Ala Tyr Ser
245 250 255

Arg Lys Pro Val Gly Leu Val Tyr Ser Phe Ala Asp Tyr Gln Pro Leu
260 265 270

Arg Gln Gly Asp Glu Glu Ala Val Lys Glu Ala Lys Gly Leu Asp Tyr
275 280 285

Ser Phe Phe Asp Ala Pro Ile Lys Gly Glu Leu Met Gly Val Thr Arg
290 295 300

Asp Asp Leu Lys Gly Arg Leu Asp Trp Ile Gly Val Asn Tyr Tyr Thr
305 310 315 320

Arg Ala Val Leu Arg Arg Arg Gln Asp Ala Gly Arg Ala Ser Val Ala
325 330 335

Val Val Asp Gly Phe Gly Tyr Ser Cys Glu Pro Gly Gly Val Ser Asn

340 345 350

Asp Arg Arg Pro Cys Ser Asp Phe Gly Trp Glu Ile Tyr Pro Glu Gly
355 360 365

Val Tyr Asn Val Leu Met Asp Leu Trp Arg Arg Tyr Arg Met Pro Met
370 375 380

Tyr Ile Thr Glu Asn Gly Ile Ala Asp Glu His Asp Lys Trp Arg Ser
385 390 395 400

Trp Phe Ile Val Ser His Leu Tyr Gln Ile His Arg Ala Met Glu Glu
405 410 415

Gly Val Asp Val Arg Gly Tyr Phe His Trp Asn Leu Ile Asp Asn Leu
420 425 430

Glu Trp Ala Ala Gly Tyr Arg Met Arg Phe Gly Leu Val Tyr Val Asp
435 440 445

Tyr Ala Thr Lys Arg Arg Tyr Phe Arg Pro Ser Ala Leu Val Met Arg
450 455 460

Glu Val Ala Lys Gln Lys Ala Ile Pro Asp Tyr Leu Glu His Tyr Ile
465 470 475 480

Lys Pro Pro Arg Ile Glu
485

<210> 11
<211> 1476
<212> DNA
<213> Sulfolobus acidocaldarius

<400> 11

atgttatcat tcccaaaggg tttcaaattt ggctggtctc agtcgggatt ccagtctgaa 60
atgggaactc caggtagcga ggatccgaac agcgattggc acgtctgggt tcatgacagg 120
gagaacatag tctcacaggt tgtcagtgga gatttacccg aaaatggtcc agggtactgg 180
gggaactata agagatttca tgacgaagca gagaaaatag gactaaatgc agtgagaatt 240
aacgtagagt ggagtagaat atttcccaga ccactaccca agcctgaaat gcaaacaggg 300
actgataaag agaacagtcc tgtcattagc gtagacttaa atgagtctaa gctgagagaa 360
atggacaact acgctaatca tgaagcgtta tcacattaca ggcaaatact ggaggatcta 420
agaaacagag gatttcacat agtactgaac atgtatcatt ggactttgcc catatggttg 480
cacgacccta tcagagtgag gagaggagac tttacaggac caacaggttg gttaaactcc 540
aggacagttt atgagttcgc taggttctcg gcttacgtag cctggaaatt agatgatttg 600
gcgagtgaat atgcaacaat gaatgaacct aacgtggttt ggggagcagg ttacgctttt 660
cctagagcag gctttccacc taattacctt agcttcaggc tttcagaaat agctaaatgg 720
aatataattc aggctcatgc gagggcttat gacgccatca agagcgtatc aaaaaagagt 780
gtaggtataa tatatgcaaa cacatcatat tacccactca gaccacaaga taacgaagct 840
gtggaaatag cagagagatt gaacagatgg agtttctttg actccattat aaagggagag 900
ataactagtg agggacaaaa tgtcagagag gacttaagga acaggttaga ctggattggc 960
gtaaactatt acacgaggac tgtggtaaca aaagctgaga gtggttattt aacccttccg 1020
ggttatggag atcgttgtga aaggaactca ttgagtttag ctaacctccc taccagtgat 1080
ttcggttggg agttctttcc tgagggtcta tatgatgtac ttttgaagta ttggaatagg 1140
tatgggttac cattatacgt aatggagaac ggtatcgctg atgacgctga ctaccaaaga 1200
ccgtattact tagtatcaca tatctaccag gtgcacaggg ctttaaacga gggagtagat 1260
gtaagaggtt atcttcattg gtctttggca gataattatg agtggtcgtc aggtttttca 1320
atgaggttcg gtctacttaa ggtagattat ctaacaaaga gattgtactg gagaccttct 1380
gcattagttt acagggagat tactaggagt aacggtattc ctgaggagct ggaacatcta 1440
aacagagtac caccaataaa acctttgaga cattaa 1476

<210> 12
<211> 491
<212> PRT
<213> Sulfolobus acidocaldarius

<400> 12

```
Met Leu Ser Phe Pro Lys Gly Phe Lys Phe Gly Trp Ser Gln Ser Gly
1               5                   10                  15

Phe Gln Ser Glu Met Gly Thr Pro Gly Ser Glu Asp Pro Asn Ser Asp
            20                  25                  30

Trp His Val Trp Val His Asp Arg Glu Asn Ile Val Ser Gln Val Val
        35                  40                  45

Ser Gly Asp Leu Pro Glu Asn Gly Pro Gly Tyr Trp Gly Asn Tyr Lys
    50                  55                  60

Arg Phe His Asp Glu Ala Glu Lys Ile Gly Leu Asn Ala Val Arg Ile
65                  70                  75                  80

Asn Val Glu Trp Ser Arg Ile Phe Pro Arg Pro Leu Pro Lys Pro Glu
                85                  90                  95

Met Gln Thr Gly Thr Asp Lys Glu Asn Ser Pro Val Ile Ser Val Asp
            100                 105                 110
```

Leu Asn Glu Ser Lys Leu Arg Glu Met Asp Asn Tyr Ala Asn His Glu
115 120 125

Ala Leu Ser His Tyr Arg Gln Ile Leu Glu Asp Leu Arg Asn Arg Gly
130 135 140

Phe His Ile Val Leu Asn Met Tyr His Trp Thr Leu Pro Ile Trp Leu
145 150 155 160

His Asp Pro Ile Arg Val Arg Arg Gly Asp Phe Thr Gly Pro Thr Gly
165 170 175

Trp Leu Asn Ser Arg Thr Val Tyr Glu Phe Ala Arg Phe Ser Ala Tyr
180 185 190

Val Ala Trp Lys Leu Asp Asp Leu Ala Ser Glu Tyr Ala Thr Met Asn
195 200 205

Glu Pro Asn Val Val Trp Gly Ala Gly Tyr Ala Phe Pro Arg Ala Gly
210 215 220

Phe Pro Pro Asn Tyr Leu Ser Phe Arg Leu Ser Glu Ile Ala Lys Trp
225 230 235 240

Asn Ile Ile Gln Ala His Ala Arg Ala Tyr Asp Ala Ile Lys Ser Val
245 250 255

Ser Lys Lys Ser Val Gly Ile Ile Tyr Ala Asn Thr Ser Tyr Tyr Pro
260 265 270

Leu Arg Pro Gln Asp Asn Glu Ala Val Glu Ile Ala Glu Arg Leu Asn
275 280 285

Arg Trp Ser Phe Phe Asp Ser Ile Ile Lys Gly Glu Ile Thr Ser Glu
290 295 300

Gly Gln Asn Val Arg Glu Asp Leu Arg Asn Arg Leu Asp Trp Ile Gly
305 310 315 320

Val Asn Tyr Tyr Thr Arg Thr Val Val Thr Lys Ala Glu Ser Gly Tyr
325 330 335

Leu Thr Leu Pro Gly Tyr Gly Asp Arg Cys Glu Arg Asn Ser Leu Ser
340 345 350

Leu Ala Asn Leu Pro Thr Ser Asp Phe Gly Trp Glu Phe Phe Pro Glu
355 360 365

```
Gly Leu Tyr Asp Val Leu Leu Lys Tyr Trp Asn Arg Tyr Gly Leu Pro
    370                 375                 380

Leu Tyr Val Met Glu Asn Gly Ile Ala Asp Asp Ala Asp Tyr Gln Arg
385                 390                 395                 400

Pro Tyr Tyr Leu Val Ser His Ile Tyr Gln Val His Arg Ala Leu Asn
                405                 410                 415

Glu Gly Val Asp Val Arg Gly Tyr Leu His Trp Ser Leu Ala Asp Asn
            420                 425                 430

Tyr Glu Trp Ser Ser Gly Phe Ser Met Arg Phe Gly Leu Leu Lys Val
        435                 440                 445

Asp Tyr Leu Thr Lys Arg Leu Tyr Trp Arg Pro Ser Ala Leu Val Tyr
    450                 455                 460

Arg Glu Ile Thr Arg Ser Asn Gly Ile Pro Glu Glu Leu Glu His Leu
465                 470                 475                 480

Asn Arg Val Pro Pro Ile Lys Pro Leu Arg His
                485                 490
```

<210> 13
<211> 1470
<212> DNA
<213> Sulfolobus solfataricus

<400> 13

```
atgtactcat ttccaaatag ctttaggttt ggttggtccc aggccggatt tcaatcagaa     60
atgggaacac cagggtcaga agatccaaat actgactggt ataaatgggt tcatgatcca    120
gaaaacatgg cagcgggatt agtaagtgga gatctaccag aaaatgggcc aggctactgg    180
ggaaactata agacatttca cgataatgca caaaaaatgg gattaaaaat agctagacta    240
aatgtggaat ggtctaggat atttcctaat ccattaccaa ggccacaaaa ctttgatgaa    300
tcaaaacaag atgtgacaga ggttgagata aacgaaaacg agttaaagag acttgacgag    360
tacgctaata aagacgcatt aaaccattac agggaaatat tcaaggatct taaaagtaga    420
ggactttact ttatactaaa catgtatcat tggccattac ctctatggtt acacgaccca    480
ataagagtaa gaagaggaga ttttactgga ccaagtggtt ggctaagtac tagaacagtt    540
tacgaattcg ctagattctc agcttatata gcttggaaat tcgatgatct agtggatgag    600
tactcaacaa tgaatgaacc taacgttgtt ggaggtttag gatacgttgg tgttaagtcc    660
ggttttcccc caggatacct aagctttgaa ctttcccgta gggcaatgta taacatcatt    720
caagctcacg caagagcgta tgatgggata aagagtgttt ctaaaaaacc agttggaatt    780
atttacgcta atagctcatt ccagccgtta acggataaag atatggaagc ggtagagatg    840
gctgaaaatg ataatagatg gtggttcttt gatgctataa taagaggtga gatcaccaga    900
ggaaacgaga agattgtaag agatgaccta aagggtagat tggattggat tggagttaat    960
tattacacta ggactgttgt gaagaggact gaaaagggat acgttagctt aggaggttac   1020
ggtcacggat gtgagaggaa ttctgtaagt ttagcgggat taccaaccag cgacttcggc   1080
tgggagttct tcccagaagg tttatatgac gttttgacga aatactggaa tagatatcat   1140
ctctatatgt acgttactga aaatggtatt gcggatgatg ccgattatca aaggccctat   1200
tatttagtat ctcacgttta tcaagttcat agagcaataa atagtggtgc agatgttaga   1260
gggtatttac attggtctct agctgataat tacgaatggg cttcaggatt ctctatgagg   1320
tttggtctgt taaaggtcga ttacaacact aagagactat actggagacc ctcagcacta   1380
gtatataggg aaatcgccac aaatggcgca ataactgatg aaatagagca cttaaatagc   1440
gtacctccag taaagccatt aaggcactaa                                     1470
```

<210> 14
<211> 489
<212> PRT
<213> Sulfolobus solfataricus

<400> 14

```
Met Tyr Ser Phe Pro Asn Ser Phe Arg Phe Gly Trp Ser Gln Ala Gly
1               5                   10                  15

Phe Gln Ser Glu Met Gly Thr Pro Gly Ser Glu Asp Pro Asn Thr Asp
            20                  25                  30

Trp Tyr Lys Trp Val His Asp Pro Glu Asn Met Ala Ala Gly Leu Val
        35                  40                  45

Ser Gly Asp Leu Pro Glu Asn Gly Pro Gly Tyr Trp Gly Asn Tyr Lys
    50                  55                  60

Thr Phe His Asp Asn Ala Gln Lys Met Gly Leu Lys Ile Ala Arg Leu
65                  70                  75                  80

Asn Val Glu Trp Ser Arg Ile Phe Pro Asn Pro Leu Pro Arg Pro Gln
                85                  90                  95

Asn Phe Asp Glu Ser Lys Gln Asp Val Thr Glu Val Glu Ile Asn Glu
            100                 105                 110

Asn Glu Leu Lys Arg Leu Asp Glu Tyr Ala Asn Lys Asp Ala Leu Asn
        115                 120                 125

His Tyr Arg Glu Ile Phe Lys Asp Leu Lys Ser Arg Gly Leu Tyr Phe
    130                 135                 140
```

Ile Leu Asn Met Tyr His Trp Pro Leu Pro Leu Trp Leu His Asp Pro
145 150 155 160

Ile Arg Val Arg Arg Gly Asp Phe Thr Gly Pro Ser Gly Trp Leu Ser
165 170 175

Thr Arg Thr Val Tyr Glu Phe Ala Arg Phe Ser Ala Tyr Ile Ala Trp
180 185 190

Lys Phe Asp Asp Leu Val Asp Glu Tyr Ser Thr Met Asn Glu Pro Asn
195 200 205

Val Val Gly Gly Leu Gly Tyr Val Gly Val Lys Ser Gly Phe Pro Pro
210 215 220

Gly Tyr Leu Ser Phe Glu Leu Ser Arg Arg Ala Met Tyr Asn Ile Ile
225 230 235 240

Gln Ala His Ala Arg Ala Tyr Asp Gly Ile Lys Ser Val Ser Lys Lys
245 250 255

Pro Val Gly Ile Ile Tyr Ala Asn Ser Ser Phe Gln Pro Leu Thr Asp
260 265 270

Lys Asp Met Glu Ala Val Glu Met Ala Glu Asn Asp Asn Arg Trp Trp
275 280 285

Phe Phe Asp Ala Ile Ile Arg Gly Glu Ile Thr Arg Gly Asn Glu Lys
290 295 300

Ile Val Arg Asp Asp Leu Lys Gly Arg Leu Asp Trp Ile Gly Val Asn
305 310 315 320

Tyr Tyr Thr Arg Thr Val Val Lys Arg Thr Glu Lys Gly Tyr Val Ser
325 330 335

Leu Gly Gly Tyr Gly His Gly Cys Glu Arg Asn Ser Val Ser Leu Ala
340 345 350

Gly Leu Pro Thr Ser Asp Phe Gly Trp Glu Phe Phe Pro Glu Gly Leu
355 360 365

Tyr Asp Val Leu Thr Lys Tyr Trp Asn Arg Tyr His Leu Tyr Met Tyr
370 375 380

Val Thr Glu Asn Gly Ile Ala Asp Asp Ala Asp Tyr Gln Arg Pro Tyr
385 390 395 400

Tyr Leu Val Ser His Val Tyr Gln Val His Arg Ala Ile Asn Ser Gly
405 410 415

Ala Asp Val Arg Gly Tyr Leu His Trp Ser Leu Ala Asp Asn Tyr Glu
420 425 430

Trp Ala Ser Gly Phe Ser Met Arg Phe Gly Leu Leu Lys Val Asp Tyr
435 440 445

Asn Thr Lys Arg Leu Tyr Trp Arg Pro Ser Ala Leu Val Tyr Arg Glu
450 455 460

Ile Ala Thr Asn Gly Ala Ile Thr Asp Glu Ile Glu His Leu Asn Ser
465 470 475 480

Val Pro Pro Val Lys Pro Leu Arg His
485

<210> 15
<211> 1488
<212> DNA
<213> Picrophilus torridus

<400> 15

atgttaccca agaacttttt acttggcttt tctctggctg gctttcagtc tgaaatgggc 60
atatcagatc ctgatagcaa ttcagattgg tggttatggg tacatgaccc ggtgaatata 120
aggactggac ttgtatctgg tgacttacct gaaaatggaa taggatactg ggatctttac 180
aaaaaatata atggtctggc tgttcaaaca ggaatgaatg ctgcaaggct tggagttgaa 240
tggagcagga tatttccaaa aagtactgaa gaagtaaagg tgatggaaga ttacaaagat 300
gatgatttaa tttccgtgga tgttaatgag ggaagtcttg aaaaacttga cagactggca 360
aatcaaaagg caattaatag atatatggaa atcttcaata atatcaagga aaataatatg 420
acgctaatag tgaatgttta ccattggcca ataccaatat atcttcacga tccaatagaa 480
gctaggaata gtggactttc aaataaaaga aatggctggc ttaatcataa aaccgttgtg 540
gaatttgtaa aatatgcaaa atatctggca tggaaattta gcgatgtggc agatatgttt 600
tctataatga atgagccaaa cgttgtattt ggtaatggat attttaatgt taaatcaggg 660
ttcccaccag catttccaag tgtgcatggc ggtttgcttg caaaaaaaca tgaaattgag 720
gctatagcaa gatcatacga cgccatgaag gagattacaa aaaaaccagt tggtctaatt 780
atggcaaatt cagatgtaca accactaaca gatgaggata aagaagcagc agaaatggct 840
acttacaatg atcgctattc attcatagat ccgctaagag ttggtgagat gaaatgggct 900
gatgaggtta ctgcaggtaa tccaattggt gaaaagagca acatcgatag atctgatcta 960
aaaaataagc tagactggat aggtgttaac tattatacaa gggccgttgt aaaaaaatct 1020

ggaaacggat atacaacatt aaaaggatat ggacactctg caaccgctgg catgccaagt 1080
agggccggaa gggatgtaag tgactttggc tgggaattct atccagaagg tcttgtaaac 1140
gtcttatcat catactggaa aagatatcac attccaatga ttgtgactga aaatggtgtt 1200
gctgactcta ttgatagact tagaccaagg taccttgtgt cacatataaa gtctgttgaa 1260
aaggctttat ctatgggtat ggatattagg ggatatcttc actggtctct gattgataac 1320
tatgaatggg catcaggttt ttcaatgaaa tttgggcttt atggtattga tttgaacaat 1380
aaaaagattc aacacagacc aagtgcactg gtatttaaag aaattgcaaa tgccaacgga 1440
gtcccggagg aatttgaatg gatggcagac cagcatcaga attcatga 1488

<210> 16
<211> 495
<212> PRT
<213> Picrophilus torridus

<400> 16

```
Met Leu Pro Lys Asn Phe Leu Leu Gly Phe Ser Leu Ala Gly Phe Gln
1               5                   10                  15

Ser Glu Met Gly Ile Ser Asp Pro Asp Ser Asn Ser Asp Trp Trp Leu
            20                  25                  30

Trp Val His Asp Pro Val Asn Ile Arg Thr Gly Leu Val Ser Gly Asp
        35                  40                  45

Leu Pro Glu Asn Gly Ile Gly Tyr Trp Asp Leu Tyr Lys Lys Tyr Asn
    50                  55                  60

Gly Leu Ala Val Gln Thr Gly Met Asn Ala Ala Arg Leu Gly Val Glu
65                  70                  75                  80

Trp Ser Arg Ile Phe Pro Lys Ser Thr Glu Glu Val Lys Val Met Glu
                85                  90                  95

Asp Tyr Lys Asp Asp Asp Leu Ile Ser Val Asp Val Asn Glu Gly Ser
            100                 105                 110

Leu Glu Lys Leu Asp Arg Leu Ala Asn Gln Lys Ala Ile Asn Arg Tyr
        115                 120                 125

Met Glu Ile Phe Asn Asn Ile Lys Glu Asn Asn Met Thr Leu Ile Val
    130                 135                 140

Asn Val Tyr His Trp Pro Ile Pro Ile Tyr Leu His Asp Pro Ile Glu
145                 150                 155                 160

Ala Arg Asn Ser Gly Leu Ser Asn Lys Arg Asn Gly Trp Leu Asn His
```

165 170 175

Lys Thr Val Val Glu Phe Val Lys Tyr Ala Lys Tyr Leu Ala Trp Lys
180 185 190

Phe Ser Asp Val Ala Asp Met Phe Ser Ile Met Asn Glu Pro Asn Val
195 200 205

Val Phe Gly Asn Gly Tyr Phe Asn Val Lys Ser Gly Phe Pro Pro Ala
210 215 220

Phe Pro Ser Val His Gly Gly Leu Leu Ala Lys Lys His Glu Ile Glu
225 230 235 240

Ala Ile Ala Arg Ser Tyr Asp Ala Met Lys Glu Ile Thr Lys Lys Pro
245 250 255

Val Gly Leu Ile Met Ala Asn Ser Asp Val Gln Pro Leu Thr Asp Glu
260 265 270

Asp Lys Glu Ala Ala Glu Met Ala Thr Tyr Asn Asp Arg Tyr Ser Phe
275 280 285

Ile Asp Pro Leu Arg Val Gly Glu Met Lys Trp Ala Asp Glu Val Thr
290 295 300

Ala Gly Asn Pro Ile Gly Glu Lys Ser Asn Ile Asp Arg Ser Asp Leu
305 310 315 320

Lys Asn Lys Leu Asp Trp Ile Gly Val Asn Tyr Tyr Thr Arg Ala Val
325 330 335

Val Lys Lys Ser Gly Asn Gly Tyr Thr Thr Leu Lys Gly Tyr Gly His
340 345 350

Ser Ala Thr Ala Gly Met Pro Ser Arg Ala Gly Arg Asp Val Ser Asp
355 360 365

Phe Gly Trp Glu Phe Tyr Pro Glu Gly Leu Val Asn Val Leu Ser Ser
370 375 380

Tyr Trp Lys Arg Tyr His Ile Pro Met Ile Val Thr Glu Asn Gly Val
385 390 395 400

Ala Asp Ser Ile Asp Arg Leu Arg Pro Arg Tyr Leu Val Ser His Ile
405 410 415

Lys Ser Val Glu Lys Ala Leu Ser Met Gly Met Asp Ile Arg Gly Tyr
420 425 430

Leu His Trp Ser Leu Ile Asp Asn Tyr Glu Trp Ala Ser Gly Phe Ser
435 440 445

Met Lys Phe Gly Leu Tyr Gly Ile Asp Leu Asn Asn Lys Lys Ile Gln
450 455 460

His Arg Pro Ser Ala Leu Val Phe Lys Glu Ile Ala Asn Ala Asn Gly
465 470 475 480

Val Pro Glu Glu Phe Glu Trp Met Ala Asp Gln His Gln Asn Ser
485 490 495

<210> 17
<211> 1446
<212> DNA
<213> Thermoplasma volcanium

<400> 17

```
atggtagaaa acaattttcc agaggatttc aaatttggtt ggtcacagtc aggttttcaa     60
tcggagatgg gctatgataa cgcaatggac gataaaagtg actggtatgt ctgggttcat    120
gataaagaaa acatccaatc agggcttgta agcggagaca tgcccgaaaa tggtccgggt    180
tactggaata actataaatc attccatgaa gctgcacaga atatgggatt aaaaatggca    240
agaatcggag ttgaatggtc aagattattc ccggaacctt tcccggaaaa aataatggca    300
gatgcaaaaa ataattcctt agaaataaac aataacattc tttcagaact tgataaatat    360
gtcaataaag atgcactcaa ccattacatt gagatattta atgatatcaa aaatagaaat    420
atagatttaa taattaatat gtaccactgg ccacttcctg tatggctaag cgatcctgta    480
tctgttagaa aaggaataaa aacagaaaga tcaggctggc tgaatgacag gatagttcaa    540
ttgtttgctt tattctcctc gtatatagta tataaaatgg aagatctggc agttgcattt    600
tcaaccatga atgaacctaa tgttgtttat ggaaatggtt ttataaatat caaatcaggt    660
tttccgcctt cctatctcag ttcagaattt gcatctaaag ttaagaacaa tatattaaaa    720
gcacattctc ttgcatacga ttctatgaaa aaaattacgg ataaacctgt gggaataatt    780
tatgcaaaca catattttac gcctttggat ccggaaaaag ataatgatgc tattgctaaa    840
gcagacagtg atgcgaaatg gtcatttttt gatccattaa taaaaggaga taaatcactt    900
ggaattaatg gcaataaact agattggatc ggaattaatt attatacaag gacaatgtta    960
aggaaagacg gagatggcta tatttcatta aaaggctatg gtcattcagg ttctcctaat   1020
actgtaacaa acgataaaag accaacaagt gatataggat gggaattcta tccggaggga   1080
ttggaatatg taattatgaa ttactggaac aggtataaat tgcctatgta cgtaacagaa   1140
aatggcatag ccgataatgg ggattatcag aggccttatt atttagtttc acacattgca   1200
agtgtactga gggcaataaa taaaggagcc aatgtaaagg gttatttgca ctggtcctta   1260
gttgataatt atgaatgggc attgggattt agcccgaaat ttggtttaat aggatacgat   1320
gaaaataaaa aactatactg gaggccaagt gctcttgttt ataaggaaat agcaacaaaa   1380
aattgcatat ccccagaatt aaagcacctc gattcaatac cgcctataaa tggtttaaga   1440
aaataa                                                               1446
```

<210> 18
<211> 481
<212> PRT
<213> Thermoplasma volcanium

<400> 18

```
Met Val Glu Asn Asn Phe Pro Glu Asp Phe Lys Phe Gly Trp Ser Gln
1               5                   10                  15

Ser Gly Phe Gln Ser Glu Met Gly Tyr Asp Asn Ala Met Asp Asp Lys
            20                  25                  30

Ser Asp Trp Tyr Val Trp Val His Asp Lys Glu Asn Ile Gln Ser Gly
        35                  40                  45

Leu Val Ser Gly Asp Met Pro Glu Asn Gly Pro Gly Tyr Trp Asn Asn
    50                  55                  60

Tyr Lys Ser Phe His Glu Ala Ala Gln Asn Met Gly Leu Lys Met Ala
65                  70                  75                  80

Arg Ile Gly Val Glu Trp Ser Arg Leu Phe Pro Glu Pro Phe Pro Glu
                85                  90                  95

Lys Ile Met Ala Asp Ala Lys Asn Asn Ser Leu Glu Ile Asn Asn Asn
            100                 105                 110

Ile Leu Ser Glu Leu Asp Lys Tyr Val Asn Lys Asp Ala Leu Asn His
        115                 120                 125

Tyr Ile Glu Ile Phe Asn Asp Ile Lys Asn Arg Asn Ile Asp Leu Ile
    130                 135                 140

Ile Asn Met Tyr His Trp Pro Leu Pro Val Trp Leu Ser Asp Pro Val
145                 150                 155                 160

Ser Val Arg Lys Gly Ile Lys Thr Glu Arg Ser Gly Trp Leu Asn Asp
                165                 170                 175

Arg Ile Val Gln Leu Phe Ala Leu Phe Ser Ser Tyr Ile Val Tyr Lys
            180                 185                 190
```

Met Glu Asp Leu Ala Val Ala Phe Ser Thr Met Asn Glu Pro Asn Val
195 200 205

Val Tyr Gly Asn Gly Phe Ile Asn Ile Lys Ser Gly Phe Pro Pro Ser
210 215 220

Tyr Leu Ser Ser Glu Phe Ala Ser Lys Val Lys Asn Asn Ile Leu Lys
225 230 235 240

Ala His Ser Leu Ala Tyr Asp Ser Met Lys Lys Ile Thr Asp Lys Pro
245 250 255

Val Gly Ile Ile Tyr Ala Asn Thr Tyr Phe Thr Pro Leu Asp Pro Glu
260 265 270

Lys Asp Asn Asp Ala Ile Ala Lys Ala Asp Ser Asp Ala Lys Trp Ser
275 280 285

Phe Phe Asp Pro Leu Ile Lys Gly Asp Lys Ser Leu Gly Ile Asn Gly
290 295 300

Asn Lys Leu Asp Trp Ile Gly Ile Asn Tyr Tyr Thr Arg Thr Met Leu
305 310 315 320

Arg Lys Asp Gly Asp Gly Tyr Ile Ser Leu Lys Gly Tyr Gly His Ser
325 330 335

Gly Ser Pro Asn Thr Val Thr Asn Asp Lys Arg Pro Thr Ser Asp Ile
340 345 350

Gly Trp Glu Phe Tyr Pro Glu Gly Leu Glu Tyr Val Ile Met Asn Tyr
355 360 365

Trp Asn Arg Tyr Lys Leu Pro Met Tyr Val Thr Glu Asn Gly Ile Ala
370 375 380

Asp Asn Gly Asp Tyr Gln Arg Pro Tyr Tyr Leu Val Ser His Ile Ala
385 390 395 400

Ser Val Leu Arg Ala Ile Asn Lys Gly Ala Asn Val Lys Gly Tyr Leu
405 410 415

His Trp Ser Leu Val Asp Asn Tyr Glu Trp Ala Leu Gly Phe Ser Pro
420 425 430

Lys Phe Gly Leu Ile Gly Tyr Asp Glu Asn Lys Lys Leu Tyr Trp Arg
435 440 445

Pro Ser Ala Leu Val Tyr Lys Glu Ile Ala Thr Lys Asn Cys Ile Ser

450 455 460

Pro Glu Leu Lys His Leu Asp Ser Ile Pro Pro Ile Asn Gly Leu Arg
465 470 475 480

Lys

<210> 19
<211> 1404
<212> DNA
<213> Fervidobacterium nodosum

<400> 19

```
atgatgtttc cgaaagattt tttatttggt gtttcgatgt ctgggtttca gtttgagatg     60
ggaaatcctc aagatgcaga agaggttgat ctaaatacag attggtatgt atgggttagg    120
gatattggaa atattgtaaa tggagtcgta agtggggact tgcctgaaaa tggttcatgg    180
tactggaagc agtacggcaa agtccaccaa ttagctgccg attttgggat ggatgtaata    240
cgaattggaa ccgaatggtc taggattttc ccagttagta cgcaaagtgt tgagtacggc    300
tcaccggata tgctcgaaaa attggataaa ttagcaaacc aaaaagcggt aagtcattac    360
aggaaaataa tggaggatat aaaagcaaag gggttaaaat tgttcgttaa cctttaccac    420
tttactttac ctatttggtt gcacgaccct atagctgttc acaaaggtga gaagacagat    480
aaaattggtt ggatttctga tgctacacct attgagtttg cgaagtatgc agagtacatg    540
gcgtggaaat ttgccgatat agttgatatg tgggcttcta tgaacgaacc acacgttgta    600
agtcagcttg gatattttgc aataaatgcg ggatttccac caagttattt taatccttca    660
tggtatatca aaagtttaga aaacgaagcg aaagcacata acttatctta tgatgctata    720
aaaaagtata caaataatcc tgttggagtt atatactctt ttacatggta cgatactgtt    780
aataaagatg acaaggaatc ttttgaaaat gctatggatc tcacaaattg gcgatttata    840
gatatggtaa aagataaaac tgattacata ggtgtaaatt attacacaag agcggttatc    900
gatagacttc ccaccactat tgactttggc gaatttaaaa tgaattggta tactttgaga    960
ggttacggtt attcttgcga agaaggagga ttctcactct ccggaaggcc ggcaagcgaa   1020
tttggatggg aaatataccc tgaagggctg tacaatattt tgatacatgt ttataataga   1080
tacaaaaaag atatttatgt tacggagaac ggtatagctg attcgaagga taaatacaga   1140
agtcttttta tcatatcgca tctttatgct atagaaaaag cattaaacga aggaatacca   1200
ataaaaggtt atttgcactg gtcgattata gacaatttcg aatgggcgaa gggctacagt   1260
aaaagatttg gacttgctta cacagatttg tcaaccaaaa aatatatacc tagaccttct   1320
atgtacattt ttagagagat aataaaggat aaatcaatcg acaaattcaa aggttacgat   1380
ccatataact tgatgaaatt ctga                                          1404
```

<210> 20
<211> 467
<212> PRT
<213> Fervidobacterium nodosum

<400> 20

```
Met Met Phe Pro Lys Asp Phe Leu Phe Gly Val Ser Met Ser Gly Phe
1               5                   10                  15

Gln Phe Glu Met Gly Asn Pro Gln Asp Ala Glu Glu Val Asp Leu Asn
            20                  25                  30

Thr Asp Trp Tyr Val Trp Val Arg Asp Ile Gly Asn Ile Val Asn Gly
        35                  40                  45

Val Val Ser Gly Asp Leu Pro Glu Asn Gly Ser Trp Tyr Trp Lys Gln
    50                  55                  60

Tyr Gly Lys Val His Gln Leu Ala Ala Asp Phe Gly Met Asp Val Ile
65                  70                  75                  80

Arg Ile Gly Thr Glu Trp Ser Arg Ile Phe Pro Val Ser Thr Gln Ser
                85                  90                  95

Val Glu Tyr Gly Ser Pro Asp Met Leu Glu Lys Leu Asp Lys Leu Ala
            100                 105                 110

Asn Gln Lys Ala Val Ser His Tyr Arg Lys Ile Met Glu Asp Ile Lys
        115                 120                 125

Ala Lys Gly Leu Lys Leu Phe Val Asn Leu Tyr His Phe Thr Leu Pro
    130                 135                 140

Ile Trp Leu His Asp Pro Ile Ala Val His Lys Gly Glu Lys Thr Asp
145                 150                 155                 160

Lys Ile Gly Trp Ile Ser Asp Ala Thr Pro Ile Glu Phe Ala Lys Tyr
                165                 170                 175

Ala Glu Tyr Met Ala Trp Lys Phe Ala Asp Ile Val Asp Met Trp Ala
            180                 185                 190

Ser Met Asn Glu Pro His Val Val Ser Gln Leu Gly Tyr Phe Ala Ile
        195                 200                 205

Asn Ala Gly Phe Pro Pro Ser Tyr Phe Asn Pro Ser Trp Tyr Ile Lys
    210                 215                 220

Ser Leu Glu Asn Glu Ala Lys Ala His Asn Leu Ser Tyr Asp Ala Ile
```

225 230 235 240

Lys Lys Tyr Thr Asn Asn Pro Val Gly Val Ile Tyr Ser Phe Thr Trp
245 250 255

Tyr Asp Thr Val Asn Lys Asp Asp Lys Glu Ser Phe Glu Asn Ala Met
260 265 270

Asp Leu Thr Asn Trp Arg Phe Ile Asp Met Val Lys Asp Lys Thr Asp
275 280 285

Tyr Ile Gly Val Asn Tyr Tyr Thr Arg Ala Val Ile Asp Arg Leu Pro
290 295 300

Thr Thr Ile Asp Phe Gly Glu Phe Lys Met Asn Trp Tyr Thr Leu Arg
305 310 315 320

Gly Tyr Gly Tyr Ser Cys Glu Glu Gly Gly Phe Ser Leu Ser Gly Arg
325 330 335

Pro Ala Ser Glu Phe Gly Trp Glu Ile Tyr Pro Glu Gly Leu Tyr Asn
340 345 350

Ile Leu Ile His Val Tyr Asn Arg Tyr Lys Lys Asp Ile Tyr Val Thr
355 360 365

Glu Asn Gly Ile Ala Asp Ser Lys Asp Lys Tyr Arg Ser Leu Phe Ile
370 375 380

Ile Ser His Leu Tyr Ala Ile Glu Lys Ala Leu Asn Glu Gly Ile Pro
385 390 395 400

Ile Lys Gly Tyr Leu His Trp Ser Ile Ile Asp Asn Phe Glu Trp Ala
405 410 415

Lys Gly Tyr Ser Lys Arg Phe Gly Leu Ala Tyr Thr Asp Leu Ser Thr
420 425 430

Lys Lys Tyr Ile Pro Arg Pro Ser Met Tyr Ile Phe Arg Glu Ile Ile
435 440 445

Lys Asp Lys Ser Ile Asp Lys Phe Lys Gly Tyr Asp Pro Tyr Asn Leu
450 455 460

Met Lys Phe
465

<210> 21

<211> 33
<212> DNA
<213> Artificial

<220>
<223> primer

<400> 21
ccacatattg gcacctctat aagcaagatc atg

<210> 22
<211> 34
<212> DNA
<213> Artificial

<220>
<223> primer

<400> 22
catgatcttg cttagtgcgg ttccaatatg ctgg

<210> 23
<211> 1446
<212> DNA
<213> Artificial Sequence

<220>
<223> Thermosphaera aggregans

<400> 23

atgggtaaat tccccaaaga cttcatgata ggctactcat cttcaccgtt tcaatttgaa 60
gctggtattc ccgggtccga agatccgaat agtgattggt gggtatgggt gcatgatccg 120
gagaacacag cagctggact agtcagcggc gattttcccg agaacggccc aggttactgg 180
aatttaaacc aaaatgacca cgacctggct gagaagctgg gggttaacac tattagagta 240
ggcgttgagt ggagtaggat tttccaaag ccaactttca atgttaaagt ccctgtagag 300
agagatgaga acggcagcat tgttcacgta gatgtcgatg ataaagcggt tgaaagactt 360
gatgaattag ccaacaagga ggccgtaaac cattacgtag aaatgtataa agactgggtt 420
gaaagaggta gaaaacttat actcaattta taccattggc ccctgcctct ctggcttcac 480
aacccaatca tggtgagaag aatgggcccg gacagagcgc cctcaggctg gcttaacgag 540
gagtccgtgg tggagtttgc caaatacgcc gcatacattg cttggaaaat gggcgagcta 600
cctgttatgt ggagcaccat gaacgaaccc aacgtcgttt atgagcaagg atacatgttc 660
gttaaagggg gtttcccacc cggctacttg agtttggaag ctgctgataa ggccaggaga 720
aatatgatcc aggctcatgc acgggcctat gacaatatta aacgcttcag taagaaacct 780
gttggactaa tatacgcttt ccaatggttc gaactattag agggtccagc agaagtattt 840
gataagttta agagctctaa gttatactat ttcacagaca tagtatcgaa gggtagttca 900
atcatcaatg ttgaatacag gagagatctt gccaataggc tagactggtt gggcgttaac 960
tactatagcc gtttagtcta caaaatcgtc gatgacaaac ctataatcct gcacgggtat 1020

ggattccttt gtacacctgg ggggatcagc ccggctgaaa atccttgtag cgattttggg 1080
tgggaggtgt atcctgaagg actctaccta cttctaaaag aactttacaa ccgatacggg 1140
gtagacttga tcgtgaccga gaacggtgtt tcagacagca gggatgcgtt gagaccggca 1200
tacctggtct cgcatgttta cagcgtatgg aaagccgcta acgagggcat tcccgtcaaa 1260
ggctacctcc actggagctt gacagacaat tacgagtggg cccagggctt caggcagaaa 1320
ttcggtttag tcatggttga cttcaaaact aagaaaaggt atctccgccc aagcgcccta 1380
gtgttccggg agatcgcaac gcataacgga ataccggatg agctacagca tcttacactg 1440
atccag 1446

<210> 24
<211> 482
<212> PRT
<213> Artificial Sequence

<220>
<223> Thermosphaera aggregans

<400> 24

```
Met Gly Lys Phe Pro Lys Asp Phe Met Ile Gly Tyr Ser Ser Ser Pro
1               5                   10                  15

Phe Gln Phe Glu Ala Gly Ile Pro Gly Ser Glu Asp Pro Asn Ser Asp
            20                  25                  30

Trp Trp Val Trp Val His Asp Pro Glu Asn Thr Ala Ala Gly Leu Val
        35                  40                  45

Ser Gly Asp Phe Pro Glu Asn Gly Pro Gly Tyr Trp Asn Leu Asn Gln
    50                  55                  60

Asn Asp His Asp Leu Ala Glu Lys Leu Gly Val Asn Thr Ile Arg Val
65                  70                  75                  80

Gly Val Glu Trp Ser Arg Ile Phe Pro Lys Pro Thr Phe Asn Val Lys
                85                  90                  95

Val Pro Val Glu Arg Asp Glu Asn Gly Ser Ile Val His Val Asp Val
            100                 105                 110

Asp Asp Lys Ala Val Glu Arg Leu Asp Glu Leu Ala Asn Lys Glu Ala
        115                 120                 125

Val Asn His Tyr Val Glu Met Tyr Lys Asp Trp Val Glu Arg Gly Arg
    130                 135                 140

Lys Leu Ile Leu Asn Leu Tyr His Trp Pro Leu Pro Leu Trp Leu His
145                 150                 155                 160
```

Asn Pro Ile Met Val Arg Arg Met Gly Pro Asp Arg Ala Pro Ser Gly
165 170 175

Trp Leu Asn Glu Glu Ser Val Val Glu Phe Ala Lys Tyr Ala Ala Tyr
180 185 190

Ile Ala Trp Lys Met Gly Glu Leu Pro Val Met Trp Ser Thr Met Asn
195 200 205

Glu Pro Asn Val Val Tyr Glu Gln Gly Tyr Met Phe Val Lys Gly Gly
210 215 220

Phe Pro Pro Gly Tyr Leu Ser Leu Glu Ala Ala Asp Lys Ala Arg Arg
225 230 235 240

Asn Met Ile Gln Ala His Ala Arg Ala Tyr Asp Asn Ile Lys Arg Phe
245 250 255

Ser Lys Lys Pro Val Gly Leu Ile Tyr Ala Phe Gln Trp Phe Glu Leu
260 265 270

Leu Glu Gly Pro Ala Glu Val Phe Asp Lys Phe Lys Ser Ser Lys Leu
275 280 285

Tyr Tyr Phe Thr Asp Ile Val Ser Lys Gly Ser Ser Ile Ile Asn Val
290 295 300

Glu Tyr Arg Arg Asp Leu Ala Asn Arg Leu Asp Trp Leu Gly Val Asn
305 310 315 320

Tyr Tyr Ser Arg Leu Val Tyr Lys Ile Val Asp Asp Lys Pro Ile Ile
325 330 335

Leu His Gly Tyr Gly Phe Leu Cys Thr Pro Gly Gly Ile Ser Pro Ala
340 345 350

Glu Asn Pro Cys Ser Asp Phe Gly Trp Glu Val Tyr Pro Glu Gly Leu
355 360 365

Tyr Leu Leu Leu Lys Glu Leu Tyr Asn Arg Tyr Gly Val Asp Leu Ile
370 375 380

Val Thr Glu Asn Gly Val Ser Asp Ser Arg Asp Ala Leu Arg Pro Ala
385 390 395 400

Tyr Leu Val Ser His Val Tyr Ser Val Trp Lys Ala Ala Asn Glu Gly
405 410 415

Ile Pro Val Lys Gly Tyr Leu His Trp Ser Leu Thr Asp Asn Tyr Glu
420 425 430

Trp Ala Gln Gly Phe Arg Gln Lys Phe Gly Leu Val Met Val Asp Phe
435 440 445

Lys Thr Lys Lys Arg Tyr Leu Arg Pro Ser Ala Leu Val Phe Arg Glu
450 455 460

Ile Ala Thr His Asn Gly Ile Pro Asp Glu Leu Gln His Leu Thr Leu
465 470 475 480

Ile Gln

<210> 25
<211> 1461
<212> DNA
<213> Artificial Sequence

<220>
<223> Caldivirga maquilingensis

<400> 25

atgggtatta agttcccaag cgacttcaga ttcggcttct ccacagtggg tactcagcat 60
gagatgggta cccctggttc tgaatttgta agtgactggt atgtgtggct tcatgaccct 120
gagaacattg cttcgggctt agttagcggt gatttacctg aacatgggcc aggttactgg 180
gacttgtata agcaggacca ctcaatagct agggatcttg ggcttgatgc agcatggata 240
actattgagt gggctagggt gttccctaag ccgacctttg acgttaaggt taaggttgat 300
gaggatgatg gaggtaacgt ggttgacgtt gaggttaatg aatcagcatt agaggagtta 360
cgcaggctag ctgacttaaa tgctgttaat cactataggg ggattttaag tgattggaag 420
gagaggggtg gtttactggt gattaacctt taccactggg ctatgcctac gtggcttcat 480
gacccaatag ccgttaggaa gaatggacct gatagagccc cctccggttg gcttgataag 540
agatccgtta ttgagttcac taagttcgca gccttcatag cccatgagtt aggtgactta 600
gctgacatgt ggtatacgat gaatgaacct ggggtagtga taactgaggg ttacctttac 660
gttaagtcag gcttcccacc aggttacctg gacttaaact ccctagccac tgcgggtaag 720
catttaattg aggctcatgc cagagcctac gacgccatta aagcctactc aaggaaacca 780
gtgggcctag tctactcctt cgcagactat cagccgctta ggcagggtga tgaggaggct 840
gttaaggagg ctaagggact tgactactca ttcttcgacg ctccaattaa gggtgaatta 900
atgggggtta ctagggatga cttgaagggt aggcttgact ggattggggt aaactactac 960
actagggccg tattgaggag gaggcaggat gctggtcggg catcagtagc cgtggtggat 1020
ggattcggct actcctgtga acctggaggc gtatctaatg ataggagacc atgcagtgac 1080

ttcggctggg aaatataccc tgagggtgtt tacaatgtct taatggacct atggaggagg 1140
tataggatgc ccatgtacat cactgagaac ggtatagctg atgagcatga taagtggagg 1200
tcatggttca tagtatcgca cctgtatcaa attcacaggg caatggagga gggggtggat 1260
gttagagggt acttccactg gaacctaata gataacttgg agtgggctgc aggatatagg 1320
atgaggttcg gcctagttta cgttgactat gcaaccaaga ggaggtattt taggccaagc 1380
gccctggtta tgagggaggt ggctaaacag aaggctatac cggattactt agagcattac 1440
attaaaccac ctagaattga a 1461

<210> 26
<211> 487
<212> PRT
<213> Artificial Sequence

<220>
<223> Caldivirga maquilingensis

<400> 26

```
Met Gly Ile Lys Phe Pro Ser Asp Phe Arg Phe Gly Phe Ser Thr Val
1               5                   10                  15

Gly Thr Gln His Glu Met Gly Thr Pro Gly Ser Glu Phe Val Ser Asp
            20                  25                  30

Trp Tyr Val Trp Leu His Asp Pro Glu Asn Ile Ala Ser Gly Leu Val
        35                  40                  45

Ser Gly Asp Leu Pro Glu His Gly Pro Gly Tyr Trp Asp Leu Tyr Lys
    50                  55                  60

Gln Asp His Ser Ile Ala Arg Asp Leu Gly Leu Asp Ala Ala Trp Ile
65                  70                  75                  80

Thr Ile Glu Trp Ala Arg Val Phe Pro Lys Pro Thr Phe Asp Val Lys
                85                  90                  95

Val Lys Val Asp Glu Asp Asp Gly Gly Asn Val Val Asp Val Glu Val
            100                 105                 110

Asn Glu Ser Ala Leu Glu Glu Leu Arg Arg Leu Ala Asp Leu Asn Ala
        115                 120                 125

Val Asn His Tyr Arg Gly Ile Leu Ser Asp Trp Lys Glu Arg Gly Gly
    130                 135                 140

Leu Leu Val Ile Asn Leu Tyr His Trp Ala Met Pro Thr Trp Leu His
145                 150                 155                 160
```

Asp Pro Ile Ala Val Arg Lys Asn Gly Pro Asp Arg Ala Pro Ser Gly
165 170 175

Trp Leu Asp Lys Arg Ser Val Ile Glu Phe Thr Lys Phe Ala Ala Phe
180 185 190

Ile Ala His Glu Leu Gly Asp Leu Ala Asp Met Trp Tyr Thr Met Asn
195 200 205

Glu Pro Gly Val Val Ile Thr Glu Gly Tyr Leu Tyr Val Lys Ser Gly
210 215 220

Phe Pro Pro Gly Tyr Leu Asp Leu Asn Ser Leu Ala Thr Ala Gly Lys
225 230 235 240

His Leu Ile Glu Ala His Ala Arg Ala Tyr Asp Ala Ile Lys Ala Tyr
245 250 255

Ser Arg Lys Pro Val Gly Leu Val Tyr Ser Phe Ala Asp Tyr Gln Pro
260 265 270

Leu Arg Gln Gly Asp Glu Glu Ala Val Lys Glu Ala Lys Gly Leu Asp
275 280 285

Tyr Ser Phe Phe Asp Ala Pro Ile Lys Gly Glu Leu Met Gly Val Thr
290 295 300

Arg Asp Asp Leu Lys Gly Arg Leu Asp Trp Ile Gly Val Asn Tyr Tyr
305 310 315 320

Thr Arg Ala Val Leu Arg Arg Arg Gln Asp Ala Gly Arg Ala Ser Val
325 330 335

Ala Val Val Asp Gly Phe Gly Tyr Ser Cys Glu Pro Gly Gly Val Ser
340 345 350

Asn Asp Arg Arg Pro Cys Ser Asp Phe Gly Trp Glu Ile Tyr Pro Glu
355 360 365

Gly Val Tyr Asn Val Leu Met Asp Leu Trp Arg Arg Tyr Arg Met Pro
370 375 380

Met Tyr Ile Thr Glu Asn Gly Ile Ala Asp Glu His Asp Lys Trp Arg
385 390 395 400

Ser Trp Phe Ile Val Ser His Leu Tyr Gln Ile His Arg Ala Met Glu
405 410 415

Glu Gly Val Asp Val Arg Gly Tyr Phe His Trp Asn Leu Ile Asp Asn
420 425 430

Leu Glu Trp Ala Ala Gly Tyr Arg Met Arg Phe Gly Leu Val Tyr Val
435 440 445

Asp Tyr Ala Thr Lys Arg Arg Tyr Phe Arg Pro Ser Ala Leu Val Met
450 455 460

Arg Glu Val Ala Lys Gln Lys Ala Ile Pro Asp Tyr Leu Glu His Tyr
465 470 475 480

Ile Lys Pro Pro Arg Ile Glu
485

<210> 27
<211> 1476
<212> DNA
<213> Artificial Sequence

<220>
<223> Sulfolobus acidocaldarius

<400> 27

atgggtttat cattcccaaa gggtttcaaa tttggctggt ctcagtcggg attccagtct 60
gaaatgggaa ctccaggtag cgaagatccg aacagcgatt ggcacgtctg ggttcatgac 120
agggagaaca tagtctcaca ggttgtcagt ggagatttac ccgaaaatgg tccagggtac 180
tgggggaact ataagagatt tcatgacgaa gcagagaaaa taggactaaa tgcagtgaga 240
attaacgtag agtggagtag aatatttccc agaccactac ccaagcctga aatgcaaaca 300
gggactgata aagagaacag tcctgtcatt agcgtagact taaatgagtc taagctgaga 360
gaaatggaca actacgctaa tcatgaagcg ttatcacatt acaggcaaat actggaggat 420
ctaagaaaca gaggatttca catagtactg aacatgtatc attggacttt gcccatatgg 480
ttgcacgacc ctatcagagt gaggagagga gactttacag gaccaacagg ttggttaaac 540
tccaggacag tttatgagtt cgctaggttc tcggcttacg tagcctggaa attagatgat 600
ttggcgagtg aatatgcaac aatgaatgaa cctaacgtgg tttggggagc aggttacgct 660
tttcctagag caggctttcc acctaattac cttagcttca ggctttcaga aatagctaaa 720
tggaatataa ttcaggctca tgcgagggct tatgacgcca tcaagagcgt atcaaaaaag 780
agtgtaggta taatatatgc aaacacatca tattacccac tcagaccaca agataacgaa 840
gctgtggaaa tagcagagag attgaacaga tggagtttct ttgactccat tataaaggga 900
gagataacta gtgagggaca aaatgtcaga gaggacttaa ggaacaggtt agactggatt 960
ggcgtaaact attacacgag gactgtggta acaaaagctg agagtggtta tttaaccctt 1020
ccgggttatg gagatcgttg tgaaaggaac tcattgagtt tagctaacct ccctaccagt 1080

gatttcggtt gggagttctt tcctgagggt ctatatgatg tacttttgaa gtattggaat 1140
aggtatgggt taccattata cgtaatggag aacggtatcg ctgatgacgc tgactaccaa 1200
agaccgtatt acttagtatc acatatctac caggtgcaca gggctttaaa cgagggagta 1260
gatgtaagag gttatcttca ttggtctttg gcagataatt atgagtggtc gtcaggtttt 1320
tcaatgaggt tcggtctact taaggtagat tatctaacaa agagattgta ctggagacct 1380
tctgcattag tttacaggga gattactagg agtaacggta ttcctgagga gctggaacat 1440
ctaaacagag taccaccaat aaaacctttg agacat 1476

<210> 28
<211> 492
<212> PRT
<213> Artificial Sequence

<220>
<223> Sulfolobus acidocaldarius

<400> 28

Met Gly Leu Ser Phe Pro Lys Gly Phe Lys Phe Gly Trp Ser Gln Ser
1 5 10 15

Gly Phe Gln Ser Glu Met Gly Thr Pro Gly Ser Glu Asp Pro Asn Ser
20 25 30

Asp Trp His Val Trp Val His Asp Arg Glu Asn Ile Val Ser Gln Val
35 40 45

Val Ser Gly Asp Leu Pro Glu Asn Gly Pro Gly Tyr Trp Gly Asn Tyr
50 55 60

Lys Arg Phe His Asp Glu Ala Glu Lys Ile Gly Leu Asn Ala Val Arg
65 70 75 80

Ile Asn Val Glu Trp Ser Arg Ile Phe Pro Arg Pro Leu Pro Lys Pro
85 90 95

Glu Met Gln Thr Gly Thr Asp Lys Glu Asn Ser Pro Val Ile Ser Val
100 105 110

Asp Leu Asn Glu Ser Lys Leu Arg Glu Met Asp Asn Tyr Ala Asn His
115 120 125

Glu Ala Leu Ser His Tyr Arg Gln Ile Leu Glu Asp Leu Arg Asn Arg
130 135 140

Gly Phe His Ile Val Leu Asn Met Tyr His Trp Thr Leu Pro Ile Trp
145 150 155 160

Leu His Asp Pro Ile Arg Val Arg Arg Gly Asp Phe Thr Gly Pro Thr
165 170 175

Gly Trp Leu Asn Ser Arg Thr Val Tyr Glu Phe Ala Arg Phe Ser Ala
180 185 190

Tyr Val Ala Trp Lys Leu Asp Asp Leu Ala Ser Glu Tyr Ala Thr Met
195 200 205

Asn Glu Pro Asn Val Val Trp Gly Ala Gly Tyr Ala Phe Pro Arg Ala
210 215 220

Gly Phe Pro Pro Asn Tyr Leu Ser Phe Arg Leu Ser Glu Ile Ala Lys
225 230 235 240

Trp Asn Ile Ile Gln Ala His Ala Arg Ala Tyr Asp Ala Ile Lys Ser
245 250 255

Val Ser Lys Lys Ser Val Gly Ile Ile Tyr Ala Asn Thr Ser Tyr Tyr
260 265 270

Pro Leu Arg Pro Gln Asp Asn Glu Ala Val Glu Ile Ala Glu Arg Leu
275 280 285

Asn Arg Trp Ser Phe Phe Asp Ser Ile Ile Lys Gly Glu Ile Thr Ser
290 295 300

Glu Gly Gln Asn Val Arg Glu Asp Leu Arg Asn Arg Leu Asp Trp Ile
305 310 315 320

Gly Val Asn Tyr Tyr Thr Arg Thr Val Val Thr Lys Ala Glu Ser Gly
325 330 335

Tyr Leu Thr Leu Pro Gly Tyr Gly Asp Arg Cys Glu Arg Asn Ser Leu
340 345 350

Ser Leu Ala Asn Leu Pro Thr Ser Asp Phe Gly Trp Glu Phe Phe Pro
355 360 365

Glu Gly Leu Tyr Asp Val Leu Leu Lys Tyr Trp Asn Arg Tyr Gly Leu
370 375 380

Pro Leu Tyr Val Met Glu Asn Gly Ile Ala Asp Asp Ala Asp Tyr Gln
385 390 395 400

Arg Pro Tyr Tyr Leu Val Ser His Ile Tyr Gln Val His Arg Ala Leu
405 410 415

Asn Glu Gly Val Asp Val Arg Gly Tyr Leu His Trp Ser Leu Ala Asp

420 425 430

Asn Tyr Glu Trp Ser Ser Gly Phe Ser Met Arg Phe Gly Leu Leu Lys
435 440 445

Val Asp Tyr Leu Thr Lys Arg Leu Tyr Trp Arg Pro Ser Ala Leu Val
450 455 460

Tyr Arg Glu Ile Thr Arg Ser Asn Gly Ile Pro Glu Glu Leu Glu His
465 470 475 480

Leu Asn Arg Val Pro Pro Ile Lys Pro Leu Arg His
485 490

<210> 29
<211> 1470
<212> DNA
<213> Artificial Sequence

<220>
<223> Sulfolobus solfataricus

<400> 29

atgggttact catttccaaa tagctttagg tttggttggt cccaggccgg atttcaatca 60
gaaatgggaa caccagggtc agaagatcca aatactgact ggtataaatg ggttcatgat 120
ccagaaaaca tggcagcggg attagtaagt ggagatctac cagaaaatgg gccaggctac 180
tggggaaact ataagacatt tcacgataat gcacaaaaaa tgggattaaa aatagctaga 240
ctaaatgtgg aatggtctag gatatttcct aatccattac caaggccaca aaactttgat 300
gaatcaaaac aagatgtgac agaggttgag ataaacgaaa acgagttaaa gagacttgac 360
gagtacgcta ataaagacgc attaaaccat tacagggaaa tattcaagga tcttaaaagt 420
agaggacttt actttatact aaacatgtat cattggccat tacctctatg gttacacgac 480
ccaataagag taagaagagg agattttact ggaccaagtg gttggctaag tactagaaca 540
gtttacgagt tcgctagatt ctcagcttat atagcttgga aattcgatga tctagtggat 600
gagtactcaa caatgaatga acctaacgtt gttggaggtt taggatacgt tggtgttaag 660
tccggttttc ccccaggata cctaagcttt gaactttccc gtagggcaat gtataacatc 720
attcaagctc acgcaagagc gtatgatggg ataaagagtg tttctaaaaa accagttgga 780
attatttacg ctaatagctc attccagccg ttaacggata aagatatgga agcggtagag 840
atggctgaaa atgataatag atggtggttc tttgatgcta taataagagg tgagatcacc 900
agaggaaacg agaagattgt aagagatgac ctaaagggta gattggattg gattggagtt 960
aattattaca ctaggactgt tgtgaagagg actgaaaagg gatacgttag cttaggaggt 1020
tacggtcacg gatgtgagag gaactctgta agtttagcgg gattaccaac cagcgacttc 1080
ggctgggagt tcttcccaga aggtttatat gacgttttga cgaaatactg gaatagatat 1140

catctctata tgtacgttac tgaaaatggt attgcggatg atgccgatta tcaaaggccc 1200
tattatttag tatctcacgt ttatcaagtt catagagcaa taaatagtgg tgcagatgtt 1260
agagggtatt tacattggtc tctagctgat aattacgaat gggcttcagg attctctatg 1320
aggtttggtc tgttaaaggt cgattacaac actaagagac tatactggag accctcagca 1380
ctagtatata gggaaatcgc cacaaatggc gcaataactg atgaaataga gcacttaaat 1440
agcgtacctc cagtaaagcc attaaggcac 1470

<210> 30
<211> 490
<212> PRT
<213> Artificial Sequence

<220>
<223> Sulfolobus solfataricus

<400> 30

Met Gly Tyr Ser Phe Pro Asn Ser Phe Arg Phe Gly Trp Ser Gln Ala
1 5 10 15

Gly Phe Gln Ser Glu Met Gly Thr Pro Gly Ser Glu Asp Pro Asn Thr
20 25 30

Asp Trp Tyr Lys Trp Val His Asp Pro Glu Asn Met Ala Ala Gly Leu
35 40 45

Val Ser Gly Asp Leu Pro Glu Asn Gly Pro Gly Tyr Trp Gly Asn Tyr
50 55 60

Lys Thr Phe His Asp Asn Ala Gln Lys Met Gly Leu Lys Ile Ala Arg
65 70 75 80

Leu Asn Val Glu Trp Ser Arg Ile Phe Pro Asn Pro Leu Pro Arg Pro
85 90 95

Gln Asn Phe Asp Glu Ser Lys Gln Asp Val Thr Glu Val Glu Ile Asn
100 105 110

Glu Asn Glu Leu Lys Arg Leu Asp Glu Tyr Ala Asn Lys Asp Ala Leu
115 120 125

Asn His Tyr Arg Glu Ile Phe Lys Asp Leu Lys Ser Arg Gly Leu Tyr
130 135 140

Phe Ile Leu Asn Met Tyr His Trp Pro Leu Pro Leu Trp Leu His Asp
145 150 155 160

Pro Ile Arg Val Arg Arg Gly Asp Phe Thr Gly Pro Ser Gly Trp Leu

165 170 175

Ser Thr Arg Thr Val Tyr Glu Phe Ala Arg Phe Ser Ala Tyr Ile Ala
180 185 190

Trp Lys Phe Asp Asp Leu Val Asp Glu Tyr Ser Thr Met Asn Glu Pro
195 200 205

Asn Val Val Gly Gly Leu Gly Tyr Val Gly Val Lys Ser Gly Phe Pro
210 215 220

Pro Gly Tyr Leu Ser Phe Glu Leu Ser Arg Arg Ala Met Tyr Asn Ile
225 230 235 240

Ile Gln Ala His Ala Arg Ala Tyr Asp Gly Ile Lys Ser Val Ser Lys
245 250 255

Lys Pro Val Gly Ile Ile Tyr Ala Asn Ser Ser Phe Gln Pro Leu Thr
260 265 270

Asp Lys Asp Met Glu Ala Val Glu Met Ala Glu Asn Asp Asn Arg Trp
275 280 285

Trp Phe Phe Asp Ala Ile Ile Arg Gly Glu Ile Thr Arg Gly Asn Glu
290 295 300

Lys Ile Val Arg Asp Asp Leu Lys Gly Arg Leu Asp Trp Ile Gly Val
305 310 315 320

Asn Tyr Tyr Thr Arg Thr Val Val Lys Arg Thr Glu Lys Gly Tyr Val
325 330 335

Ser Leu Gly Gly Tyr Gly His Gly Cys Glu Arg Asn Ser Val Ser Leu
340 345 350

Ala Gly Leu Pro Thr Ser Asp Phe Gly Trp Glu Phe Phe Pro Glu Gly
355 360 365

Leu Tyr Asp Val Leu Thr Lys Tyr Trp Asn Arg Tyr His Leu Tyr Met
370 375 380

Tyr Val Thr Glu Asn Gly Ile Ala Asp Asp Ala Asp Tyr Gln Arg Pro
385 390 395 400

Tyr Tyr Leu Val Ser His Val Tyr Gln Val His Arg Ala Ile Asn Ser
405 410 415

Gly Ala Asp Val Arg Gly Tyr Leu His Trp Ser Leu Ala Asp Asn Tyr
420 425 430

Glu Trp Ala Ser Gly Phe Ser Met Arg Phe Gly Leu Leu Lys Val Asp
435 440 445

Tyr Asn Thr Lys Arg Leu Tyr Trp Arg Pro Ser Ala Leu Val Tyr Arg
450 455 460

Glu Ile Ala Thr Asn Gly Ala Ile Thr Asp Glu Ile Glu His Leu Asn
465 470 475 480

Ser Val Pro Pro Val Lys Pro Leu Arg His
485 490

<210> 31
<211> 1488
<212> DNA
<213> Artificial Sequence

<220>
<223> Picrophilus torridus

<400> 31

```
atgggtttac ccaagaactt tttacttggc ttttctctgg ctggctttca gtctgaaatg     60
ggcatatcag atcctgatag caattcagat tggtggttat gggtacatga cccggtgaat    120
ataaggactg gacttgtatc tggtgactta cctgaaaatg gaataggata ctgggatctt    180
tacaaaaaat ataatggtct ggctgttcaa acaggaatga atgctgcaag gcttggagtt    240
gaatggagca ggatatttcc aaaaagtact gaagaagtaa aggtgatgga agattacaaa    300
gatgatgatt taatttccgt ggatgttaat gagggaagtc ttgaaaaact tgacagactg    360
gcaaatcaaa aggcaattaa tagatatatg gaaatcttca ataatatcaa ggaaaataat    420
atgacgctaa tagtgaatgt ttaccattgg ccaataccaa tatatcttca cgatccaata    480
gaagctagga atagtggact ttcaaataaa agaaatggct ggcttaatca taaaaccgtt    540
gtggaatttg taaaatatgc aaaatatctg gcatggaaat ttagcgatgt ggcagatatg    600
ttttctataa tgaatgagcc aaacgttgta tttggtaatg gatattttaa tgttaaatca    660
gggttcccac cagcatttcc aagtgtgcat ggcggtttgc ttgcaaaaaa acatgaaatt    720
gaggctatag caagatcata cgacgccatg aaggagatta caaaaaaacc agttggtcta    780
attatggcaa attcagatgt acaaccacta acagatgagg ataaagaagc agcagaaatg    840
gctacttaca atgatcgcta ttcattcata gatccgctaa gagttggtga gatgaaatgg    900
gctgatgagg ttactgcagg taatccaatt ggtgaaaaga gcaacatcga tagatctgat    960
ctaaaaaata agctagactg gataggtgtt aactattata caagggccgt tgtaaaaaaa   1020
tctggaaacg gatatacaac attaaaagga tatggacact ctgcaaccgc tggcatgcca   1080
agtagggccg gaagggatgt aagtgacttt ggctgggaat tttatccaga aggtcttgta   1140

aacgtcttat catcatactg gaaaagatat cacattccaa tgattgtgac tgaaaatggt   1200
gttgctgact ctattgatag acttagacca aggtaccttg tgtcacatat aaagtctgtt   1260
gaaaaggctt tatctatggg tatggatatt aggggatatc ttcactggtc tctgattgat   1320
aactatgaat gggcatcagg tttttcaatg aaatttgggc tttatggtat tgatttgaac   1380
aataaaaaga ttcaacacag accaagtgca ctggtattta aagaaattgc aaatgccaac   1440
ggagtcccgg aggaatttga atggatggca gaccagcatc agaactca                 1488
```

<210> 32
<211> 496
<212> PRT
<213> Artificial Sequence

<220>
<223> Picrophilus torridus

<400> 32

Met Gly Leu Pro Lys Asn Phe Leu Leu Gly Phe Ser Leu Ala Gly Phe
1 5 10 15

Gln Ser Glu Met Gly Ile Ser Asp Pro Asp Ser Asn Ser Asp Trp Trp
20 25 30

Leu Trp Val His Asp Pro Val Asn Ile Arg Thr Gly Leu Val Ser Gly
35 40 45

Asp Leu Pro Glu Asn Gly Ile Gly Tyr Trp Asp Leu Tyr Lys Lys Tyr
50 55 60

Asn Gly Leu Ala Val Gln Thr Gly Met Asn Ala Ala Arg Leu Gly Val
65 70 75 80

Glu Trp Ser Arg Ile Phe Pro Lys Ser Thr Glu Glu Val Lys Val Met
85 90 95

Glu Asp Tyr Lys Asp Asp Asp Leu Ile Ser Val Asp Val Asn Glu Gly
100 105 110

Ser Leu Glu Lys Leu Asp Arg Leu Ala Asn Gln Lys Ala Ile Asn Arg
115 120 125

Tyr Met Glu Ile Phe Asn Asn Ile Lys Glu Asn Asn Met Thr Leu Ile
130 135 140

Val Asn Val Tyr His Trp Pro Ile Pro Ile Tyr Leu His Asp Pro Ile
145 150 155 160

Glu Ala Arg Asn Ser Gly Leu Ser Asn Lys Arg Asn Gly Trp Leu Asn
165 170 175

His Lys Thr Val Val Glu Phe Val Lys Tyr Ala Lys Tyr Leu Ala Trp
180 185 190

Lys Phe Ser Asp Val Ala Asp Met Phe Ser Ile Met Asn Glu Pro Asn
195 200 205

Val Val Phe Gly Asn Gly Tyr Phe Asn Val Lys Ser Gly Phe Pro Pro
210 215 220

Ala Phe Pro Ser Val His Gly Gly Leu Leu Ala Lys Lys His Glu Ile
225 230 235 240

Glu Ala Ile Ala Arg Ser Tyr Asp Ala Met Lys Glu Ile Thr Lys Lys
245 250 255

Pro Val Gly Leu Ile Met Ala Asn Ser Asp Val Gln Pro Leu Thr Asp
260 265 270

Glu Asp Lys Glu Ala Ala Glu Met Ala Thr Tyr Asn Asp Arg Tyr Ser
275 280 285

Phe Ile Asp Pro Leu Arg Val Gly Glu Met Lys Trp Ala Asp Glu Val
290 295 300

Thr Ala Gly Asn Pro Ile Gly Glu Lys Ser Asn Ile Asp Arg Ser Asp
305 310 315 320

Leu Lys Asn Lys Leu Asp Trp Ile Gly Val Asn Tyr Tyr Thr Arg Ala
325 330 335

Val Val Lys Lys Ser Gly Asn Gly Tyr Thr Thr Leu Lys Gly Tyr Gly
340 345 350

His Ser Ala Thr Ala Gly Met Pro Ser Arg Ala Gly Arg Asp Val Ser
355 360 365

Asp Phe Gly Trp Glu Phe Tyr Pro Glu Gly Leu Val Asn Val Leu Ser
370 375 380

Ser Tyr Trp Lys Arg Tyr His Ile Pro Met Ile Val Thr Glu Asn Gly
385 390 395 400

Val Ala Asp Ser Ile Asp Arg Leu Arg Pro Arg Tyr Leu Val Ser His
405 410 415

Ile Lys Ser Val Glu Lys Ala Leu Ser Met Gly Met Asp Ile Arg Gly
420 425 430

Tyr Leu His Trp Ser Leu Ile Asp Asn Tyr Glu Trp Ala Ser Gly Phe
435 440 445

Ser Met Lys Phe Gly Leu Tyr Gly Ile Asp Leu Asn Asn Lys Lys Ile
450 455 460

Gln His Arg Pro Ser Ala Leu Val Phe Lys Glu Ile Ala Asn Ala Asn
465 470 475 480

Gly Val Pro Glu Glu Phe Glu Trp Met Ala Asp Gln His Gln Asn Ser
485 490 495

<210> 33
<211> 1443
<212> DNA
<213> Artificial Sequence

<220>
<223> Thermoplasma volcanium

<400> 33

```
atggtagaaa acaattttcc agaggatttc aaatttggtt ggtcacagtc aggttttcaa      60
tcggagatgg gctatgataa cgcaatggac gataaaagtg actggtatgt ctgggttcat     120
gataaagaaa acatccaatc agggcttgta agcggagaca tgcccgaaaa tggtccgggt     180
tactggaata actataaatc attccatgaa gctgcacaga atatgggatt aaaaatggca     240
agaatcggag ttgaatggtc aagattattc ccggaacctt tcccggaaaa aataatggca     300
gatgcaaaaa ataattcctt agaaataaac aataacattc tttcagaact tgataaatat     360
gtcaataaag atgcactcaa ccattacatt gagatattta atgatatcaa aaatagaaat     420
atagatttaa taattaatat gtaccactgg ccacttcctg tatggctaag cgatcctgta     480
tctgttagaa aaggaataaa aacagaaaga tcaggctggc tgaatgacag gatagttcaa     540
ttgtttgctt tattctcctc gtatatagta tataaaatgg aagatctggc agttgcattt     600
tcaaccatga atgaacctaa tgttgtttat ggaaatggtt ttataaatat caaatcaggt     660
tttccgcctt cctatctcag ttcagaattt gcatctaaag ttaagaacaa tatattaaaa     720
gcacattctc ttgcatacga ttctatgaaa aaaattacgg ataaacctgt gggaataatt     780
tatgcaaaca catattttac gcctttggac ccggaaaaag ataatgatgc tattgctaaa     840
gcagacagtg atgcgaaatg gtcatttttt gatccattaa taaaaggaga taaatcactt     900
ggaattaatg gcaataaact agattggatc ggaattaatt attatacaag gacaatgtta     960
aggaaagacg gagatggcta tatttcatta aaaggctatg gtcattcagg ttctcctaat    1020
actgtaacaa acgataaaag accaacaagt gatataggat gggaatttta tccggaggga    1080
ttggaatatg taattatgaa ttactggaac aggtataaat tgcctatgta cgtaacagaa    1140
aatggcatag ccgataatgg ggattatcag aggccttatt atttagtttc acacattgca    1200

agtgtactga gggcaataaa taaaggagcc aatgtaaagg gttatttgca ctggtcctta    1260
gttgataatt atgaatgggc attgggattt agcccgaaat ttggtttaat aggatacgat    1320
gaaaataaaa aactatactg gaggccaagt gctcttgttt ataaggaaat agcaacaaaa    1380
aattgcatat ccccagaatt aaagcacctc gattcaatac cgcctataaa tggtttaaga    1440
aaa                                                                  1443
```

<210> 34
<211> 481
<212> PRT
<213> Artificial Sequence

<220>
<223> Thermoplasma volcanium

<400> 34

```
Met Val Glu Asn Asn Phe Pro Glu Asp Phe Lys Phe Gly Trp Ser Gln
1               5                   10                  15

Ser Gly Phe Gln Ser Glu Met Gly Tyr Asp Asn Ala Met Asp Asp Lys
            20                  25                  30

Ser Asp Trp Tyr Val Trp Val His Asp Lys Glu Asn Ile Gln Ser Gly
        35                  40                  45

Leu Val Ser Gly Asp Met Pro Glu Asn Gly Pro Gly Tyr Trp Asn Asn
    50                  55                  60

Tyr Lys Ser Phe His Glu Ala Ala Gln Asn Met Gly Leu Lys Met Ala
65                  70                  75                  80

Arg Ile Gly Val Glu Trp Ser Arg Leu Phe Pro Glu Pro Phe Pro Glu
                85                  90                  95

Lys Ile Met Ala Asp Ala Lys Asn Asn Ser Leu Glu Ile Asn Asn Asn
            100                 105                 110

Ile Leu Ser Glu Leu Asp Lys Tyr Val Asn Lys Asp Ala Leu Asn His
        115                 120                 125

Tyr Ile Glu Ile Phe Asn Asp Ile Lys Asn Arg Asn Ile Asp Leu Ile
    130                 135                 140

Ile Asn Met Tyr His Trp Pro Leu Pro Val Trp Leu Ser Asp Pro Val
145                 150                 155                 160

Ser Val Arg Lys Gly Ile Lys Thr Glu Arg Ser Gly Trp Leu Asn Asp
                165                 170                 175
```

Arg Ile Val Gln Leu Phe Ala Leu Phe Ser Ser Tyr Ile Val Tyr Lys
180 185 190

Met Glu Asp Leu Ala Val Ala Phe Ser Thr Met Asn Glu Pro Asn Val
195 200 205

Val Tyr Gly Asn Gly Phe Ile Asn Ile Lys Ser Gly Phe Pro Pro Ser
210 215 220

Tyr Leu Ser Ser Glu Phe Ala Ser Lys Val Lys Asn Asn Ile Leu Lys
225 230 235 240

Ala His Ser Leu Ala Tyr Asp Ser Met Lys Lys Ile Thr Asp Lys Pro
245 250 255

Val Gly Ile Ile Tyr Ala Asn Thr Tyr Phe Thr Pro Leu Asp Pro Glu
260 265 270

Lys Asp Asn Asp Ala Ile Ala Lys Ala Asp Ser Asp Ala Lys Trp Ser
275 280 285

Phe Phe Asp Pro Leu Ile Lys Gly Asp Lys Ser Leu Gly Ile Asn Gly
290 295 300

Asn Lys Leu Asp Trp Ile Gly Ile Asn Tyr Tyr Thr Arg Thr Met Leu
305 310 315 320

Arg Lys Asp Gly Asp Gly Tyr Ile Ser Leu Lys Gly Tyr Gly His Ser
325 330 335

Gly Ser Pro Asn Thr Val Thr Asn Asp Lys Arg Pro Thr Ser Asp Ile
340 345 350

Gly Trp Glu Phe Tyr Pro Glu Gly Leu Glu Tyr Val Ile Met Asn Tyr
355 360 365

Trp Asn Arg Tyr Lys Leu Pro Met Tyr Val Thr Glu Asn Gly Ile Ala
370 375 380

Asp Asn Gly Asp Tyr Gln Arg Pro Tyr Tyr Leu Val Ser His Ile Ala
385 390 395 400

Ser Val Leu Arg Ala Ile Asn Lys Gly Ala Asn Val Lys Gly Tyr Leu
405 410 415

His Trp Ser Leu Val Asp Asn Tyr Glu Trp Ala Leu Gly Phe Ser Pro
420 425 430

Lys Phe Gly Leu Ile Gly Tyr Asp Glu Asn Lys Lys Leu Tyr Trp Arg
435 440 445

Pro Ser Ala Leu Val Tyr Lys Glu Ile Ala Thr Lys Asn Cys Ile Ser
450 455 460

Pro Glu Leu Lys His Leu Asp Ser Ile Pro Pro Ile Asn Gly Leu Arg
465 470 475 480

Lys

<210> 35
<211> 1404
<212> DNA
<213> Artificial Sequence

<220>
<223> Fervidobacterium nodosum

<400> 35

atgggtatgt ttccgaaaga ttttttattt ggtgtttcga tgtctgggtt tcagtttgag 60
atgggaaatc ctcaagatgc agaagaggtt gatctaaata cagattggta tgtatgggtt 120
agggatattg gaaatattgt aaatggagtc gtaagtgggg acttgcctga aaatggttca 180
tggtactgga agcagtacgg caaagtccac caattagctg ccgattttgg gatggatgta 240
atacgaattg gaaccgaatg gtctaggatt ttcccagtta gtacgcaaag tgttgagtac 300
ggctcaccgg atatgctcga aaaattggat aaattagcaa accaaaaagc ggtaagtcat 360
tacaggaaaa taatggagga tataaaagca aaggggttaa aattgttcgt taacctttac 420
cactttactt tacctatttg gttgcacgac cctatagctg ttcacaaagg tgagaagaca 480
gataaaattg gttggatttc tgatgctaca cctattgagt ttgcgaagta tgcagagtac 540
atggcgtgga aatttgccga tatagttgat atgtgggctt ctatgaacga accacacgtt 600
gtaagtcagc ttggatattt tgcaataaat gcgggatttc caccaagtta ttttaatcct 660
tcatggtata tcaaaagttt agaaaacgaa gcgaaagcac ataacttatc ttatgatgct 720
ataaaaaagt atacaaataa tcctgttgga gttatatact cttttacatg gtacgatact 780
gttaataaag atgacaagga atcttttgaa aatgctatgg atctcacaaa ttggcgattt 840
atagatatgg taaaagataa aactgattac ataggtgtaa attattacac aagagcggtt 900
atcgatagac ttcccaccac tattgacttt ggcgaattta aaatgaattg gtatactttg 960
agaggttacg gttattcttg cgaagaagga ggattctcac tctccggaag gccggcaagc 1020
gaatttggat gggaaatata ccctgaaggg ctgtacaata ttttgataca tgtttataat 1080
agatacaaaa aagatattta tgttacggag aacggtatag ctgattcgaa ggataaatac 1140
agaagtcttt ttatcatatc gcatctttat gctatagaaa aagcattaaa cgaaggaata 1200
ccaataaaag gttatttgca ctggtcgatt atagacaatt tcgaatgggc gaagggctac 1260
agtaaaagat ttggacttgc ttacacagat ttgtcaacca aaaaatatat acctagacct 1320
tctatgtaca tttttagaga gataataaag gataaatcaa tcgacaaatt caaaggttac 1380
gatccatata acttgatgaa attc 1404

<210> 36
<211> 468
<212> PRT
<213> Artificial Sequence

<220>
<223> Fervidobacterium nodosum

<400> 36

Met Gly Met Phe Pro Lys Asp Phe Leu Phe Gly Val Ser Met Ser Gly
1               5                   10                  15

Phe Gln Phe Glu Met Gly Asn Pro Gln Asp Ala Glu Glu Val Asp Leu
            20                  25                  30

Asn Thr Asp Trp Tyr Val Trp Val Arg Asp Ile Gly Asn Ile Val Asn
        35                  40                  45

Gly Val Val Ser Gly Asp Leu Pro Glu Asn Gly Ser Trp Tyr Trp Lys
    50                  55                  60

Gln Tyr Gly Lys Val His Gln Leu Ala Ala Asp Phe Gly Met Asp Val
65                  70                  75                  80

Ile Arg Ile Gly Thr Glu Trp Ser Arg Ile Phe Pro Val Ser Thr Gln
                85                  90                  95

Ser Val Glu Tyr Gly Ser Pro Asp Met Leu Glu Lys Leu Asp Lys Leu
            100                 105                 110

Ala Asn Gln Lys Ala Val Ser His Tyr Arg Lys Ile Met Glu Asp Ile
        115                 120                 125

Lys Ala Lys Gly Leu Lys Leu Phe Val Asn Leu Tyr His Phe Thr Leu
    130                 135                 140

Pro Ile Trp Leu His Asp Pro Ile Ala Val His Lys Gly Glu Lys Thr
145                 150                 155                 160

Asp Lys Ile Gly Trp Ile Ser Asp Ala Thr Pro Ile Glu Phe Ala Lys
                165                 170                 175

Tyr Ala Glu Tyr Met Ala Trp Lys Phe Ala Asp Ile Val Asp Met Trp
            180                 185                 190

Ala Ser Met Asn Glu Pro His Val Val Ser Gln Leu Gly Tyr Phe Ala
195 200 205

Ile Asn Ala Gly Phe Pro Pro Ser Tyr Phe Asn Pro Ser Trp Tyr Ile
210 215 220

Lys Ser Leu Glu Asn Glu Ala Lys Ala His Asn Leu Ser Tyr Asp Ala
225 230 235 240

Ile Lys Lys Tyr Thr Asn Asn Pro Val Gly Val Ile Tyr Ser Phe Thr
245 250 255

Trp Tyr Asp Thr Val Asn Lys Asp Asp Lys Glu Ser Phe Glu Asn Ala
260 265 270

Met Asp Leu Thr Asn Trp Arg Phe Ile Asp Met Val Lys Asp Lys Thr
275 280 285

Asp Tyr Ile Gly Val Asn Tyr Tyr Thr Arg Ala Val Ile Asp Arg Leu
290 295 300

Pro Thr Thr Ile Asp Phe Gly Glu Phe Lys Met Asn Trp Tyr Thr Leu
305 310 315 320

Arg Gly Tyr Gly Tyr Ser Cys Glu Glu Gly Gly Phe Ser Leu Ser Gly
325 330 335

Arg Pro Ala Ser Glu Phe Gly Trp Glu Ile Tyr Pro Glu Gly Leu Tyr
340 345 350

Asn Ile Leu Ile His Val Tyr Asn Arg Tyr Lys Lys Asp Ile Tyr Val
355 360 365

Thr Glu Asn Gly Ile Ala Asp Ser Lys Asp Lys Tyr Arg Ser Leu Phe
370 375 380

Ile Ile Ser His Leu Tyr Ala Ile Glu Lys Ala Leu Asn Glu Gly Ile
385 390 395 400

Pro Ile Lys Gly Tyr Leu His Trp Ser Ile Ile Asp Asn Phe Glu Trp
405 410 415

Ala Lys Gly Tyr Ser Lys Arg Phe Gly Leu Ala Tyr Thr Asp Leu Ser
420 425 430

Thr Lys Lys Tyr Ile Pro Arg Pro Ser Met Tyr Ile Phe Arg Glu Ile
435 440 445

Ile Lys Asp Lys Ser Ile Asp Lys Phe Lys Gly Tyr Asp Pro Tyr Asn
450 455 460

Leu Met Lys Phe
465

<210> 37
<211> 1446
<212> DNA
<213> Artificial Sequence

<220>
<223> DNA encording mutant beta-glycosidase of Thermosphaera aggregans having glycosylation site.

<400> 37

atgggtaaat tccccaaaga cttcatgata ggctactcat cttcaccgtt tcaatttgaa 60
gctggtattc ccgggtccga agatccgaat agtgattggt gggtatgggt gcatgatccg 120
gagaacacag cagctggact agtcagcggc gattttcccg agaacggccc aggttactgg 180
aaccgcactc aaaatgacca cgacctggct gagaagctgg gggttaacac tattagagta 240
ggcgttgagt ggagtaggat ttttccaaag ccaactttca atgttaaagt ccctgtagag 300
agagatgaga acggcagcat tgttcacgta gatgtcgatg ataaagcggt tgaaagactt 360
gatgaattag ccaacaagga ggccgtaaac cattacgtag aaatgtataa agactgggtt 420
gaaagaggta gaaaacttat actcaattta taccattggc cctgcctct ctggcttcac 480
aacccaatca tggtgagaag aatgggcccg gacagagcgc cctcaggctg gcttaacgag 540
gagtccgtgg tggagtttgc caaatacgcc gcatacattg cttggaaaat gggcgagcta 600
cctgttatgt ggagcaccat gaacgaaccc aacgtcgttt atgagcaagg atacatgttc 660
gttaaagggg gtttcccacc cggctacttg agtttggaag ctgctgataa ggccaggaga 720
aatatgatcc aggctcatgc acgggcctat gacaatatta aacgcttcag taagaaacct 780
gttggactaa tatacgcttt ccaatggttc gaactattag agggtccagc agaagtattt 840
gataagttta agagctctaa gttatactat ttcacagaca tagtatcgaa gggtagttca 900
atcatcaatg ttgaatacag gagagatctt gccaataggc tagactggtt gggcgttaac 960
tactatagcc gtttagtcta caaaatcgtc gatgacaaac ctataatcct gcacgggtat 1020
ggattccttt gtacacctgg ggggatcagc ccggctgaaa atccttgtag cgattttggg 1080
tgggaggtgt atcctgaagg actctaccta cttctaaaag aactttacaa ccgatacggg 1140
gtagacttga tcgtgaccga gaacggtgtt tcagacagca gggatgcgtt gagaccggca 1200
tacctggtct cgcatgttta cagcgtatgg aaagccgcta acgagggcat tcccgtcaaa 1260
ggctacctcc actggagctt gacagacaat tacgagtggg cccagggctt caggcagaaa 1320
ttcggtttag tcatggttga cttcaaaact aagaaaaggt atctccgccc aagcgcccta 1380

gtgttccggg agatcgcaac gcataacgga ataccggatg agctacagca tcttacactg 1440
atccag 1446

<210> 38
<211> 482
<212> PRT
<213> Artificial Sequence

<220>
<223> Mutant beta-glycosidase of Thermosphaera aggregans having glycosy lation site.

<220>
<221> CARBOHYD

<222> (61)..(63)
<223>

<400> 38

```
Met Gly Lys Phe Pro Lys Asp Phe Met Ile Gly Tyr Ser Ser Ser Pro
1               5                   10                  15

Phe Gln Phe Glu Ala Gly Ile Pro Gly Ser Glu Asp Pro Asn Ser Asp
            20                  25                  30

Trp Trp Val Trp Val His Asp Pro Glu Asn Thr Ala Ala Gly Leu Val
        35                  40                  45

Ser Gly Asp Phe Pro Glu Asn Gly Pro Gly Tyr Trp Asn Arg Thr Gln
    50                  55                  60

Asn Asp His Asp Leu Ala Glu Lys Leu Gly Val Asn Thr Ile Arg Val
65                  70                  75                  80

Gly Val Glu Trp Ser Arg Ile Phe Pro Lys Pro Thr Phe Asn Val Lys
                85                  90                  95

Val Pro Val Glu Arg Asp Glu Asn Gly Ser Ile Val His Val Asp Val
            100                 105                 110

Asp Asp Lys Ala Val Glu Arg Leu Asp Glu Leu Ala Asn Lys Glu Ala
        115                 120                 125

Val Asn His Tyr Val Glu Met Tyr Lys Asp Trp Val Glu Arg Gly Arg
    130                 135                 140

Lys Leu Ile Leu Asn Leu Tyr His Trp Pro Leu Pro Leu Trp Leu His
145                 150                 155                 160

Asn Pro Ile Met Val Arg Arg Met Gly Pro Asp Arg Ala Pro Ser Gly
                165                 170                 175
```

Trp Leu Asn Glu Glu Ser Val Val Glu Phe Ala Lys Tyr Ala Ala Tyr
180 185 190

Ile Ala Trp Lys Met Gly Glu Leu Pro Val Met Trp Ser Thr Met Asn
195 200 205

Glu Pro Asn Val Val Tyr Glu Gln Gly Tyr Met Phe Val Lys Gly Gly
210 215 220

Phe Pro Pro Gly Tyr Leu Ser Leu Glu Ala Ala Asp Lys Ala Arg Arg
225 230 235 240

Asn Met Ile Gln Ala His Ala Arg Ala Tyr Asp Asn Ile Lys Arg Phe
245 250 255

Ser Lys Lys Pro Val Gly Leu Ile Tyr Ala Phe Gln Trp Phe Glu Leu
260 265 270

Leu Glu Gly Pro Ala Glu Val Phe Asp Lys Phe Lys Ser Ser Lys Leu
275 280 285

Tyr Tyr Phe Thr Asp Ile Val Ser Lys Gly Ser Ser Ile Ile Asn Val
290 295 300

Glu Tyr Arg Arg Asp Leu Ala Asn Arg Leu Asp Trp Leu Gly Val Asn
305 310 315 320

Tyr Tyr Ser Arg Leu Val Tyr Lys Ile Val Asp Asp Lys Pro Ile Ile
325 330 335

Leu His Gly Tyr Gly Phe Leu Cys Thr Pro Gly Gly Ile Ser Pro Ala
340 345 350

Glu Asn Pro Cys Ser Asp Phe Gly Trp Glu Val Tyr Pro Glu Gly Leu
355 360 365

Tyr Leu Leu Leu Lys Glu Leu Tyr Asn Arg Tyr Gly Val Asp Leu Ile
370 375 380

Val Thr Glu Asn Gly Val Ser Asp Ser Arg Asp Ala Leu Arg Pro Ala
385 390 395 400

Tyr Leu Val Ser His Val Tyr Ser Val Trp Lys Ala Ala Asn Glu Gly
405 410 415

Ile Pro Val Lys Gly Tyr Leu His Trp Ser Leu Thr Asp Asn Tyr Glu
420 425 430

Trp Ala Gln Gly Phe Arg Gln Lys Phe Gly Leu Val Met Val Asp Phe

435 440 445

Lys Thr Lys Lys Arg Tyr Leu Arg Pro Ser Ala Leu Val Phe Arg Glu
450 455 460

Ile Ala Thr His Asn Gly Ile Pro Asp Glu Leu Gln His Leu Thr Leu
465 470 475 480

Ile Gln

<210> 39
<211> 1461
<212> DNA
<213> Artificial Sequence

<220>
<223> DNA encording mutant glycoside hydrolase family protein of Caldiv irga maquilingensis having glycosylation site.

<400> 39

```
atgggtatta agttcccaag cgacttcaga ttcggcttct ccacagtggg tactcagcat      60
gagatgggta cccctggttc tgaatttgta agtgactggt atgtgtggct tcatgaccct     120
gagaacattg cttcgggctt agttagcggt gatttacctg aacatgggcc aggttactgg     180
aaccgcacta agcaggacca ctcaatagct agggatcttg ggcttgatgc agcatggata     240
actattgagt gggctagggt gttccctaag ccgacctttg acgttaaggt taaggttgat     300
gaggatgatg gaggtaacgt ggttgacgtt gaggttaatg aatcagcatt agaggagtta     360
cgcaggctag ctgacttaaa tgctgttaat cactataggg ggattttaag tgattggaag     420
gagaggggtg gtttactggt gattaacctt taccactggg ctatgcctac gtggcttcat     480
gacccaatag ccgttaggaa gaatggacct gatagagccc cctccggttg gcttgataag     540
agatccgtta ttgagttcac taagttcgca gccttcatag cccatgagtt aggtgactta     600
gctgacatgt ggtatacgat gaatgaacct ggggtagtga taactgaggg ttacctttac     660
gttaagtcag gcttcccacc aggttacctg gacttaaact ccctagccac tgcgggtaag     720
catttaattg aggctcatgc cagagcctac gacgccatta aagcctactc aaggaaacca     780
gtgggcctag tctactcctt cgcagactat cagccgctta ggcagggtga tgaggaggct     840
gttaaggagg ctaagggact tgactactca ttcttcgacg ctccaattaa gggtgaatta     900
atgggggtta ctagggatga cttgaagggt aggcttgact ggattggggt aaactactac     960
actagggccg tattgaggag gaggcaggat gctggtcggg catcagtagc cgtggtggat    1020
ggattcggct actcctgtga acctggaggc gtatctaatg ataggagacc atgcagtgac    1080
ttcggctggg aaatataccc tgagggtgtt tacaatgtct taatggacct atggaggagg    1140
tataggatgc ccatgtacat cactgagaac ggtatagctg atgagcatga taagtggagg    1200
tcatggttca tagtatcgca cctgtatcaa attcacaggg caatggagga gggggtggat    1260
gttagagggt acttccactg gaacctaata gataacttgg agtgggctgc aggatatagg    1320
atgaggttcg gcctagttta cgttgactat gcaaccaaga ggaggtattt taggccaagc    1380
gccctggtta tgagggaggt ggctaaacag aaggctatac cggattactt agagcattac    1440
attaaaccac ctagaattga a                                              1461
```

<210> 40
<211> 487
<212> PRT
<213> Artificial Sequence

<220>
<223> Mutant glycoside hydrolase family protein of Caldivirga maquiling ensis having glycosylation site.

<220>
<221> CARBOHYD
<222> (61)..(63)

<223>

<400> 40

Met Gly Ile Lys Phe Pro Ser Asp Phe Arg Phe Gly Phe Ser Thr Val
1               5                   10                  15

Gly Thr Gln His Glu Met Gly Thr Pro Gly Ser Glu Phe Val Ser Asp
            20                  25                  30

Trp Tyr Val Trp Leu His Asp Pro Glu Asn Ile Ala Ser Gly Leu Val
        35                  40                  45

Ser Gly Asp Leu Pro Glu His Gly Pro Gly Tyr Trp Asn Arg Thr Lys
    50                  55                  60

Gln Asp His Ser Ile Ala Arg Asp Leu Gly Leu Asp Ala Ala Trp Ile
65                  70                  75                  80

Thr Ile Glu Trp Ala Arg Val Phe Pro Lys Pro Thr Phe Asp Val Lys
                85                  90                  95

Val Lys Val Asp Glu Asp Asp Gly Gly Asn Val Val Asp Val Glu Val
            100                 105                 110

Asn Glu Ser Ala Leu Glu Glu Leu Arg Arg Leu Ala Asp Leu Asn Ala
        115                 120                 125

Val Asn His Tyr Arg Gly Ile Leu Ser Asp Trp Lys Glu Arg Gly Gly
    130                 135                 140

Leu Leu Val Ile Asn Leu Tyr His Trp Ala Met Pro Thr Trp Leu His
145                 150                 155                 160

Asp Pro Ile Ala Val Arg Lys Asn Gly Pro Asp Arg Ala Pro Ser Gly
165 170 175

Trp Leu Asp Lys Arg Ser Val Ile Glu Phe Thr Lys Phe Ala Ala Phe
180 185 190

Ile Ala His Glu Leu Gly Asp Leu Ala Asp Met Trp Tyr Thr Met Asn
195 200 205

Glu Pro Gly Val Val Ile Thr Glu Gly Tyr Leu Tyr Val Lys Ser Gly
210 215 220

Phe Pro Pro Gly Tyr Leu Asp Leu Asn Ser Leu Ala Thr Ala Gly Lys
225 230 235 240

His Leu Ile Glu Ala His Ala Arg Ala Tyr Asp Ala Ile Lys Ala Tyr
245 250 255

Ser Arg Lys Pro Val Gly Leu Val Tyr Ser Phe Ala Asp Tyr Gln Pro
260 265 270

Leu Arg Gln Gly Asp Glu Glu Ala Val Lys Glu Ala Lys Gly Leu Asp
275 280 285

Tyr Ser Phe Phe Asp Ala Pro Ile Lys Gly Glu Leu Met Gly Val Thr
290 295 300

Arg Asp Asp Leu Lys Gly Arg Leu Asp Trp Ile Gly Val Asn Tyr Tyr
305 310 315 320

Thr Arg Ala Val Leu Arg Arg Arg Gln Asp Ala Gly Arg Ala Ser Val
325 330 335

Ala Val Val Asp Gly Phe Gly Tyr Ser Cys Glu Pro Gly Gly Val Ser
340 345 350

Asn Asp Arg Arg Pro Cys Ser Asp Phe Gly Trp Glu Ile Tyr Pro Glu
355 360 365

Gly Val Tyr Asn Val Leu Met Asp Leu Trp Arg Arg Tyr Arg Met Pro
370 375 380

Met Tyr Ile Thr Glu Asn Gly Ile Ala Asp Glu His Asp Lys Trp Arg
385 390 395 400

Ser Trp Phe Ile Val Ser His Leu Tyr Gln Ile His Arg Ala Met Glu
405 410 415

Glu Gly Val Asp Val Arg Gly Tyr Phe His Trp Asn Leu Ile Asp Asn
420 425 430

Leu Glu Trp Ala Ala Gly Tyr Arg Met Arg Phe Gly Leu Val Tyr Val
435 440 445

Asp Tyr Ala Thr Lys Arg Arg Tyr Phe Arg Pro Ser Ala Leu Val Met
450 455 460

Arg Glu Val Ala Lys Gln Lys Ala Ile Pro Asp Tyr Leu Glu His Tyr
465 470 475 480

Ile Lys Pro Pro Arg Ile Glu
485

<210> 41
<211> 1476
<212> DNA
<213> Artificial Sequence

<220>
<223> DNA encording mutant beta-galactosidase of Sulfolobus acidocaldar ius having glycosylation site.

<400> 41

```
atgggtttat cattcccaaa gggtttcaaa tttggctggt ctcagtcggg attccagtct     60
gaaatgggaa ctccaggtag cgaagatccg aacagcgatt ggcacgtctg ggttcatgac    120
agggagaaca tagtctcaca ggttgtcagt ggagatttac ccgaaaatgg tccagggtac    180
tggaaccgca ctaagagatt tcatgacgaa gcagagaaaa taggactaaa tgcagtgaga    240
attaacgtag agtggagtag aatatttccc agaccactac ccaagcctga aatgcaaaca    300
gggactgata aagagaacag tcctgtcatt agcgtagact taaatgagtc taagctgaga    360
gaaatggaca actacgctaa tcatgaagcg ttatcacatt acaggcaaat actggaggat    420
ctaagaaaca gaggatttca catagtactg aacatgtatc attggacttt gcccatatgg    480
ttgcacgacc ctatcagagt gaggagagga gactttacag gaccaacagg ttggttaaac    540
tccaggacag tttatgagtt cgctaggttc tcggcttacg tagcctggaa attagatgat    600
ttggcgagtg aatatgcaac aatgaatgaa cctaacgtgg tttggggagc aggttacgct    660
tttcctagag caggctttcc acctaattac cttagcttca ggctttcaga aatagctaaa    720
tggaatataa ttcaggctca tgcgagggct tatgacgcca tcaagagcgt atcaaaaaag    780
agtgtaggta taatatatgc aaacacatca tattacccac tcagaccaca agataacgaa    840
gctgtggaaa tagcagagag attgaacaga tggagtttct ttgactccat tataaaggga    900
gagataacta gtgagggaca aaatgtcaga gaggacttaa ggaacaggtt agactggatt    960
ggcgtaaact attacacgag gactgtggta acaaaagctg agagtggtta tttaaccctt   1020

ccgggttatg gagatcgttg tgaaaggaac tcattgagtt tagctaacct ccctaccagt   1080
gatttcggtt gggagttctt tcctgagggt ctatatgatg tacttttgaa gtattggaat   1140
aggtatgggt taccattata cgtaatggag aacggtatcg ctgatgacgc tgactaccaa   1200
agaccgtatt acttagtatc acatatctac caggtgcaca gggctttaaa cgagggagta   1260
gatgtaagag gttatcttca ttggtctttg gcagataatt atgagtggtc gtcaggtttt   1320
tcaatgaggt tcggtctact taaggtagat tatctaacaa agagattgta ctggagacct   1380
tctgcattag tttacaggga gattactagg agtaacggta ttcctgagga gctggaacat   1440
ctaaacagag taccaccaat aaaacctttg agacat                              1476
```

<210> 42
<211> 492
<212> PRT
<213> Artificial Sequence

<220>
<223> Mutant beta-galactosidase of Sulfolobus acidocaldarius having gly cosylation site.

<220>
<221> CARBOHYD

<222> (62)..(64)
<223>

<400> 42

Met Gly Leu Ser Phe Pro Lys Gly Phe Lys Phe Gly Trp Ser Gln Ser
1 5 10 15

Gly Phe Gln Ser Glu Met Gly Thr Pro Gly Ser Glu Asp Pro Asn Ser
20 25 30

Asp Trp His Val Trp Val His Asp Arg Glu Asn Ile Val Ser Gln Val
35 40 45

Val Ser Gly Asp Leu Pro Glu Asn Gly Pro Gly Tyr Trp Asn Arg Thr
50 55 60

Lys Arg Phe His Asp Glu Ala Glu Lys Ile Gly Leu Asn Ala Val Arg
65 70 75 80

Ile Asn Val Glu Trp Ser Arg Ile Phe Pro Arg Pro Leu Pro Lys Pro
85 90 95

Glu Met Gln Thr Gly Thr Asp Lys Glu Asn Ser Pro Val Ile Ser Val
100 105 110

Asp Leu Asn Glu Ser Lys Leu Arg Glu Met Asp Asn Tyr Ala Asn His
115 120 125

Glu Ala Leu Ser His Tyr Arg Gln Ile Leu Glu Asp Leu Arg Asn Arg
130 135 140

Gly Phe His Ile Val Leu Asn Met Tyr His Trp Thr Leu Pro Ile Trp
145 150 155 160

Leu His Asp Pro Ile Arg Val Arg Arg Gly Asp Phe Thr Gly Pro Thr
165 170 175

Gly Trp Leu Asn Ser Arg Thr Val Tyr Glu Phe Ala Arg Phe Ser Ala
180 185 190

Tyr Val Ala Trp Lys Leu Asp Asp Leu Ala Ser Glu Tyr Ala Thr Met
195 200 205

Asn Glu Pro Asn Val Val Trp Gly Ala Gly Tyr Ala Phe Pro Arg Ala
210 215 220

Gly Phe Pro Pro Asn Tyr Leu Ser Phe Arg Leu Ser Glu Ile Ala Lys
225 230 235 240

Trp Asn Ile Ile Gln Ala His Ala Arg Ala Tyr Asp Ala Ile Lys Ser
245 250 255

Val Ser Lys Lys Ser Val Gly Ile Ile Tyr Ala Asn Thr Ser Tyr Tyr
260 265 270

Pro Leu Arg Pro Gln Asp Asn Glu Ala Val Glu Ile Ala Glu Arg Leu
275 280 285

Asn Arg Trp Ser Phe Phe Asp Ser Ile Ile Lys Gly Glu Ile Thr Ser
290 295 300

Glu Gly Gln Asn Val Arg Glu Asp Leu Arg Asn Arg Leu Asp Trp Ile
305 310 315 320

Gly Val Asn Tyr Tyr Thr Arg Thr Val Val Thr Lys Ala Glu Ser Gly
325 330 335

Tyr Leu Thr Leu Pro Gly Tyr Gly Asp Arg Cys Glu Arg Asn Ser Leu
340 345 350

Ser Leu Ala Asn Leu Pro Thr Ser Asp Phe Gly Trp Glu Phe Phe Pro
355 360 365

Glu Gly Leu Tyr Asp Val Leu Leu Lys Tyr Trp Asn Arg Tyr Gly Leu
370 375 380

Pro Leu Tyr Val Met Glu Asn Gly Ile Ala Asp Asp Ala Asp Tyr Gln

385 390 395 400

Arg Pro Tyr Tyr Leu Val Ser His Ile Tyr Gln Val His Arg Ala Leu
405 410 415

Asn Glu Gly Val Asp Val Arg Gly Tyr Leu His Trp Ser Leu Ala Asp
420 425 430

Asn Tyr Glu Trp Ser Ser Gly Phe Ser Met Arg Phe Gly Leu Leu Lys
435 440 445

Val Asp Tyr Leu Thr Lys Arg Leu Tyr Trp Arg Pro Ser Ala Leu Val
450 455 460

Tyr Arg Glu Ile Thr Arg Ser Asn Gly Ile Pro Glu Glu Leu Glu His
465 470 475 480

Leu Asn Arg Val Pro Pro Ile Lys Pro Leu Arg His
485 490

<210> 43
<211> 1470
<212> DNA
<213> Artificial Sequence

<220>
<223> DNA encording mutant beta-galactosidase of Sulfolobus solfataricu s having glycosylation site.

<400> 43

```
atgggttact catttccaaa tagctttagg tttggttggt cccaggccgg atttcaatca      60
gaaatgggaa caccagggtc agaagatcca aatactgact ggtataaatg ggttcatgat     120
ccagaaaaca tggcagcggg attagtaagt ggagatctac cagaaaatgg gccaggctac     180
tggaaccgca ctaagacatt tcacgataat gcacaaaaaa tgggattaaa aatagctaga     240
ctaaatgtgg aatggtctag gatatttcct aatccattac caaggccaca aaactttgat     300
gaatcaaaac aagatgtgac agaggttgag ataaacgaaa acgagttaaa gagacttgac     360
gagtacgcta ataaagacgc attaaaccat tacagggaaa tattcaagga tcttaaaagt     420
agaggacttt actttatact aaacatgtat cattggccat tacctctatg gttacacgac     480
ccaataagag taagaagagg agattttact ggaccaagtg gttggctaag tactagaaca     540
gtttacgagt tcgctagatt ctcagcttat atagcttgga aattcgatga tctagtggat     600
gagtactcaa caatgaatga acctaacgtt gttggaggtt taggatacgt tggtgttaag     660
tccggttttc ccccaggata cctaagcttt gaactttccc gtagggcaat gtataacatc     720
attcaagctc acgcaagagc gtatgatggg ataaagagtg tttctaaaaa accagttgga     780
attatttacg ctaatagctc attccagccg ttaacggata aagatatgga agcggtagag     840

atggctgaaa atgataatag atggtggttc tttgatgcta taataagagg tgagatcacc     900
agaggaaacg agaagattgt aagagatgac ctaaagggta gattggattg gattggagtt     960
aattattaca ctaggactgt tgtgaagagg actgaaaagg gatacgttag cttaggaggt    1020
tacggtcacg gatgtgagag gaactctgta agtttagcgg gattaccaac cagcgacttc    1080
ggctgggagt tcttcccaga aggtttatat gacgttttga cgaaatactg gaatagatat    1140
catctctata tgtacgttac tgaaaatggt attgcggatg atgccgatta tcaaaggccc    1200
tattatttag tatctcacgt ttatcaagtt catagagcaa taaatagtgg tgcagatgtt    1260
agagggtatt tacattggtc tctagctgat aattacgaat gggcttcagg attctctatg    1320
aggtttggtc tgttaaaggt cgattacaac actaagagac tatactggag accctcagca    1380
ctagtatata gggaaatcgc cacaaatggc gcaataactg atgaaataga gcacttaaat    1440
agcgtacctc cagtaaagcc attaaggcac                                     1470
```

<210> 44
<211> 490
<212> PRT
<213> Artificial Sequence

<220>
<223> Mutant beta-galactosidase of Sulfolobus solfataricus having glyco sylation site.

<220>
<221> CARBOHYD
<222> (62)..(64)

<223>

<400> 44

```
Met Gly Tyr Ser Phe Pro Asn Ser Phe Arg Phe Gly Trp Ser Gln Ala
1               5                   10                  15

Gly Phe Gln Ser Glu Met Gly Thr Pro Gly Ser Glu Asp Pro Asn Thr
            20                  25                  30

Asp Trp Tyr Lys Trp Val His Asp Pro Glu Asn Met Ala Ala Gly Leu
        35                  40                  45

Val Ser Gly Asp Leu Pro Glu Asn Gly Pro Gly Tyr Trp Asn Arg Thr
    50                  55                  60

Lys Thr Phe His Asp Asn Ala Gln Lys Met Gly Leu Lys Ile Ala Arg
65                  70                  75                  80

Leu Asn Val Glu Trp Ser Arg Ile Phe Pro Asn Pro Leu Pro Arg Pro
                85                  90                  95

Gln Asn Phe Asp Glu Ser Lys Gln Asp Val Thr Glu Val Glu Ile Asn
            100                 105                 110
```

Glu Asn Glu Leu Lys Arg Leu Asp Glu Tyr Ala Asn Lys Asp Ala Leu
115 120 125

Asn His Tyr Arg Glu Ile Phe Lys Asp Leu Lys Ser Arg Gly Leu Tyr
130 135 140

Phe Ile Leu Asn Met Tyr His Trp Pro Leu Pro Leu Trp Leu His Asp
145 150 155 160

Pro Ile Arg Val Arg Arg Gly Asp Phe Thr Gly Pro Ser Gly Trp Leu
165 170 175

Ser Thr Arg Thr Val Tyr Glu Phe Ala Arg Phe Ser Ala Tyr Ile Ala
180 185 190

Trp Lys Phe Asp Asp Leu Val Asp Glu Tyr Ser Thr Met Asn Glu Pro
195 200 205

Asn Val Val Gly Gly Leu Gly Tyr Val Gly Val Lys Ser Gly Phe Pro
210 215 220

Pro Gly Tyr Leu Ser Phe Glu Leu Ser Arg Arg Ala Met Tyr Asn Ile
225 230 235 240

Ile Gln Ala His Ala Arg Ala Tyr Asp Gly Ile Lys Ser Val Ser Lys
245 250 255

Lys Pro Val Gly Ile Ile Tyr Ala Asn Ser Ser Phe Gln Pro Leu Thr
260 265 270

Asp Lys Asp Met Glu Ala Val Glu Met Ala Glu Asn Asp Asn Arg Trp
275 280 285

Trp Phe Phe Asp Ala Ile Ile Arg Gly Glu Ile Thr Arg Gly Asn Glu
290 295 300

Lys Ile Val Arg Asp Asp Leu Lys Gly Arg Leu Asp Trp Ile Gly Val
305 310 315 320

Asn Tyr Tyr Thr Arg Thr Val Val Lys Arg Thr Glu Lys Gly Tyr Val
325 330 335

Ser Leu Gly Gly Tyr Gly His Gly Cys Glu Arg Asn Ser Val Ser Leu
340 345 350

Ala Gly Leu Pro Thr Ser Asp Phe Gly Trp Glu Phe Phe Pro Glu Gly
355 360 365

Leu Tyr Asp Val Leu Thr Lys Tyr Trp Asn Arg Tyr His Leu Tyr Met
370 375 380

Tyr Val Thr Glu Asn Gly Ile Ala Asp Asp Ala Asp Tyr Gln Arg Pro
385 390 395 400

Tyr Tyr Leu Val Ser His Val Tyr Gln Val His Arg Ala Ile Asn Ser
405 410 415

Gly Ala Asp Val Arg Gly Tyr Leu His Trp Ser Leu Ala Asp Asn Tyr
420 425 430

Glu Trp Ala Ser Gly Phe Ser Met Arg Phe Gly Leu Leu Lys Val Asp
435 440 445

Tyr Asn Thr Lys Arg Leu Tyr Trp Arg Pro Ser Ala Leu Val Tyr Arg
450 455 460

Glu Ile Ala Thr Asn Gly Ala Ile Thr Asp Glu Ile Glu His Leu Asn
465 470 475 480

Ser Val Pro Pro Val Lys Pro Leu Arg His
485 490

<210> 45
<211> 1488
<212> DNA
<213> Artificial Sequence

<220>
<223> DNA encording mutant beta-galactosidase of Picrophilus torridus having glycosylation site.

<400> 45

```
atgggtttac ccaagaactt tttacttggc ttttctctgg ctggctttca gtctgaaatg      60
ggcatatcag atcctgatag caattcagat tggtggttat gggtacatga cccggtgaat     120
ataaggactg gacttgtatc tggtgactta cctgaaaatg gaataggata ctggaaccgc     180
actaaaaaat ataatggtct ggctgttcaa acaggaatga atgctgcaag gcttggagtt     240
gaatggagca ggatatttcc aaaaagtact gaagaagtaa aggtgatgga agattacaaa     300
gatgatgatt taatttccgt ggatgttaat gagggaagtc ttgaaaaact tgacagactg     360
gcaaatcaaa aggcaattaa tagatatatg gaaatcttca ataatatcaa ggaaaataat     420
atgacgctaa tagtgaatgt ttaccattgg ccaataccaa tatatcttca cgatccaata     480
gaagctagga atagtggact ttcaaataaa agaaatggct ggcttaatca taaaaccgtt     540
gtggaatttg taaaatatgc aaaatatctg gcatggaaat ttagcgatgt ggcagatatg     600
ttttctataa tgaatgagcc aaacgttgta tttggtaatg gatattttaa tgttaaatca     660
gggttcccac cagcatttcc aagtgtgcat ggcggtttgc ttgcaaaaaa acatgaaatt     720
gaggctatag caagatcata cgacgccatg aaggagatta caaaaaaacc agttggtcta     780
attatggcaa attcagatgt acaaccacta acagatgagg ataaagaagc agcagaaatg     840
gctacttaca atgatcgcta ttcattcata gatccgctaa gagttggtga gatgaaatgg     900
gctgatgagg ttactgcagg taatccaatt ggtgaaaaga gcaacatcga tagatctgat     960
ctaaaaaata agctagactg gataggtgtt aactattata caagggccgt tgtaaaaaaa    1020
tctggaaacg gatatacaac attaaaagga tatggacact ctgcaaccgc tggcatgcca    1080
agtagggccg gaagggatgt aagtgacttt ggctgggaat tttatccaga aggtcttgta    1140
aacgtcttat catcatactg gaaaagatat cacattccaa tgattgtgac tgaaaatggt    1200
gttgctgact ctattgatag acttagacca aggtaccttg tgtcacatat aaagtctgtt    1260
gaaaaggctt tatctatggg tatggatatt aggggatatc ttcactggtc tctgattgat    1320
aactatgaat gggcatcagg tttttcaatg aaatttgggc tttatggtat tgatttgaac    1380
aataaaaaga ttcaacacag accaagtgca ctggtattta aagaaattgc aaatgccaac    1440
ggagtcccgg aggaatttga atggatggca gaccagcatc agaactca                  1488
```

<210> 46
<211> 496
<212> PRT
<213> Artificial Sequence

<220>
<223> Mutant beta-galactosidase of Picrophilus torridus having glycosyl ation site.

<220>
<221> CARBOHYD
<222> (59)..(61)

<223>

<400> 46

```
Met Gly Leu Pro Lys Asn Phe Leu Leu Gly Phe Ser Leu Ala Gly Phe
1               5                   10                  15

Gln Ser Glu Met Gly Ile Ser Asp Pro Asp Ser Asn Ser Asp Trp Trp
            20                  25                  30

Leu Trp Val His Asp Pro Val Asn Ile Arg Thr Gly Leu Val Ser Gly
        35                  40                  45

Asp Leu Pro Glu Asn Gly Ile Gly Tyr Trp Asn Arg Thr Lys Lys Tyr
    50                  55                  60

Asn Gly Leu Ala Val Gln Thr Gly Met Asn Ala Ala Arg Leu Gly Val
65                  70                  75                  80
```

Glu Trp Ser Arg Ile Phe Pro Lys Ser Thr Glu Glu Val Lys Val Met
85 90 95

Glu Asp Tyr Lys Asp Asp Asp Leu Ile Ser Val Asp Val Asn Glu Gly
100 105 110

Ser Leu Glu Lys Leu Asp Arg Leu Ala Asn Gln Lys Ala Ile Asn Arg
115 120 125

Tyr Met Glu Ile Phe Asn Asn Ile Lys Glu Asn Asn Met Thr Leu Ile
130 135 140

Val Asn Val Tyr His Trp Pro Ile Pro Ile Tyr Leu His Asp Pro Ile
145 150 155 160

Glu Ala Arg Asn Ser Gly Leu Ser Asn Lys Arg Asn Gly Trp Leu Asn
165 170 175

His Lys Thr Val Val Glu Phe Val Lys Tyr Ala Lys Tyr Leu Ala Trp
180 185 190

Lys Phe Ser Asp Val Ala Asp Met Phe Ser Ile Met Asn Glu Pro Asn
195 200 205

Val Val Phe Gly Asn Gly Tyr Phe Asn Val Lys Ser Gly Phe Pro Pro
210 215 220

Ala Phe Pro Ser Val His Gly Gly Leu Leu Ala Lys Lys His Glu Ile
225 230 235 240

Glu Ala Ile Ala Arg Ser Tyr Asp Ala Met Lys Glu Ile Thr Lys Lys
245 250 255

Pro Val Gly Leu Ile Met Ala Asn Ser Asp Val Gln Pro Leu Thr Asp
260 265 270

Glu Asp Lys Glu Ala Ala Glu Met Ala Thr Tyr Asn Asp Arg Tyr Ser
275 280 285

Phe Ile Asp Pro Leu Arg Val Gly Glu Met Lys Trp Ala Asp Glu Val
290 295 300

Thr Ala Gly Asn Pro Ile Gly Glu Lys Ser Asn Ile Asp Arg Ser Asp
305 310 315 320

Leu Lys Asn Lys Leu Asp Trp Ile Gly Val Asn Tyr Tyr Thr Arg Ala
325 330 335

Val Val Lys Lys Ser Gly Asn Gly Tyr Thr Thr Leu Lys Gly Tyr Gly

340 345 350

His Ser Ala Thr Ala Gly Met Pro Ser Arg Ala Gly Arg Asp Val Ser
355 360 365

Asp Phe Gly Trp Glu Phe Tyr Pro Glu Gly Leu Val Asn Val Leu Ser
370 375 380

Ser Tyr Trp Lys Arg Tyr His Ile Pro Met Ile Val Thr Glu Asn Gly
385 390 395 400

Val Ala Asp Ser Ile Asp Arg Leu Arg Pro Arg Tyr Leu Val Ser His
405 410 415

Ile Lys Ser Val Glu Lys Ala Leu Ser Met Gly Met Asp Ile Arg Gly
420 425 430

Tyr Leu His Trp Ser Leu Ile Asp Asn Tyr Glu Trp Ala Ser Gly Phe
435 440 445

Ser Met Lys Phe Gly Leu Tyr Gly Ile Asp Leu Asn Asn Lys Lys Ile
450 455 460

Gln His Arg Pro Ser Ala Leu Val Phe Lys Glu Ile Ala Asn Ala Asn
465 470 475 480

Gly Val Pro Glu Glu Phe Glu Trp Met Ala Asp Gln His Gln Asn Ser
485 490 495

<210> 47
<211> 1443
<212> DNA
<213> Artificial Sequence

<220>
<223> DNA encording mutant beta-galactosidase of Thermoplasma volcanium having glycosylation site.

<400> 47

atggtagaaa acaattttcc agaggatttc aaatttggtt ggtcacagtc aggttttcaa 60
tcggagatgg gctatgataa cgcaatggac gataaaagtg actggtatgt ctgggttcat 120
gataaagaaa acatccaatc agggcttgta agcggagaca tgcccgaaaa tggtccgggt 180
tactggaacc gcactaaatc attccatgaa gctgcacaga atatgggatt aaaaatggca 240
agaatcggag ttgaatggtc aagattattc ccggaacctt tcccggaaaa aataatggca 300
gatgcaaaaa ataattcctt agaaataaac aataacattc tttcagaact tgataaatat 360
gtcaataaag atgcactcaa ccattacatt gagatattta atgatatcaa aaatagaaat 420
atagatttaa taattaatat gtaccactgg ccacttcctg tatggctaag cgatcctgta 480

tctgttagaa aaggaataaa aacagaaaga tcaggctggc tgaatgacag gatagttcaa 540
ttgtttgctt tattctcctc gtatatagta tataaaatgg aagatctggc agttgcattt 600
tcaaccatga atgaacctaa tgttgtttat ggaaatggtt ttataaatat caaatcaggt 660
tttccgcctt cctatctcag ttcagaattt gcatctaaag ttaagaacaa tatattaaaa 720
gcacattctc ttgcatacga ttctatgaaa aaaattacgg ataaacctgt gggaataatt 780
tatgcaaaca catattttac gcctttggac ccggaaaaag ataatgatgc tattgctaaa 840
gcagacagtg atgcgaaatg gtcatttttt gatccattaa taaaaggaga taaatcactt 900
ggaattaatg gcaataaact agattggatc ggaattaatt attatacaag gacaatgtta 960
aggaaagacg gagatggcta tatttcatta aaaggctatg gtcattcagg ttctcctaat 1020
actgtaacaa acgataaaag accaacaagt gatataggat gggaatttta tccggaggga 1080
ttggaatatg taattatgaa ttactggaac aggtataaat tgcctatgta cgtaacagaa 1140
aatggcatag ccgataatgg ggattatcag aggccttatt atttagtttc acacattgca 1200
agtgtactga gggcaataaa taaaggagcc aatgtaaagg gttatttgca ctggtcctta 1260
gttgataatt atgaatgggc attgggattt agcccgaaat ttggtttaat aggatacgat 1320
gaaaataaaa aactatactg gaggccaagt gctcttgttt ataaggaaat agcaacaaaa 1380
aattgcatat ccccagaatt aaagcacctc gattcaatac cgcctataaa tggtttaaga 1440
aaa 1443

<210> 48
<211> 481
<212> PRT
<213> Artificial Sequence

<220>
<223> Mutant beta-galactosidase of Thermoplasma volcanium aggregans hav ing glycosylation site.

<220>
<221> CARBOHYD

<222> (63)..(65)
<223>

<400> 48

```
Met Val Glu Asn Asn Phe Pro Glu Asp Phe Lys Phe Gly Trp Ser Gln
1               5                   10                  15

Ser Gly Phe Gln Ser Glu Met Gly Tyr Asp Asn Ala Met Asp Asp Lys
            20                  25                  30

Ser Asp Trp Tyr Val Trp Val His Asp Lys Glu Asn Ile Gln Ser Gly
        35                  40                  45

Leu Val Ser Gly Asp Met Pro Glu Asn Gly Pro Gly Tyr Trp Asn Arg
    50                  55                  60
```

Thr Lys Ser Phe His Glu Ala Ala Gln Asn Met Gly Leu Lys Met Ala
65 70 75 80

Arg Ile Gly Val Glu Trp Ser Arg Leu Phe Pro Glu Pro Phe Pro Glu
85 90 95

Lys Ile Met Ala Asp Ala Lys Asn Asn Ser Leu Glu Ile Asn Asn Asn
100 105 110

Ile Leu Ser Glu Leu Asp Lys Tyr Val Asn Lys Asp Ala Leu Asn His
115 120 125

Tyr Ile Glu Ile Phe Asn Asp Ile Lys Asn Arg Asn Ile Asp Leu Ile
130 135 140

Ile Asn Met Tyr His Trp Pro Leu Pro Val Trp Leu Ser Asp Pro Val
145 150 155 160

Ser Val Arg Lys Gly Ile Lys Thr Glu Arg Ser Gly Trp Leu Asn Asp
165 170 175

Arg Ile Val Gln Leu Phe Ala Leu Phe Ser Ser Tyr Ile Val Tyr Lys
180 185 190

Met Glu Asp Leu Ala Val Ala Phe Ser Thr Met Asn Glu Pro Asn Val
195 200 205

Val Tyr Gly Asn Gly Phe Ile Asn Ile Lys Ser Gly Phe Pro Pro Ser
210 215 220

Tyr Leu Ser Ser Glu Phe Ala Ser Lys Val Lys Asn Asn Ile Leu Lys
225 230 235 240

Ala His Ser Leu Ala Tyr Asp Ser Met Lys Lys Ile Thr Asp Lys Pro
245 250 255

Val Gly Ile Ile Tyr Ala Asn Thr Tyr Phe Thr Pro Leu Asp Pro Glu
260 265 270

Lys Asp Asn Asp Ala Ile Ala Lys Ala Asp Ser Asp Ala Lys Trp Ser
275 280 285

Phe Phe Asp Pro Leu Ile Lys Gly Asp Lys Ser Leu Gly Ile Asn Gly
290 295 300

Asn Lys Leu Asp Trp Ile Gly Ile Asn Tyr Tyr Thr Arg Thr Met Leu
305 310 315 320

Arg Lys Asp Gly Asp Gly Tyr Ile Ser Leu Lys Gly Tyr Gly His Ser
325 330 335

Gly Ser Pro Asn Thr Val Thr Asn Asp Lys Arg Pro Thr Ser Asp Ile
340 345 350

Gly Trp Glu Phe Tyr Pro Glu Gly Leu Glu Tyr Val Ile Met Asn Tyr
355 360 365

Trp Asn Arg Tyr Lys Leu Pro Met Tyr Val Thr Glu Asn Gly Ile Ala
370 375 380

Asp Asn Gly Asp Tyr Gln Arg Pro Tyr Tyr Leu Val Ser His Ile Ala
385 390 395 400

Ser Val Leu Arg Ala Ile Asn Lys Gly Ala Asn Val Lys Gly Tyr Leu
405 410 415

His Trp Ser Leu Val Asp Asn Tyr Glu Trp Ala Leu Gly Phe Ser Pro
420 425 430

Lys Phe Gly Leu Ile Gly Tyr Asp Glu Asn Lys Lys Leu Tyr Trp Arg
435 440 445

Pro Ser Ala Leu Val Tyr Lys Glu Ile Ala Thr Lys Asn Cys Ile Ser
450 455 460

Pro Glu Leu Lys His Leu Asp Ser Ile Pro Pro Ile Asn Gly Leu Arg
465 470 475 480

Lys

<210> 49
<211> 1404
<212> DNA
<213> Artificial Sequence

<220>
<223> DNA encording mutant glycoside hydrolase family protein of Fervid obacterium nodosum having glycosylation site.

<400> 49

```
atgggtatgt ttccgaaaga ttttttattt ggtgtttcga tgtctgggtt tcagtttgag      60
atgggaaatc ctcaagatgc agaagaggtt gatctaaata cagattggta tgtatgggtt     120
agggatattg gaaatattgt aaatggagtc gtaagtgggg acttgcctga aaatggttca     180
tggtactgga accgcactgg caaagtccac caattagctg ccgattttgg gatggatgta     240
atacgaattg gaaccgaatg gtctaggatt ttcccagtta gtacgcaaag tgttgagtac     300

ggctcaccgg atatgctcga aaaattggat aaattagcaa accaaaaagc ggtaagtcat     360
tacaggaaaa taatggagga tataaaagca aaggggttaa aattgttcgt taacctttac     420
cactttactt tacctatttg gttgcacgac cctatagctg ttcacaaagg tgagaagaca     480
gataaaattg gttggatttc tgatgctaca cctattgagt ttgcgaagta tgcagagtac     540
atggcgtgga aatttgccga tatagttgat atgtgggctt ctatgaacga accacacgtt     600
gtaagtcagc ttggatattt tgcaataaat gcgggatttc caccaagtta ttttaatcct     660
tcatggtata tcaaaagttt agaaaacgaa gcgaaagcac ataacttatc ttatgatgct     720
ataaaaaagt atacaaataa tcctgttgga gttatatact cttttacatg gtacgatact     780
gttaataaag atgacaagga atcttttgaa aatgctatgg atctcacaaa ttggcgattt     840
atagatatgg taaaagataa aactgattac ataggtgtaa attattacac aagagcggtt     900
atcgatagac ttcccaccac tattgacttt ggcgaattta aaatgaattg gtatactttg     960
agaggttacg gttattcttg cgaagaagga ggattctcac tctccggaag gccggcaagc    1020
gaatttggat gggaaatata ccctgaaggg ctgtacaata ttttgataca tgtttataat    1080
agatacaaaa aagatattta tgttacggag aacggtatag ctgattcgaa ggataaatac    1140
agaagtcttt ttatcatatc gcatctttat gctatagaaa aagcattaaa cgaaggaata    1200
ccaataaaag gttatttgca ctggtcgatt atagacaatt tcgaatgggc gaagggctac    1260
agtaaaagat ttggacttgc ttacacagat ttgtcaacca aaaaatatat acctagacct    1320
tctatgtaca tttttagaga gataataaag gataaatcaa tcgacaaatt caaaggttac    1380
gatccatata acttgatgaa attc                                           1404
```

<210> 50
<211> 468
<212> PRT
<213> Artificial Sequence

<220>
<223> Mutant glycoside hydrolase family protein of Fervidobacterium nod osum having glycosylation site.

<220>
<221> CARBOHYD
<222> (64) .. (66)
<223>

<400> 50

Met Gly Met Phe Pro Lys Asp Phe Leu Phe Gly Val Ser Met Ser Gly
1               5                   10                  15

Phe Gln Phe Glu Met Gly Asn Pro Gln Asp Ala Glu Glu Val Asp Leu
            20                  25                  30

Asn Thr Asp Trp Tyr Val Trp Val Arg Asp Ile Gly Asn Ile Val Asn
        35                  40                  45

Gly Val Val Ser Gly Asp Leu Pro Glu Asn Gly Ser Trp Tyr Trp Asn
50 55 60

Arg Thr Gly Lys Val His Gln Leu Ala Ala Asp Phe Gly Met Asp Val
65 70 75 80

Ile Arg Ile Gly Thr Glu Trp Ser Arg Ile Phe Pro Val Ser Thr Gln
85 90 95

Ser Val Glu Tyr Gly Ser Pro Asp Met Leu Glu Lys Leu Asp Lys Leu
100 105 110

Ala Asn Gln Lys Ala Val Ser His Tyr Arg Lys Ile Met Glu Asp Ile
115 120 125

Lys Ala Lys Gly Leu Lys Leu Phe Val Asn Leu Tyr His Phe Thr Leu
130 135 140

Pro Ile Trp Leu His Asp Pro Ile Ala Val His Lys Gly Glu Lys Thr
145 150 155 160

Asp Lys Ile Gly Trp Ile Ser Asp Ala Thr Pro Ile Glu Phe Ala Lys
165 170 175

Tyr Ala Glu Tyr Met Ala Trp Lys Phe Ala Asp Ile Val Asp Met Trp
180 185 190

Ala Ser Met Asn Glu Pro His Val Val Ser Gln Leu Gly Tyr Phe Ala
195 200 205

Ile Asn Ala Gly Phe Pro Pro Ser Tyr Phe Asn Pro Ser Trp Tyr Ile
210 215 220

Lys Ser Leu Glu Asn Glu Ala Lys Ala His Asn Leu Ser Tyr Asp Ala
225 230 235 240

Ile Lys Lys Tyr Thr Asn Asn Pro Val Gly Val Ile Tyr Ser Phe Thr
245 250 255

Trp Tyr Asp Thr Val Asn Lys Asp Asp Lys Glu Ser Phe Glu Asn Ala
260 265 270

Met Asp Leu Thr Asn Trp Arg Phe Ile Asp Met Val Lys Asp Lys Thr
275 280 285

Asp Tyr Ile Gly Val Asn Tyr Tyr Thr Arg Ala Val Ile Asp Arg Leu
290 295 300

Pro Thr Thr Ile Asp Phe Gly Glu Phe Lys Met Asn Trp Tyr Thr Leu
305 310 315 320

Arg Gly Tyr Gly Tyr Ser Cys Glu Glu Gly Gly Phe Ser Leu Ser Gly
325 330 335

Arg Pro Ala Ser Glu Phe Gly Trp Glu Ile Tyr Pro Glu Gly Leu Tyr
340 345 350

Asn Ile Leu Ile His Val Tyr Asn Arg Tyr Lys Lys Asp Ile Tyr Val
355 360 365

Thr Glu Asn Gly Ile Ala Asp Ser Lys Asp Lys Tyr Arg Ser Leu Phe
370 375 380

Ile Ile Ser His Leu Tyr Ala Ile Glu Lys Ala Leu Asn Glu Gly Ile
385 390 395 400

Pro Ile Lys Gly Tyr Leu His Trp Ser Ile Ile Asp Asn Phe Glu Trp
405 410 415

Ala Lys Gly Tyr Ser Lys Arg Phe Gly Leu Ala Tyr Thr Asp Leu Ser
420 425 430

Thr Lys Lys Tyr Ile Pro Arg Pro Ser Met Tyr Ile Phe Arg Glu Ile
435 440 445

Ile Lys Asp Lys Ser Ile Asp Lys Phe Lys Gly Tyr Asp Pro Tyr Asn
450 455 460

Leu Met Lys Phe
465

<210> 51
<211> 1419
<212> DNA
<213> Artificial

<220>
<223> DNA encording mutant beta-glucosidase having glycosylation site.

<400> 51

atggcaaagt tcccaaaaaa cttcatgttt ggatattctt ggtctggttt ccagtttgag 60
atgggactgc caggaagtga agtggaaagc gactggtggg tgtgggttaa cgacacggag 120
aacatagcat caggtctagt aagtggagat ctaccagaga acggcccagc atattggaac 180
cgcactaagc aagatcatga cattgcagaa aagctaggaa tggattgtat tagaggtggc 240
attgagtggg caagaatttt tccaaagcca acatttgacg ttaaagttga tgtggaaaag 300
gatgaagaag gcaacataat ttccgtagac gttccagaga gtacaataaa agagctagag 360

aaaattgcca acatggaggc ccttgaacat tatcgcaaga tttactcaga ctggaaggag 420
aggggcaaaa ccttcatatt aaacctctac cactggcctc ttccattatg gattcatgac 480
ccaattgcag taaggaaact tggcccggat agggctcctg caggatggtt agatgagaag 540
acagtggtag agtttgtgaa gtttgccgcc ttcgttgctt atcaccttga tgacctcgtt 600
gacatgtgga gcacaatgaa cgaaccaaac gtagtctaca atcaaggtta cattaatcta 660
cgttcaggat ttccaccagg atatctaagc tttgaagcag cagaaaaggc aaaattcaac 720
ttaattcagg ctcacatcgg agcatatgat gccataaaag agtattcaga aaaatccgtg 780
ggagtgatat acgcctttgc ttggcacgat cctctagcgg aggagtataa ggatgaagta 840
gaggaaatca gaaagaaaga ctatgagttt gtaacaattc tacactcaaa aggaaagcta 900
gactggatcg gcgtaaacta ctactccagg ctggtatatg gagccaaaga tggacaccta 960
gttcctttac ctggatatgg atttatgagt gagagaggag gatttgcaaa gtcaggaaga 1020
cctgctagtg actttggatg ggaaatgtac ccagagggcc ttgagaacct tcttaagtat 1080
ttaaacaatg cctacgagct accaatgata attacagaga acggtatggc cgatgcagca 1140
gatagataca ggccacacta tctcgtaagc catctaaagg cagtttacaa tgctatgaaa 1200
gaaggtgctg atgttagagg gtatctccac tggtctctaa cagacaacta cgaatgggcc 1260
caaggcttca ggatgagatt tggattggtt tacgtggatt tcgagacaaa gaagagatat 1320
ttaaggccaa gcgccctggt attcagagaa atagccactc aaaaagaaat tccagaagaa 1380
ttagctcacc tcgcagacct caaatttgtt acaagaaag 1419

<210> 52
<211> 473
<212> PRT
<213> Artificial

<220>
<223> Mutant beta-glucosidase having glycosylation site.

<220>
<221> CARBOHYD
<222> (37)..(39)
<223>

<220>
<221> CARBOHYD
<222> (60)..(62)
<223>

<400> 52

Met Ala Lys Phe Pro Lys Asn Phe Met Phe Gly Tyr Ser Trp Ser Gly
1               5                   10                  15

Phe Gln Phe Glu Met Gly Leu Pro Gly Ser Glu Val Glu Ser Asp Trp
            20                  25                  30

```
Trp Val Trp Val Asn Asp Thr Glu Asn Ile Ala Ser Gly Leu Val Ser
        35                  40                  45

Gly Asp Leu Pro Glu Asn Gly Pro Ala Tyr Trp Asn Arg Thr Lys Gln
    50                  55                  60

Asp His Asp Ile Ala Glu Lys Leu Gly Met Asp Cys Ile Arg Gly Gly
65                  70                  75                  80

Ile Glu Trp Ala Arg Ile Phe Pro Lys Pro Thr Phe Asp Val Lys Val
                85                  90                  95

Asp Val Glu Lys Asp Glu Glu Gly Asn Ile Ile Ser Val Asp Val Pro
            100                 105                 110

Glu Ser Thr Ile Lys Glu Leu Glu Lys Ile Ala Asn Met Glu Ala Leu
        115                 120                 125

Glu His Tyr Arg Lys Ile Tyr Ser Asp Trp Lys Glu Arg Gly Lys Thr
    130                 135                 140

Phe Ile Leu Asn Leu Tyr His Trp Pro Leu Pro Leu Trp Ile His Asp
145                 150                 155                 160

Pro Ile Ala Val Arg Lys Leu Gly Pro Asp Arg Ala Pro Ala Gly Trp
                165                 170                 175

Leu Asp Glu Lys Thr Val Val Glu Phe Val Lys Phe Ala Ala Phe Val
            180                 185                 190

Ala Tyr His Leu Asp Asp Leu Val Asp Met Trp Ser Thr Met Asn Glu
        195                 200                 205

Pro Asn Val Val Tyr Asn Gln Gly Tyr Ile Asn Leu Arg Ser Gly Phe
    210                 215                 220

Pro Pro Gly Tyr Leu Ser Phe Glu Ala Ala Glu Lys Ala Lys Phe Asn
225                 230                 235                 240

Leu Ile Gln Ala His Ile Gly Ala Tyr Asp Ala Ile Lys Glu Tyr Ser
                245                 250                 255

Glu Lys Ser Val Gly Val Ile Tyr Ala Phe Ala Trp His Asp Pro Leu
            260                 265                 270

Ala Glu Glu Tyr Lys Asp Glu Val Glu Glu Ile Arg Lys Lys Asp Tyr
        275                 280                 285
```

Glu Phe Val Thr Ile Leu His Ser Lys Gly Lys Leu Asp Trp Ile Gly
    290                 295                 300

Val Asn Tyr Tyr Ser Arg Leu Val Tyr Gly Ala Lys Asp Gly His Leu
305                 310                 315                 320

Val Pro Leu Pro Gly Tyr Gly Phe Met Ser Glu Arg Gly Gly Phe Ala
                325                 330                 335

Lys Ser Gly Arg Pro Ala Ser Asp Phe Gly Trp Glu Met Tyr Pro Glu
            340                 345                 350

Gly Leu Glu Asn Leu Leu Lys Tyr Leu Asn Asn Ala Tyr Glu Leu Pro
        355                 360                 365

Met Ile Ile Thr Glu Asn Gly Met Ala Asp Ala Ala Asp Arg Tyr Arg
    370                 375                 380

Pro His Tyr Leu Val Ser His Leu Lys Ala Val Tyr Asn Ala Met Lys
385                 390                 395                 400

Glu Gly Ala Asp Val Arg Gly Tyr Leu His Trp Ser Leu Thr Asp Asn
                405                 410                 415

Tyr Glu Trp Ala Gln Gly Phe Arg Met Arg Phe Gly Leu Val Tyr Val
            420                 425                 430

Asp Phe Glu Thr Lys Lys Arg Tyr Leu Arg Pro Ser Ala Leu Val Phe
        435                 440                 445

Arg Glu Ile Ala Thr Gln Lys Glu Ile Pro Glu Glu Leu Ala His Leu
    450                 455                 460

Ala Asp Leu Lys Phe Val Thr Arg Lys
465                 470

<210> 53
<211> 1419
<212> DNA
<213> Artificial

<220>
<223> DNA encording mutant beta-glucosidase having glycosylation site.

<400> 53

atggcaaagt tcccaaaaaa cttcatgttt ggatattctt ggtctggttt ccagtttgag 60
atgggactgc caggaagtga agtggaaagc gactggtggg tgtgggttca cgacaaggag 120
aacatagcat caggtctagt aagtggagat ctaccagaga acggcccagc atattggaac 180
cgcactaagc aagatcatga cattgcagaa aagctaggaa tggattgtat tagaggtggc 240
attgagtggg caagaatttt tccaaagcca acatttgacg ttaaagttga tgtggaaaag 300
gatgaagaag gcaacataat ttccgtagac gttccagaga gtacaataaa agagctagag 360
aaaattgcca acatggaggc ccttgaacat tatcgcaaga tttactcaga ctggaaggag 420
aggggcaaaa ccttcatatt aaacctctac cactggcctc ttccattatg gattcatgac 480
ccaattgcag taaggaaact tggcccggat agggctcctg caggatggtt agatgagaag 540
acagtggtag agtttgtgaa gtttgccgcc ttcgttgctt atcaccttga tgacctcgtt 600
gacatgtgga gcacaatgaa cgaaccaaac gtagtctaca atcaaggtta cattaatcta 660
cgttcaggat ttccaccagg atatctaaac tttacagcag cagaaaaggc aaaattcaac 720
ttaattcagg ctcacatcgg agcatatgat gccataaaag agtattcaga aaaatccgtg 780
ggagtgatat acgcctttgc ttggcacgat cctctagcgg aggagtataa ggatgaagta 840
gaggaaatca gaaagaaaga ctatgagttt gtaacaattc tacactcaaa aggaaagcta 900
gactggatcg gcgtaaacta ctactccagg ctggtatatg gagccaaaga tggacaccta 960
gttcctttac ctggatatgg atttatgagt gagagaggag gatttgcaaa gtcaggaaga 1020
cctgctagtg actttggatg ggaaatgtac ccagagggcc ttgagaacct tcttaagtat 1080
ttaaacaatg cctacgagct accaatgata attacagaga acggtatggc cgatgcagca 1140
gatagataca ggccacacta tctcgtaagc catctaaagg cagtttacaa tgctatgaaa 1200
gaaggtgctg atgttagagg gtatctccac tggtctctaa cagacaacta cgaatgggcc 1260
caagggttca ggatgagatt tggattggtt tacgtggatt tcgagacaaa gaagagatat 1320
ttaaggccaa gcgccctggt attcagagaa atagccactc aaaaagaaat tccagaagaa 1380
ttagctcacc tcgcagacct caaatttgtt acaagaaag 1419

<210> 54
<211> 473
<212> PRT
<213> Artificial

<220>
<223> Mutant beta-glucosidase having glycosylation site.

<220>
<221> CARBOHYD
<222> (60)..(62)
<223>

<220>
<221> CARBOHYD
<222> (230)..(232)
<223>

<400> 54

Met Ala Lys Phe Pro Lys Asn Phe Met Phe Gly Tyr Ser Trp Ser Gly
1 5 10 15

Phe Gln Phe Glu Met Gly Leu Pro Gly Ser Glu Val Glu Ser Asp Trp
20 25 30

Trp Val Trp Val His Asp Lys Glu Asn Ile Ala Ser Gly Leu Val Ser
35 40 45

Gly Asp Leu Pro Glu Asn Gly Pro Ala Tyr Trp Asn Arg Thr Lys Gln
50 55 60

Asp His Asp Ile Ala Glu Lys Leu Gly Met Asp Cys Ile Arg Gly Gly
65 70 75 80

Ile Glu Trp Ala Arg Ile Phe Pro Lys Pro Thr Phe Asp Val Lys Val
85 90 95

Asp Val Glu Lys Asp Glu Glu Gly Asn Ile Ile Ser Val Asp Val Pro
100 105 110

Glu Ser Thr Ile Lys Glu Leu Glu Lys Ile Ala Asn Met Glu Ala Leu
115 120 125

Glu His Tyr Arg Lys Ile Tyr Ser Asp Trp Lys Glu Arg Gly Lys Thr
130 135 140

Phe Ile Leu Asn Leu Tyr His Trp Pro Leu Pro Leu Trp Ile His Asp
145 150 155 160

Pro Ile Ala Val Arg Lys Leu Gly Pro Asp Arg Ala Pro Ala Gly Trp
165 170 175

Leu Asp Glu Lys Thr Val Val Glu Phe Val Lys Phe Ala Ala Phe Val
180 185 190

Ala Tyr His Leu Asp Asp Leu Val Asp Met Trp Ser Thr Met Asn Glu
195 200 205

Pro Asn Val Val Tyr Asn Gln Gly Tyr Ile Asn Leu Arg Ser Gly Phe
210 215 220

Pro Pro Gly Tyr Leu Asn Phe Thr Ala Ala Glu Lys Ala Lys Phe Asn
225 230 235 240

Leu Ile Gln Ala His Ile Gly Ala Tyr Asp Ala Ile Lys Glu Tyr Ser
245 250 255

Glu Lys Ser Val Gly Val Ile Tyr Ala Phe Ala Trp His Asp Pro Leu
260 265 270

Ala Glu Glu Tyr Lys Asp Glu Val Glu Glu Ile Arg Lys Lys Asp Tyr

```
        275                 280                 285

Glu Phe Val Thr Ile Leu His Ser Lys Gly Lys Leu Asp Trp Ile Gly
    290                 295                 300

Val Asn Tyr Tyr Ser Arg Leu Val Tyr Gly Ala Lys Asp Gly His Leu
305                 310                 315                 320

Val Pro Leu Pro Gly Tyr Gly Phe Met Ser Glu Arg Gly Gly Phe Ala
                325                 330                 335

Lys Ser Gly Arg Pro Ala Ser Asp Phe Gly Trp Glu Met Tyr Pro Glu
            340                 345                 350

Gly Leu Glu Asn Leu Leu Lys Tyr Leu Asn Asn Ala Tyr Glu Leu Pro
        355                 360                 365

Met Ile Ile Thr Glu Asn Gly Met Ala Asp Ala Ala Asp Arg Tyr Arg
    370                 375                 380

Pro His Tyr Leu Val Ser His Leu Lys Ala Val Tyr Asn Ala Met Lys
385                 390                 395                 400

Glu Gly Ala Asp Val Arg Gly Tyr Leu His Trp Ser Leu Thr Asp Asn
                405                 410                 415

Tyr Glu Trp Ala Gln Gly Phe Arg Met Arg Phe Gly Leu Val Tyr Val
            420                 425                 430

Asp Phe Glu Thr Lys Lys Arg Tyr Leu Arg Pro Ser Ala Leu Val Phe
        435                 440                 445

Arg Glu Ile Ala Thr Gln Lys Glu Ile Pro Glu Glu Leu Ala His Leu
    450                 455                 460

Ala Asp Leu Lys Phe Val Thr Arg Lys
465                 470
```

<210> 55
<211> 1419
<212> DNA
<213> Artificial

<220>
<223> DNA encording mutant beta-glucosidase having glycosylation site.

<400> 55

```
atggcaaagt tcccaaaaaa cttcatgttt ggatattctt ggtctggttt ccagtttgag     60
atgggactgc caggaagtga agtggaaagc gactggtggg tgtgggttca cgacaaggag    120
aacatagcat caggtctagt aagtggagat ctaccagaga acggcccagc atattggaac    180
cgcactaagc aagatcatga cattgcagaa aagctaggaa tggattgtat tagaggtggc    240
attgagtggg caagaatttt tccaaagcca acatttgacg ttaaagttga tgtggaaaag    300
gatgaagaag gcaacataat ttccgtagac gttccagaga gtacaataaa agagctagag    360
aaaattgcca acatggaggc ccttgaacat tatcgcaaga tttactcaga ctggaaggag    420
aggggcaaaa ccttcatatt aaacctctac cactggcctc ttccattatg gattcatgac    480
ccaattgcag taaggaaact tggcccggat agggctcctg caggatggtt agatgagaag    540
acagtggtag agtttgtgaa gtttgccgcc ttcgttgctt atcaccttga tgacctcgtt    600
gacatgtgga gcacaatgaa cgaaccaaac gtagtctaca atcaaggtta cattaatcta    660
cgttcaggat ttccaccagg atatctaagc tttgaagcag cagaaaaggc aaaattcaac    720
ttaattcagg ctcacatcgg agcatatgat gccataaaag agtattcaga aaaatccgtg    780
ggagtgatat acgcctttgc ttggcacgat cctctagcgg aggagtataa ggatgaagta    840
gaggaaatca gaaagaaaga ctatgagttt gtaacaattc tacactcaaa aggaaagcta    900
gactggatcg gcgtaaacta ctactccagg ctggtatatg gagccaaaga tggacaccta    960
gttcctttac ctggatatgg atttatgagt gagagaggag gatttgcaaa gtcaggaaga   1020
cctgctagtg actttggatg ggaaatgtac ccagagggcc ttgagaacct tcttaagtat   1080
ttaaacaata actacacgct accaatgata attacagaga acggtatggc cgatgcagca   1140
gatagataca ggccacacta tctcgtaagc catctaaagg cagtttacaa tgctatgaaa   1200
gaaggtgctg atgttagagg gtatctccac tggtctctaa cagacaacta cgaatgggcc   1260
caagggttca ggatgagatt tggattggtt tacgtggatt tcgagacaaa gaagagatat   1320
ttaaggccaa gcgccctggt attcagagaa atagccactc aaaaagaaat tccagaagaa   1380
ttagctcacc tcgcagacct caaatttgtt acaagaaag                           1419
```

<210> 56
<211> 473
<212> PRT
<213> Artificial

<220>
<223> Mutant beta-glucosidase having glycosylation site.

<220>
<221> CARBOHYD
<222> (60)..(62)
<223>

<220>
<221> CARBOHYD
<222> (364)..(366)
<223>

<400> 56

Met Ala Lys Phe Pro Lys Asn Phe Met Phe Gly Tyr Ser Trp Ser Gly

1 5 10 15

Phe Gln Phe Glu Met Gly Leu Pro Gly Ser Glu Val Glu Ser Asp Trp
20 25 30

Trp Val Trp Val His Asp Lys Glu Asn Ile Ala Ser Gly Leu Val Ser
35 40 45

Gly Asp Leu Pro Glu Asn Gly Pro Ala Tyr Trp Asn Arg Thr Lys Gln
50 55 60

Asp His Asp Ile Ala Glu Lys Leu Gly Met Asp Cys Ile Arg Gly Gly
65 70 75 80

Ile Glu Trp Ala Arg Ile Phe Pro Lys Pro Thr Phe Asp Val Lys Val
85 90 95

Asp Val Glu Lys Asp Glu Glu Gly Asn Ile Ile Ser Val Asp Val Pro
100 105 110

Glu Ser Thr Ile Lys Glu Leu Glu Lys Ile Ala Asn Met Glu Ala Leu
115 120 125

Glu His Tyr Arg Lys Ile Tyr Ser Asp Trp Lys Glu Arg Gly Lys Thr
130 135 140

Phe Ile Leu Asn Leu Tyr His Trp Pro Leu Pro Leu Trp Ile His Asp
145 150 155 160

Pro Ile Ala Val Arg Lys Leu Gly Pro Asp Arg Ala Pro Ala Gly Trp
165 170 175

Leu Asp Glu Lys Thr Val Val Glu Phe Val Lys Phe Ala Ala Phe Val
180 185 190

Ala Tyr His Leu Asp Asp Leu Val Asp Met Trp Ser Thr Met Asn Glu
195 200 205

Pro Asn Val Val Tyr Asn Gln Gly Tyr Ile Asn Leu Arg Ser Gly Phe
210 215 220

Pro Pro Gly Tyr Leu Ser Phe Glu Ala Ala Glu Lys Ala Lys Phe Asn
225 230 235 240

Leu Ile Gln Ala His Ile Gly Ala Tyr Asp Ala Ile Lys Glu Tyr Ser
245 250 255

Glu Lys Ser Val Gly Val Ile Tyr Ala Phe Ala Trp His Asp Pro Leu
260 265 270

Ala Glu Glu Tyr Lys Asp Glu Val Glu Glu Ile Arg Lys Lys Asp Tyr
275 280 285

Glu Phe Val Thr Ile Leu His Ser Lys Gly Lys Leu Asp Trp Ile Gly
290 295 300

Val Asn Tyr Tyr Ser Arg Leu Val Tyr Gly Ala Lys Asp Gly His Leu
305 310 315 320

Val Pro Leu Pro Gly Tyr Gly Phe Met Ser Glu Arg Gly Gly Phe Ala
325 330 335

Lys Ser Gly Arg Pro Ala Ser Asp Phe Gly Trp Glu Met Tyr Pro Glu
340 345 350

Gly Leu Glu Asn Leu Leu Lys Tyr Leu Asn Asn Asn Tyr Thr Leu Pro
355 360 365

Met Ile Ile Thr Glu Asn Gly Met Ala Asp Ala Ala Asp Arg Tyr Arg
370 375 380

Pro His Tyr Leu Val Ser His Leu Lys Ala Val Tyr Asn Ala Met Lys
385 390 395 400

Glu Gly Ala Asp Val Arg Gly Tyr Leu His Trp Ser Leu Thr Asp Asn
405 410 415

Tyr Glu Trp Ala Gln Gly Phe Arg Met Arg Phe Gly Leu Val Tyr Val
420 425 430

Asp Phe Glu Thr Lys Lys Arg Tyr Leu Arg Pro Ser Ala Leu Val Phe
435 440 445

Arg Glu Ile Ala Thr Gln Lys Glu Ile Pro Glu Glu Leu Ala His Leu
450 455 460

Ala Asp Leu Lys Phe Val Thr Arg Lys
465 470

<210> 57
<211> 36
<212> DNA
<213> Artificial

<220>
<223> primer

<400> 57
gtgggtgtgg gttaacgaca cggagaacat agcatc 36

<210> 58
<211> 36
<212> DNA
<213> Artificial

<220>
<223> primer

<400> 58
cacccacacc caattgctgt gcctcttgta tcgtag 36

<210> 59
<211> 36
<212> DNA
<213> Artificial

<220>
<223> primer

<400> 59
ccaggatatc taaactttac agcagcagaa aaggca 36

<210> 60
<211> 36
<212> DNA
<213> Artificial

<220>
<223> primer

<400> 60
ggtcctatag atttgaaatg tcgtcgtctt ttccgt 36

<210> 61
<211> 35
<212> DNA
<213> Artificial

<220>
<223> primer

<400> 61
agtatttaaa caataactac acgctaccaa tgata 35

<210> 62
<211> 35
<212> DNA
<213> Artificial

<220>
<223> primer

<400> 62
tcataaattt gttattgatg tgcgatggtt actat 35

## Description

### Technical Field

The present invention relates to a method for producing a glycosylated mutant glucosidase derived from a thermophile, an enzyme composition containing this enzyme, and a method for hydrolyzing biomass using the enzyme composition.

### Background Art

Various techniques are available for saccharification of cellulose, among which an enzymatic saccharification method, which uses mild reaction conditions and achieves high sugar yield, has become the mainstream of development.

Cellulase, which is a cellulose degrading enzyme, is roughly classified into cellobiohydrolase, which acts on the crystalline regions of cellulose, and endoglucanase, which reduces the molecular weight by acting on within the cellulose molecular chain. These cellulases are known to be inhibited by cellobiose, which is one of the products of cellulose degradation. Meanwhile, β-glucosidase is an enzyme that acts on a water-soluble oligosaccharide or cellobiose and catalyzes a hydrolysis reaction of the β-glycosidic bond. Particularly, β-glucosidase is an enzyme necessary for the acquisition of plenty of glucose, which is useful as a fermentation raw material. Also, it is known that the reactions mediated by cellobiohydrolase or endoglucanase are inhibited by the accumulation of cellobiose, which is produced by cellulose degradation. That is, β-glucosidase has an effect of greatly improving the cellulose degradation efficiency, owing to its capability of drastically reducing the accumulation of cellobiose produced by cellulose degradation.

Cellulose is contained abundantly in herbaceous plants and woody plants, which are collectively called cellulosic biomass. Cellulosic biomass contains, in addition to cellulose, hemicellulose such as xylan and arabinan, and lignin. Particularly, being an aromatic polymer compound, lignin contained in cellulosic biomass is known to act in an inhibitory manner in the enzymatic saccharification by cellulase derived from filamentous fungi. Although the mechanism of inhibition of cellulase derived from filamentous fungi by lignin has not been entirely elucidated yet, the reduced degradation efficiency caused by adsorption of cellulase to lignin is proposed as one of the causes (Non Patent Literature 1).

A heat-resistant enzyme is highly stable and retains its activity for a long time even under high temperature conditions; therefore, the application of a heat-resistant enzyme as an industrial enzyme is under study. A large number of heat-resistant enzymes have been confirmed among the enzymes possessed by thermophiles or hyperthermophiles.

Also with regard to heat-resistant β-glucosidase, it has been identified from several species of thermophiles or hyperthermophiles. Specifically, heat-resistant β-glucosidase has been identified from organisms such as *Pyrococcus furiosus, Pyrococcus horikoshii, Thermotoga maritima, Sulfolobus shibatae,* and *Clostridium thermocellum.*

Cellulase or β-glucosidase derived from filamentous fungi is known to be glycosylated (Non Patent Literature 2). As a general function of a sugar chain in such a glycosylated protein, effects such as improving protein solubility, improving physical stability, and improving protease resistance are known (Non Patent Literature 3). As a function conferred by the possession of a sugar chain by a saccharification enzyme such as cellulase, it is disclosed that glycosylation of xylanase with N-linked sugar chains results in an increased expression level of xylanase (Patent Literature 1).

### Citation List

#### Patent Literature

Patent Literature 1: WO/2005/093072

#### Non Patent Literature

Non Patent Literature 1: Hetti P. et al., Journal of Biotechnology, 107, 65 to 72 (2004)
Non Patent Literature 2: Christian P. et al., Trichoderma and Gliocladium: Basic Biology, Taxonomy and Genetics., vol. 1, 121 to 138 (1998)
Non Patent Literature 3: H. Ohba et al., Biosci. Biotech. Biochem., 59, 1581 to 1583 (1995)

Summary of Invention

Technical Problem

An object of the present invention is to provide a glycosylated mutant glucosidase derived from a thermophile, and further, to provide an enzyme composition exhibiting high degradation efficiency in the process of hydrolysis of cellulose, particularly lignin-containing lignocellulose, by mixing the above glucosidase and cellulase.

Solution to Problem

The present inventors conducted an intensive research to achieve the aforementioned object. As a result, they have found that a glycosylated mutant glucosidase derived from a thermophile can be applied to cellulose degradation.
That is, the present invention is composed of the following technical means.

(1) A method for producing a mutant glucosidase derived from a thermophile that has selectively attached thereto a sugar chain and also has a glucosidase activity, comprising:

(i) preparing DNA encoding a mutant glucosidase derived from a thermophile by introducing a DNA sequence encoding Asn-X-Ser or Asn-X-Thr (wherein, X is any amino acid except proline) into DNA encoding a glucosidase derived from a thermophile that is originally devoid of a glycosylation sequence, and further, adding a DNA sequence encoding a secretion signal sequence to the DNA encoding the mutant glucosidase,
(ii) introducing the DNA encoding the mutant glucosidase to which the DNA sequence encoding the secretion signal sequence has been added into an eukaryotic microorganism so that a mutant glucosidase encoded by the DNA of the mutant glucosidase is expressed as a secretory protein, and
(iii) isolating and purifying the mutant glucosidase thus expressed as a secretory protein.

(2) The method for producing a mutant glucosidase according to (1), wherein the sugar chain is a high mannose type sugar chain.
(3) The method for producing a mutant glucosidase according to (1) or (2), wherein the glucosidase derived from a thermophile is a glucosidase derived from a thermophile selected from the group consisting of the genus *Sulofolobus,* the genus *Thermoplasma,* the genus *Caldivirgra,* the genus *Thermosphaera,* the genus *Pyrococcus,* the genus *Picrophilus,* the genus *Caldivirgra,* and the genus *Fervidobacterium.*
(4) The method for producing a glucosidase according to any of (1) to (3), wherein the glucosidase derived from a thermophile is a protein comprising:

(i) a same amino acid sequence as any of amino acid sequences shown in SEQ ID NO: 4, SEQ ID NO: 8, SEQ ID NO: 10, SEQ ID NO: 12, SEQ ID NO: 14, SEQ ID NO: 16, SEQ ID NO: 18, and SEQ ID NO: 20, or
(ii) an amino acid sequence having 85% or more identity with any of amino acid sequences shown in SEQ ID NO: 4, SEQ ID NO: 8, SEQ ID NO: 10, SEQ ID NO: 12, SEQ ID NO: 14, SEQ ID NO: 16, SEQ ID NO: 18, and SEQ ID NO: 20, and also a β-glucosidase activity.

(5) The method for producing a mutant glucosidase according to any of (1) to (4), wherein the eukaryotic microorganism is *Pichia pastoris.*
(6) The method for producing a mutant glucosidase according to any of (1) to (5), wherein the secretion signal sequence is a α factor secretion signal sequence.
(7) The method for producing a mutant glucosidase according to any of (1) to (6), wherein the mutant glucosidase derived from a thermophile comprises an amino acid sequence shown in any of SEQ ID NO: 6, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, and SEQ ID NO: 56.
(8) An enzyme composition for saccharification of biomass comprising cellulase and the mutant glucosidase derived from a thermophile obtained by the production method according to any of (1) to (7).
(9) The enzyme composition for saccharification of biomass according to (8), wherein the cellulase is a mixture of cellulases derived from filamentous fungi.
(10) The enzyme composition for saccharification of biomass according to (8) or (9), wherein the mixture of cellulases derived from filamentous fungi is a mixture of cellulases derived from the genus *Trichoderma.*
(11) A method for hydrolyzing biomass, comprising using the enzyme composition according to any of (8) to (10).
(12) The method for hydrolyzing biomass according to (11), comprising filtering a hydrolysate obtained by the aforementioned enzyme composition through an ultrafiltration membrane, and separating and recovering the used enzyme composition.

The present specification encompasses the contents described in the specification and/or drawings of JP Patent Application No. 2010-044242, based on which the present application claims priority.

Advantageous Effects of Invention

Compared to the use of a glucosidase derived from a thermophile with an unglycosylated cellulase mixture, the glycosylated mutant glucosidase derived from a thermophile obtained by the present invention can achieve higher cellulose degradation efficiency in the hydrolysis of cellulosic biomass. This effect is prominent particularly in the hydrolysis of lignocellulose. Also, the glycosylated mutant glucosidase derived from a thermophile according to the present invention has low adsorptivity for cellulosic biomass, particularly for lignocellulose, and for an ultrafiltration membrane, and also for an ultrafiltration membrane used for separation of a sugar solution from the hydrolysate, and thus achieves excellent enzyme recovery from the hydrolysate.

Brief Description of Drawings

[Figure 1] Figure 1 shows an alignment of a β-glucosidase derived from *Pyrococcus furiosus* (PfuBGL) shown in SEQ ID NO: 4, a β-glucosidase derived from *Trichoderma reesei* (TriReBGL) shown in SEQ ID NO: 1, and a β-glucosidase derived from *Aspergillus niger* (AspNgBGL) shown in SEQ ID NO: 2 in Example 1. The glycosylation sequence in SEQ ID NO: 1 and SEQ ID NO: 2 was underlined, and the glycosylation sequence-introduction site (H60, L61, and Y62) in SEQ ID NO: 4 was similarly underlined.

[Figure 2-1] Figure 2-1 shows an alignment of the amino acid sequence of SEQ ID NO: 6 (gPfuBGL) and the amino acid sequences of SEQ ID NO: 8 (ThAggBGY), SEQ ID NO: 10 (CmGHFP), SEQ ID NO: 12 (SaBGAL), SEQ ID NO: 14 (SsoBGAL), SEQ ID NO: 16 (PtBGAL), SEQ ID NO: 18 (TvBGAL), and SEQ ID NO: 20 (FnGHFP) in Example 2. The glycosylation sequence Asn-Arg-Thr (N-R-T) inserted in the sequence of SEQ ID NO: 6 was underlined. Also, the site corresponding to the glycosylation site Asn-Arg-Thr (N-R-T) in the sequence of SEQ ID NO: 6 in SEQ ID NO: 8, SEQ ID NO: 10, SEQ ID NO: 12, SEQ ID NO: 14, SEQ ID NO: 16, SEQ ID NO: 18, or SEQ ID NO: 20 was similarly underlined.

[Figure 2-2] Figure 2-2 is a sequel to Figure 2-1.

[Figure 3] Figure 3 is a diagram showing the band pattern of polyacrylamide gel electrophoresis for PfuBGL (left) prepared in Comparative Example 1 and the glycosylated mutant PfuBGL prepared in Example 2 without EndoH treatment (right) and with EndoH treatment (middle). A reduction in the molecular weight of the glycosylated mutant PfuBGL by EndoH treatment is confirmed.

[Figure 4] Figure 4 is a graph showing the results of evaluation of enzyme heat stability by measuring the changes in the amount of glucose produced by cellobiose degradation by PfuBGL at a heat retention time of 50 to 90°C in Example 4.

[Figure 5] Figure 5 is a graph showing the results of evaluation of enzyme heat stability by measuring the changes in the amount of glucose produced by cellobiose degradation by the glycosylated mutant PfuBGL at a heat retention time of 50 to 90°C in Example 4.

[Figure 6] Figure 6 is a graph showing the results of comparison of the changes in the amount of glucose produced between the case in which an enzyme composition containing a cellulase derived from the genus *Trichoderma* + the glycosylated mutant PfuBGL was allowed to act on the lignocellulose substrate and the case in which an enzyme composition containing a cellulase derived from the genus *Trichoderma* + PfuBGL was allowed to act on the lignocellulose substrate in Example 5. As the substrate, 5 wt.% lignocellulose was used, and the reactions were allowed to proceed up to 28 hours at 50°C, and the reaction product was sampled as appropriate and measured for the glucose concentration. Cellulase was added at 0.5 mg/mL and glucosidase was added at 0.005 mg/mL (1/100 of the amount of cellulase).

[Figure 7] Figure 7 is a diagram showing the basic structure of an N-linked sugar chain. In the basic structure of an N-linked sugar chain, two N-acetylglucosamine residues, and further, three mannose residues are bound to the Asn side chain of a glucosidase derived from a thermophile.

[Figure 8] Figure 8 is a graph showing the results of evaluation of enzyme heat stability by measuring the changes in the residual activity of AggBGY (relative value to 0 hour of heating) in cellobiose degradation at a heat retention time of 50 to 90°C in Example 12.

[Figure 9] Figure 9 is a graph showing the results of evaluation of enzyme heat stability by measuring the changes in the residual activity of the glycosylated mutant AggBGY (relative value to 0 hour of heating) in cellobiose degradation at a heat retention time of 50 to 90°C in Example 12.

[Figure 10] Figure 10 is a graph showing the results of evaluation of enzyme heat stability by measuring the changes in the residual activity of CmGHFP (relative value to 0 hour of heating) in cellobiose degradation at a heat retention time of 50 to 90°C in Example 12.

[Figure 11] Figure 11 is a graph showing the results of evaluation of enzyme heat stability by measuring the changes in the residual activity of the glycosylated mutant CmGHFP (relative value to 0 hour of heating) in cellobiose degradation at a heat retention time of 50 to 90°C in Example 12.

[Figure 12] Figure 12 is a graph showing the results of evaluation of enzyme heat stability by measuring the changes in the residual activity of SaBGAL (relative value to 0 hour of heating) in cellobiose degradation at a heat retention time of 50 to 90°C in Example 12.

[Figure 13] Figure 13 is a graph showing the results of evaluation of enzyme heat stability by measuring the changes in the residual activity of the glycosylated mutant SaBGAL (relative value to 0 hour of heating) in cellobiose degradation at a heat retention time of 50 to 90°C in Example 12.

[Figure 14] Figure 14 is a graph showing the results of evaluation of enzyme heat stability by measuring the changes in the residual activity of SsoBGAL (relative value to 0 hour of heating) in cellobiose degradation at a heat retention time of 50 to 90°C in Example 12.

[Figure 15] Figure 15 is a graph showing the results of evaluation of enzyme heat stability by measuring the changes in the residual activity of the glycosylated mutant SsoBGAL (relative value to 0 hour of heating) in cellobiose degradation at a heat retention time of 50 to 90°C in Example 12.

[Figure 16] Figure 16 is a graph showing the results of evaluation of enzyme heat stability by measuring the changes in the residual activity of PtBGAL (relative value to 0 hour of heating) in cellobiose degradation at a heat retention time of 50 to 90°C in Example 12.

[Figure 17] Figure 17 is a graph showing the results of evaluation of enzyme heat stability by measuring the changes in the residual activity of the glycosylated mutant PtBGAL (relative value to 0 hour of heating) in cellobiose degradation at a heat retention time of 50 to 90°C in Example 12.

[Figure 18] Figure 18 is a graph showing the results of evaluation of enzyme heat stability by measuring the changes in the residual activity of TvBGAL (relative value to 0 hour of heating) in cellobiose degradation at a heat retention time of 50 to 90°C in Example 12.

[Figure 19] Figure 19 is a graph showing the results of evaluation of enzyme heat stability by measuring the changes in the residual activity of the glycosylated mutant TvBGAL (relative value to 0 hour of heating) in cellobiose degradation at a heat retention time of 50 to 90°C in Example 12.

[Figure 20] Figure 20 is a graph showing the results of evaluation of enzyme heat stability by measuring the changes in the residual activity of FnGHFP (relative value to 0 hour of heating) in cellobiose degradation at a heat retention time of 50 to 90°C in Example 12.

[Figure 21] Figure 21 is a graph showing the results of evaluation of enzyme heat stability by measuring the changes in the residual activity of the glycosylated mutant FnGHFP (relative value to 0 hour of heating) in cellobiose degradation at a heat retention time of 50 to 90°C in Example 12.

## Description of Embodiments

Hereinbelow, the present invention will be described in detail.

The "glucosidase" in the present invention refers to an enzyme having an activity of hydrolyzing a disaccharide having a β-glycosidic bond (i.e., the β-glucosidase activity). Although a group of enzymes belonging to β-glucosidase is listed under the Enzyme Commission (EC) No: EC 3.2.1.21, a protein not belong to β-glucosidase in terms of EC number but having the aforementioned β-glucosidase activity is also encompassed by glucosidase in the present invention. Examples of the glucosidase include galactosidase, mannosidase, and a glucosidic bond hydrolase family protein.

In the present invention, a thermophile is a generic term for a group of microorganisms that can live at 50°C or higher, and particularly, a hyperthermophile refers to a group of microorganisms that can live at 80°C or higher. Examples of the thermophile include the genus *Sulofolobus,* the genus *Thermoplasma,* the genus *Caldivirgra,* the genus *Thermosphaera,* the genus *Pyrococcus,* the genus *Picrophilus,* the genus *Caldivirgra,* and the genus *Fervidobacterium.*

The glucosidase derived from a thermophile is publicly known, and for example, it is registered at GenBank under NP_577802, which can be used in the present invention. Preferably, the glucosidase derived from a thermophile has the amino acid sequence shown in SEQ ID NOs: 4, 8, 10, 12, 14, 16, 18, and 20. More preferably, the glucosidase derived from a thermophile consists of the amino acid sequence shown in SEQ ID NOs: 4, 8, 10, 12, 14, 16, 18, and 20. In the present invention, the glucosidase derived from a thermophile also encompasses a protein having the amino acid sequence of SEQ ID NOs: 4, 8, 10, 12, 14, 16, 18 and 20 that has been subjected to one or multiple deletion, substitution, addition, or insertion, or deletion, substitution, addition, or insertion of one or a plurality of amino acids, and having a β-glucosidase activity. Here, the range of "one or a plurality" is not particularly limited; however, it is preferably 10 or less, more preferably five or less, particularly preferably four or less, or one or two. Also, in the present invention, the glucosidase derived from a thermophile also encompasses a protein having an amino acid sequence with 85% or more, more preferably 90% or more, and most preferably 95% or more identity with the amino acid sequence shown in SEQ ID NOs: 4, 8, 10, 12, 14, 16, 18, and 20 according to calculation using The Basic Local Alignment Search Tool (BLAST) at the National Center for Biological Information and the like (for example, default parameter(s), i.e., the initially set parameter(s)), preferably consisting of the above amino acid sequence, and having the β-glucosidase activity. Here, the term "identity" refers to the percentage of the identical amino acid and homologous amino acid residues relative to the overlapping total amino acid residues in the optimal alignment when two amino acid sequences are aligned either with or without a gap introduced between the amino acid sequences. The identity can be obtained by using a method commonly known to those skilled in the art, sequence analysis software (a publicly known algorithm such as BLAST and FASTA), and the like. The "β-glucosidase activity" is as defined above, and this activity can be measured by, for example, adding an enzyme solution to a cellobiose substrate solution obtained by dissolving cellobiose in a 50 mM acetic acid - sodium acetate buffer (pH 5.0), allowing the reaction to proceed at 30 to 85°C for 30 minutes, terminating the reaction by changing pH as needed, and quantitating the glucose concentration in the resulting reaction solution using a glucose quantitation kit.

In the present invention, the "glucosidase derived from a thermophile" does not encompass a glucosidase naturally having a glycosylation sequence in its amino acid sequence, and it is limited to a glucosidase that is naturally devoid of a glycosylation sequence.

A "sugar chain" as used to in the present invention has a structure in which monosaccharides are linked via a glycosidic bond, and it is terminally bonded to the amino acid side chain of the peptide sequence of the glucosidase derived from a thermophile by covalent bonding. The presence or absence of "sugar chain" can be confirmed by staining glucosidase separated by SDS electrophoresis by a generally known periodic acid-Schiff base (PAS) reaction.

A sugar chain is mainly classified into an N-linked sugar chain, which binds to the asparagine side chain, and an O-linked sugar chain, which binds to the serine and threonine side chains, and the sugar chain is preferably an N-linked sugar chain. For an N-linked sugar chain, a structure having a basic skeleton having two N-acetylglucosamine residues and three mannose residues with respect to the asparagine side chain is given as an example (Figure 7). Sugar molecules are additionally bound to this basic structure by the enzymatic action, whereby various sugar chain structures are composed. The sugar chain structure varies depending on the kind of a microorganism used as a host, the culture condition of the host, and the like. The glycosylated glucosidase derived from a thermophile refers to a compound to which various sugar chain structures are attached.

Whether the sugar chain bonded to the glucosidase derived from a thermophile is either N-linked or O-linked can be confirmed by, for example, allowing each of N-linked glycanase that specifically hydrolyzes the terminal portion of an N-linked sugar chain and O-linked glycanase that specifically hydrolyzes the terminal portion of an O-linked sugar chain to act on the glucosidase, performing SDS electrophoresis, and then comparing the changes in the molecular weight of the glucosidase. As the N-linked glycanase employed here, N-glucosidase F derived from *Flavobacterium meningosepticum* (PNGaseF), endo-β-N-acetylglucosaminidase derived from *Streptomyces plicatus,* and the like can be used. Also, as the O-linked glycanase, endo-α-N-acetylgalactosaminidase derived from *Streptococcus* and the like can be used.

The sugar chain in the mutant glucosidase of the present invention is preferably a high mannose type. Here, the term "high mannose type" refers to an N-linked sugar chain in which four or more mannose residues are linked per two N-acetylglucosamine residues or glucosamine residues that compose the sugar chain. As the sugar other than mannose, other monosaccharides such as glucose may be contained. When glucose is contained, it is normally bound to the non-reducing end of mannose of a high mannose type sugar chain.

Examples of an N-linked sugar chain other than a high mannose type include a complex type sugar chain. A complex type is characterized by containing, as a sugar other than mannose and N-acetylglucosamine, various kinds of sugars such as fructose and sialic acid as its components. Compared to a high mannose type, the ratio of N-acetylglucosamine in a sugar chain is increased, and the ratio of mannose to two N-acetylglucosamine residues is three or less.

Whether or not a given N-linked sugar chain is a high mannose type or a hybrid type can be confirmed by, for example, transferring glucosidase having been subjected to electrophoresis to a PVDF membrane, reacting it with sugar chain-specific lectin, and examining the color development. Examples of the sugar chain-specific lectin used here include Concanavalin A (ConA), lectin from *Ricinus communis* (RCA12), lectin from *Ulex europaeus* (UEA-1), and peanut lectin (PNA). If the glucosidase is stained with ConA, it can be confirmed as a high mannose type N-linked sugar chain, while if it is stained with RCA120, it can be confirmed as a hybrid type N-linked sugar chain. Also, as another judgment technique, the sugar chain structure can be confirmed by separating the sugar chain-composing sugar from sufficiently purified glucosidase and quantitating the monosaccharide component of the sugar thus separated by analyzing it with MALDI-TOF/MS or HPLC.

The "glycosylation sequence" refers to the amino acid sequence of the part that is subjected to glycosylation in the process of expression and translation in a eukaryotic organism.

Examples of the glycosylation sequence include the consensus sequence of an N-linked sugar chain, which is Asn-X-Ser or Asn-X-Thr (wherein, X is any amino acid except proline), and the consensus sequence of an O-linked sugar chain, which is Cys-X-Ser-X-Pro-Cys (wherein, X is any amino acid except proline); however, the glycosylation sequence is not limited thereto. Preferably, the glycosylation sequence is the consensus sequence of an N-linked sugar chain. Here, examples of amino acid except proline include Gly, Ala, Val, Leu, Ile, Phe, Tyr, Trp, Met, Cys, Ser, Thr, Asp, Glu, His, Lys, Arg, Asn, and Gln.

The glycosylated mutant glucosidase derived from a thermophile according to the present invention (hereinbelow, referred to as a "mutant glucosidase") refers to a mutant glucosidase resulting from introduction of the amino acid sequence composing the aforementioned glycosylation sequence into the aforementioned amino acid sequence of a glucosidase derived from a thermophile, wherein the amino acid sequence is selectively glycosylated. One or two or more glycosylation sequences may be introduced, and these sequences may be all the same or contain multiple kinds of glycosylation sequences.

As the glycosylation sequence-introduction site, such a position that does not inactivate the original enzyme activity by the introduction is preferably selected. The method for determining such a glycosylation sequence-introduction site can be carried out by the following step 1) and step 2).

Step 1) Perform the amino acid sequence alignment analysis between a glucosidase derived from filamentous fungi naturally possessing a glycosylation sequence and a glucosidase derived from a thermophile that is naturally devoid of a glycosylation sequence to clarify a relative positional relationship of the glycosylation sequence of the glucosidase derived from filamentous fungi in the glucosidase derived from a thermophile to specify the glycosylation sequence-introduction site. As the alignment tool, a plenty of well-known software such as ClustalW can be used. The glucosidase derived from filamentous fungi naturally possessing a glycosylation sequence is preferably a glucosidase derived from the genus *Trichoderma* or a glucosidase derived from the genus *Aspergillosis*. The amino acid sequences of these glucosidases derived from filamentous fungi are publicly known, and preferably, a β-glucosidase derived from *Trichoderma reesei* having the amino acid sequence of SEQ ID NO: 1 or a β-glucosidase derived from *Aspergillus niger* having the amino acid sequence of SEQ ID NO: 2 is used.

Step 2) Next, confirm if the glycosylation sequence-introduction site in the glucosidase derived from a thermophile that has been specified by the aforementioned alignment analysis is present on the surface of the enzyme. Whether or not it is present on the surface of the enzyme can be found out by using the crystal structure of the objective glucosidase derived from a thermophile. Such a crystal structure can be retrieved from a database such as Protein Data Bank if it is known. Also, a crystal structure may be obtained by actually performing X-ray crystallography, etc.

In the present invention, the glycosylation sequence-introduction site is preferably selected in accordance with the aforementioned step 1) and step 2); however, as another technique, it can be selected in accordance with the following step 3) and step 4).

Step 3) Obtain the Accessible Surface Area (ASA) ($Å^2$) of the amino acid residue by analytical software, and based on the value thus obtained, select the amino acid residue that is exposed near the surface of the glucosidase derived from a thermophile. The ASA of each amino acid residue can be calculated using ASA analytical software such as AREAIMOL (ccp4 package) (Collaborative Computing Project Number 4 (CCP4) of UK Science and Engineering Research Council), SURFace (Barry Honig's group, the Department of Biochemistry and Molecular Biophysics and Center of Computational Biology and Bioinformatics of Columbia), and ASAP(Institute for Molecular Bioscience, University of Queensland and the ARC Centre in Bioinformatics), all of which are obtainable from websites. If an amino acid residue is calculated as having an ASA of 1 $Å^2$ or greater, then it is assumed to be exposed to the surface, and as the glycosylation sequence-introduction site, an amino acid residue with an ASA of 2 $Å^2$ or greater is preferably selected. Particularly, in order to introduce the three amino acid residues (Asn-X-Ser or Asn-X-Thr: X is any amino acid except proline), which form the consensus sequence for an N-linked sugar chain, it is preferable to select a part containing three or more consecutive amino acids with an ASA of 2 $Å^2$ or greater.

Step 4) Select, from among the glycosylation sequence-introduction sites in a glucosidase derived from a thermophile selected by the step 3) above, a position that is too far from the enzyme active site of the glucosidase derived from a thermophile to cause a reduction in the enzyme activity by introduction of the glycosylation sequence. The distance from the enzyme active site can be found out by using the crystal structure of the objective glucosidase derived from a thermophile in a similar manner to the above. Specifically, after excluding the amino acid residues within a distance of 3.5 A from the enzyme active site, the amino acid residue that is further apart than this distance is preferably selected.

For example, application of the aforementioned step 3) and step 4) to PfuBGL represented by the amino acid sequence of SEQ ID NO: 4 will be as follows. First of all, using AREAIMOL (ccp4 package), ASA is calculated by assuming that the solvent molecule has 1.4 Å, and the amino acid residue with an ASA of 2 $Å^2$ or greater is extracted (Table 1 (i)). Subsequently, a part containing three or more consecutive amino acids with an ASA of 2 $Å^2$ or greater is extracted (Table 1 (ii)). Further, based on the information of the crystal structure of PfuBGL, the amino acid residues within a distance of 3.5 Å from the enzyme active site are specified (23 residues of S13, R78, N207, I263, I303, G304, V305, N306, Y307, S343, D344, L367, I370, I371, T372, D378, R384, Y387, H391, D404, V405, R406, N407, Y408, L409, H410, W411, and F427) and then excluded. As a result, the glycosylation sequence-introduction site can be selected (Table 1 (iii)).

[Table 1]

| Example of selection of the glycosylation sequence-introduction site in PfuBGL |
|---|
| (i) Amino acid residues with an ASA of 2 Å$^2$ or greater |
| M1-M9, F17-F19, G22-G25, E27-V34, H37-E40, I42-L51, E53, N54, Y58-Q64, D67, I68, E70, I68, E70, K71, G73, D75, E82-R85, P88-P90, F92-D110, P112-115, K117, E118, E120-A127, E129, H130, R132, K133, S136, D137, K139-F145, Y150-W152, P155-D160, I162, A163, R165-L167, P169-P173, W176-T181, V183, E184, K187, Y194, H195, D197-L199, D201, M202, E208, N210-Q215, Y217-S222, F224, P225, G227-K236, K238-N240, I242, I246, Y249, D250, K253-S259, A265-E279, E281-D287, E289, T292, I293, H295-W302, Y308, R310-Y313, A315-Y326, F328-R340, D344, F345, W374, Y350-E352, E355, N356, K359, Y360, N362-P368, I370, E373, M376, A379-Y383, P385-Y387, S390, K363, A394, Y396, N397, M399-R406, W411, E418-Q421, F423-F427, Y431, D433, P443, L446, R449, E450, T453-E460, A462-K473 |
| (ii) Among (i), a part containing three or more consecutive amino acid residues |
| M1-M9, F17-F19, G22-G25, E27-V34, H37-E40, I42-L51, Y58-Q64, E82-R85, P88-P90, F92-D110, P112-115, E120-A127, K139-F145, Y150-W152, P155-D160, R165-L167, P169-P173, W176-T181, D197-L199, N210-Q215, Y217-S222, G227-K236, K238-N240, K253-S259, A265-E279, E281-D287, H295-W302, R310-Y313, A315-Y326, F328-R340, Y350-E352, N362-P368, A379-Y383, P385-Y387, M399-R406, E418-Q421, F423-F427, T453-E460, A462-K473 |
| (iii) Among (ii), an amino acid residue away from the enzyme active site by 3.5 Å or more |
| M1-M9, F17-F19, G22-G25, E27-V34, H37-E40, I42-L51, Y58-Q64, E82-R85, P88-P90, F92-D110, P112-115, E120-A127, K139-F145, Y150-W152, P155-D160, R165-L167, P169-P173, W176-T181, D197-L199, N210-Q215, Y217-S222, G227-K236, K238-N240, K253-S259, A265-E279, E281-D287, H295-W302, R310-Y313, A315-Y326, F328-R340, Y350-E352, N362-E366, A379-Y383, M399-A403, E418-Q421, F423-R426, T453-E460, A462-K473 |

The glycosylation sequence-introduction site is present at one site or preferably at two to five sites.

In the present invention, the term "introduction" indicates that the aforementioned glycosylation sequence is translated into a polypeptide. That is, in the original amino acid sequence of a glucosidase derived from a thermophile, the aforementioned glycosylation sequence may substitute for the existing amino acid sequence, or it may be inserted into the existing amino acid sequence. That is, in the case of substitution, the length of polypeptide remains unchanged relative to before mutation, while in the case of insertion, the length of polypeptide becomes longer by the length of the inserted glycosylation sequence. However, from the viewpoint of retention of the enzyme activity, the introduction of the glycosylation sequence preferably takes place by substitution of the existing amino acid sequence.

Also, as the glycosylation sequence to be introduced, any of the aforementioned glycosylation sequences may be adopted, and as X, any amino acid except proline can be used; however, a glycosylation sequence and an amino acid that are expected to bring little impact when introduced as a mutation should be selected. For example, in the case of an amino acid substitution, a conservative amino acid substitution is desirable. The conservative amino acid substitution refers to substitution that takes place between the amino acids having similar electrical properties, structural properties, polarity or hydrophobicity, etc., and the substitution between these similar amino acids is expected not to alter the phenotype of protein. Examples include a basic amino acid (Lys, Arg, and His), an acidic amino acid (Glu and Asp), an aromatic amino acid (Trp, Phe, Tyr, and His), a branched amino acid (Val, Ile, and Thr), a polar amino acid (Ser, Thr, Tyr, Cys, Met, Gln, Asn, and Gly), and a hydrophobic amino acid (Ala, Val, Leu, and Ile).

In one embodiment, using the aforementioned technique, the glycosylation sequence-introduction site in the amino acid sequence (SEQ ID NO: 4) of the β-glucosidase derived from *Pyrococcus furiosus* can be specified as H60-L61-Y62 (Figure 1).

Also, according to the present invention, using the amino acid sequence of the glucosidase derived from a thermophile in which the glycosylation sequence-introduction site is determined in the aforementioned technique, the glycosylation sequence-introduction site can be determined also in another glucosidase derived from a thermophile having the amino acid sequence that is highly identical to the above amino acid sequence. For example, after determining the glycosylation sequence-introduction site in the β-glucosidase derived from *Pyrococcus furiosus* shown in SEQ ID NO: 4 by the aforementioned technique, an alignment analysis is performed with respect to another enzyme having the amino acid sequence having high identity with the β-glucosidase shown in the SEQ ID NO: 4, and the position in the amino acid sequence of this enzyme that corresponds to the determined introduction site in SEQ ID NO: 4 (for example, H60-L61-Y62) can be determined as the glycosylation sequence-introduction site. Examples of another enzyme having the amino acid sequence having high identity with the β-glucosidase shown in the SEQ ID NO: 4 include the enzymes having the amino sequences shown in SEQ ID NO: 8, SEQ ID NO: 10, SEQ ID NO: 12, SEQ ID NO: 14, SEQ ID NO: 16, SEQ ID NO: 18, and SEQ ID NO: 20.

In one embodiment, using the aforementioned technique, the glycosylation sequence-introduction site in the amino acid sequences shown in SEQ ID NO: 8, SEQ ID NO: 10, SEQ ID NO: 12, SEQ ID NO: 14, SEQ ID NO: 16, SEQ ID NO: 18, and SEQ ID NO: 20 can be specified as N60-L61-N62, D60-L61-Y62, G61-N62-Y63, G61-N62-Y63, D58-L59-Y60, N63-N64-Y65, and K63-Q64-Y65, respectively (Figure 2-1 and Figure 2-2).

In one embodiment, the mutant glucosidase according to the present invention comprises, for example, the amino acid sequence shown in SEQ ID NO: 6. Preferably, the mutant glucosidase according to the present invention consists of, for example, the amino acid sequence shown in SEQ ID NO: 6.

In the present invention, the glucosidase activity refers to the cellobiose degradation activity. That is, it is an activity of catalyzing the reaction for producing glucose by hydrolysis of cellobiose, when it is used as a substrate. The mutant glucosidase of the present invention retains preferably 40% or more, more preferably 50% or more, even more preferably 60% or more, and particularly preferably 70% or more activity relative to the cellobiose degradation activity of the wild-type glucosidase. The cellobiose degradation activity of the mutant glucosidase of the present invention relative to the wild-type can be evaluated by, for example, adding the mutant glucosidase or the wild-type glucosidase to a 10 mM cellobiose 50 mM acetate buffer solution, carrying out the enzymatic reaction at 50°C, and then measuring the amount of glucose produced. Here, the amount of glucose produced can be quantitated in accordance with a publicly known technique such as the enzymic method and HPLC.

The mutant glucosidase according to the present invention may be one obtained by any method; however, in order for the introduced glycosylation sequence to be selectively glycosylated, the mutant glucosidase is desirably provided by culturing a eukaryotic cell containing DNA encoding the mutant glucosidase.

DNA encoding the mutant glucosidase can be produced by the following method. That is, it can be produced by determining the glycosylation sequence-introduction site in the amino acid sequence of a glucosidase derived from a thermophile by the aforementioned technique, and then introducing DNA encoding the glycosylation sequence into the part of the nucleotide sequence in DNA encoding the glucosidase that corresponds to the introduction site. Here, the term "DNA" encompasses any nucleic acid that encodes the glucosidase or the glycosylation sequence, which can be cDNA, genomic DNA, a gene, and the like.

Examples of DNA encoding a glucosidase derived from a thermophile include DNA encoding the aforementioned glucosidase derived from a thermophile, for example, DNA comprising the nucleotide sequence shown in SEQ ID NOs: 3, 7, 9, 11, 13, 15, 17, and 19, preferably DNA consisting of the above nucleotide sequences. Also, DNA encoding a glucosidase derived from a thermophile encompasses DNA comprising a nucleotide sequence capable of hybridizing with DNA consisting of a nucleotide sequence complementary to the nucleotide sequence shown in SEQ ID NOs: 3, 7, 9, 11, 13, 15, 17, and 19 under stringent conditions, preferably DNA consisting of the above nucleotide sequence, and encoding a protein having the β-glucosidase activity. The stringent condition may be a low stringent condition, a medium stringent condition, or a high stringent condition. The stringent condition includes, for example, carrying out hybridization in 2 to 5 × SSC and 0.2% SDS (wherein, 1 × SSC indicates 150 mM sodium chloride, 15 mM sodium citrate, and pH 7.0) at 45 to 70°C, followed by washing with 0.1 to 1 × SSC and 0.1 to 0.2% SDS at 45 to 65°C. The stringent condition is described in, for example, Sambrook et al., Molecular Cloning, A Laboratory Manual, 2nd edition, 1989, Cold Spring Harbor Laboratory Press. Further, DNA encoding a glucosidase derived from a thermophile encompasses DNA comprising the nucleotide sequence shown in SEQ ID NOs: 3, 7, 9, 11, 13, 15, 17 and 19 that has been subjected to one or multiple deletion, substitution, addition, or insertion, or deletion, substitution, addition, or insertion of one or a plurality of nucleotides and encoding a protein having the β-glucosidase activity. Here, the range of "one or a plurality" is not particularly limited; however, it is preferably 10 or less, more preferably five or less, particularly preferably four or less, or one or two. Furthermore, DNA encoding a glucosidase derived from a thermophile encompasses DNA comprising a nucleotide sequence having 85% or more, more preferably 90% or more, and most preferably 95% or more identity with the nucleotide sequence shown in SEQ ID NOs: 3, 7, 9, 11, 13, 15, 17, and 19 according to calculation using BLAST and the like (for example, default parameter(s), i.e., the initially set parameter(s)), preferably DNA consisting of the above nucleotide sequence, and encoding a protein having the β-glucosidase activity.

Examples of DNA encoding the glycosylation sequence include DNA encoding the aforementioned glycosylation sequence, and it can be appropriately determined in consideration of the host organisms and the degeneracy of the genetic code.

DNA encoding the glycosylation sequence can be introduced into DNA encoding a glucosidase by a publicly known technique such as site-directed mutagenesis and specific mutation introduction by PCR (Sambrook et al., described above).

In one embodiment, examples of DNA encoding the mutant glucosidase include DNA comprising the nucleotide sequence shown in SEQ ID NO: 5. Preferably, examples of DNA encoding the mutant glucosidase include DNA consisting of the nucleotide sequence shown in SEQ ID NO: 5. In SEQ ID NO: 5, DNA encoding the glycosylation sequence is introduced as -aaccgcact- in the positions 178 to 186.

Further, DNA encoding a secretion signal sequence that is suitable for the host may be added to DNA encoding the aforementioned mutant glucosidase. The secretion signal sequence can be appropriately added to the 5' terminus or 3' terminus of DNA encoding the mutant glucosidase, and preferably, it is added to the 5' terminus. DNA encoding the secretion signal sequence may also be incorporated in an expression vector in advance. For example, when the host is yeast, the α factor signal sequence, the α-amylase signal sequence, the glucoamylase signal sequence, the serum albumin signal sequence, the inulinase-derived signal sequence, the invertase signal sequence, the killer protein signal sequence, the lysozyme signal sequence, and the like are used. Particularly in *Pichia pastoris,* the α factor secretion signal sequence is preferred. The α factor secretion signal sequence is publicly known, and for example, it is registered at GenBank under NP_015137, which can be used in the present invention.

When the genus *Trichoderma* is used as the host, the cellulase-related signal sequence can be used. The genus *Trichoderma* has a characteristic of secreting, as cellulase, cellobiohydrolase, xylanase, endoglucanase, xylosidase, and xyloglucanase outside the cell, and these enzymes each have a secretion signal sequence: These signal sequences are publicly known, and peptide sequences containing these signal sequences can be used by functionally linking them to the mutant glucosidase.

DNA encoding the mutant glucosidase having one or more glycosylation sequences that can be subjected to glycosylation by a eukaryotic cell prepared as above is linked to the downstream of the promoter in an appropriate expression vector using a restriction enzyme and a DNA ligase, whereby an expression vector carrying the DNA can be produced.

Examples of the expression vector include a bacterial plasmid, a yeast plasmid, DNA of a phage (such as lambda phage), DNA of a virus such as a retrovirus, a baculovirus, a vaccinia virus, and an adenovirus, a derivative of SV40, and an agrobacterium as a vector for a plant cell, and any other vector can be used as long as it is replicable and viable, and glycosylation is feasible in the host cell. Examples of the expression vector include, when the host is yeast, pPink-HC, pPink-LC, pPinkα-HC, pPCIZ, pPCIZα, pPCI6, pPCI6α, pFLD1, pFLD1α, pGAPZ, pGAPZα, pPIC9K, and pPIC9.

As the promoter, any promoter may be used as long as it is a suitable promoter corresponding to the host to be used for gene expression, and it may be either a constitutive promoter or an inducible promoter. Examples of the promoter include, when the host is yeast, the AOX1 promoter, the TEF1 promoter, the ADE2 promoter, the CYCl promoter, the GALL1 promoter, the AOX2 promoter, the YPT1 promoter, the GAP promoter, and the FLD promoter.

The host cell to be used in the present invention may be any host cell as long as it has the glycosylation mechanism. Preferred examples of the host cell include a yeast cell, a fungal cell, an insect cell, a plant cell, and an animal cell. Examples of the yeast cell include the genus *Pichia,* the genus *Saccharomyces,* and the genus *Schizosaccharomyces.* Examples of the fungal cell include the genus *Aspergillus* and the genus *Trichoderma.* Examples of the insect cell include Sf9. Examples of the plant cell include the dicotyledons. Examples of the animal cell include CHO, HeLa, and HEK293. More preferably, the host cell is a yeast cell, and even more preferably, it is *Pichia pastoris.*

Transformation or transfection can be carried out by a publicly known method such as the calcium phosphate method and electroporation. The mutant glucosidase can be obtained by expressing it in the host cell that has been transformed or transfected as described above under the control of the promoter, and then collecting the expression product. For expression of the mutant glucosidase, the host cell is allowed to proliferate or grow to an appropriate cell density, and the promoter is induced by a temperature shift or chemical induction means such as the addition of isopropyl-1-thio-β-D-galactoside (IPTG), and then the cell is further cultured for a certain period of time.

When the mutant glucosidase is excreted out of the cell, it is directly purified from the medium. When the mutant glucosidase is present outside the cell, the cell is disrupted by physical means such as ultrasonic disintegration and mechanical disintegration or by chemical means such as a cell lysis agent, and then the mutant glucosidase is purified. The mutant glucosidase can be partially or completely purified from the medium of the recombinant cell by a combination of techniques such as ammonium sulfate precipitation or ethanol precipitation, acid extraction, anion or cation exchange chromatography, reverse-phase high-performance chromatography, affinity chromatography, gel filtration chromatography, and electrophoresis.

In the hydrolysis of cellulosic biomass, the glycosylated mutant glucosidase of the present invention has higher heat resistance and can achieve a cellulose degradation efficiency that is 1.2 times, 1.3 time, 1.4 times, 1.5 times, 1.6 times, 1.7 times, 1.8 times, 1.9 times, and twice or more as high as the cellulose degradation efficiency achieved when a glucosidase derived from a thermophile is used with an unglycosylated cellulase mixture. The mutant glucosidase of the present invention can be applied to cellulosic biomass such as cellulosic biomass containing a lignin component, Avicel containing almost no lignin component, Solka Floc, and industrial pulp, and it can achieve profound cellulose degradation efficiency particularly in cellulosic biomass containing a lignin component.

The mutant glucosidase obtained as above can be used as an enzyme composition for saccharification of biomass by mixing with cellulase. The "cellulase" as used herein is not particularly limited as long as it is an enzyme having a cellulose degradation activity, and it may be a mixture of one or more kinds of cellulases. Examples of such an enzyme include cellulase, hemicellulase, cellobiohydrolase, endoglucanase, exoglucanase, xylanase, and mannanase.

The cellulase used in the present invention is preferably a mixture of cellulases derived from filamentous fungi. The mixture of cellulases derived from filamentous fungi is a mixture containing at least both endoglucanase and cellobiohydrolase. In order to carry out more efficient saccharification of cellulose, a mixture of cellulases derived from filamentous fungi containing two or more kinds of endoglucanases and/or two or more kinds of cellobiohydrolases is preferred. Examples of the microorganism producing the aforementioned mixture of cellulases derived from filamentous fungi include the genus *Trichoderma,* the genus *Aspergillus,* the genus *Cellulomonas,* the genus *Clostridium,* the genus *Streptomyces,* the genus *Humicola,* the genus *Acremonium,* the genus *Irpex,* the genus *Mucor,* and the genus *Talaromyces.* Because these microorganisms produce a cellulase in the liquid culture medium, the liquid culture medium may be directly used as an unpurified mixture of cellulases derived from filamentous fungi, or a preparation obtained from a purified product of the liquid culture medium may be used as the mixture of cellulases derived from filamentous fungi. The mixture of cellulases derived from filamentous fungi may simultaneously contain a β-glucosidase produced by the microorganism; however, considering that it does not exist in an adequate amount for cellulose degradation, and also, it is clearly distinguishable from the β-glucosidase derived from the genus *Pyrococcus* as will be described later, the β-glucosidase produced by the cellulase-producing microorganism is also encompassed by cellulase in the present invention. When a preparation obtained from a purified product of the aforementioned liquid culture medium is used, a substance other than an enzyme such as a protease inhibitor, a dispersant, a solubilizing agent, and a stabilizer can be added and the resulting product may be used as a cellulase preparation.

The mixture of cellulases derived from filamentous fungi used in the present invention is preferably a mixture of cellulases produced by the genus *Trichoderma.* The genus *Trichoderma* produces a mixture of cellulases containing at least two kinds of endoglucanases and at least two kinds of cellobiohydrolases into the liquid culture medium, and a mixture of cellulases prepared from such a liquid culture medium is preferably used in the present invention. Among the organisms belonging to the genus *Trichoderma,* a mixture of cellulases derived from *Trichoderma reesei* is more preferred. Examples of the mixture of cellulases derived from *Trichoderma reesei* include a mixture of cellulases derived from *Trichoderma reesei* QM9414, *Trichoderma reesei* QM9123, *Trichoderma reesei* RutC-30, *Trichoderma reesei* PC3-7, *Trichoderma reesei* ATCC66589, *Trichoderma reesei* CL-847, *Trichoderma reesei* MCG77, *Trichoderma reesei* MCG80, and *Trichoderma viride* QM9123. Also, a mutant strain derived from the aforementioned genus *Trichoderma* that has achieved improved cellulose productivity through mutagenic treatment using a mutagen, ultraviolet ray irradiation, or the like may also be used.

The mixture of cellulases derived from the genus *Trichoderma* used in the present invention is an enzyme composition containing a plurality of enzyme components such as cellobiohydrolase, endoglucanase, exoglucanase, β-glucosidase, xylanase, and xylosidase. The mixture of cellulases derived from the genus *Trichoderma* can carry out effective hydrolysis of cellulose owing to the concerted effect or the complementary effect of a plurality of enzyme components in cellulose hydrolysis.

Cellobiohydrolase is a generic term for cellulase that is characterized by starting hydrolysis from the terminal portion of cellulose, and a group of enzymes belonging to cellobiohydrolase is listed under EC No: EC3.2.1.91.

Endoglucanase is a generic term for cellulase that is characterized by starting hydrolysis from the middle part of the cellulose molecular chain, and a group of enzymes belonging to endoglucanase is listed under EC NOs: EC3.2.1.4, EC3.2.1.6, EC3.2.1.39, and EC3.2.1.73.

Exoglucanase is a generic term for cellulase that is characterized by starting hydrolysis from the terminus of the cellulose molecular chain, and a group of enzymes belonging to exoglucanase is listed under EC NOs: EC3.2.1.74 and EC3.2.1.58.

β-glucosidase is a generic term for cellulase that is characterized by acting on cellooligosaccharides or cellobiose, and a group of enzymes belonging to β-glucosidase is listed under EC No: EC3.2.1.21.

Xylanase is a generic term for cellulase that is characterized by acting on hemicellulose or, particularly, xylan, and a group of enzymes belonging to xylanase is listed under EC No: EC3.2.1.8.

Xylosidase is a generic term for cellulase that is characterized by acting on xylooligosaccharides, and a group of enzymes belonging to xylosidase is listed under EC No: EC3.2.1.37.

In the present invention, cellulose-containing biomass is used as a substrate of the enzymatic reaction. Cellulose-containing biomass is cellulose that is widely derived from plant biomass. More specifically, cellulose-containing biomass is bagasse, corn stover, corncob, switchgrass, rice straw, wheat straw, trees, wood materials, building material waste, newspaper, used paper, pulp, and the like. Although the above cellulose-containing biomass contains an impurity such as the macromolecular aromatic compound lignin and hemicellulose, cellulose-containing biomass in which lignin and hemicellulose are partially degraded by using an acid, an alkali, pressurized hot water, and the like as pre-treatment may also be used as cellulose. Here, pre-treated cellulose-containing biomass as described above is provided as "lignocellulose", which can be used as a substrate of the enzymatic reaction.

As the cellulose-containing biomass used in the present invention, one that has been subjected to pre-treatment such as ammonia treatment, diluted sulfuric acid treatment, and hydrothermal treatment by a publicly known technique can be used.

For ammonia treatment, the methods described in JP Patent Publication (Kokai) No. 2008-161125 A and JP Patent Publication (Kokai) No. 2008-535664 A can be applied. Specifically, to biomass, ammonia is added at a concentration of 0.1 to 15 wt.%, and treatment is carried out at 4 to 200°C, preferably at 90 to 150°C. Ammonia to be added may be either in the liquid state or in the gaseous state. When ammonia is in the liquid state, either liquid ammonia or an aqueous solution of ammonia may be used. The number of treatment is not particularly limited, and it may be performed at least once. When the treatment is performed twice or more, the first treatment and the second treatment may be performed under different conditions. The product obtained by the ammonia treatment needs to be subjected to neutralization of ammonia or removal of ammonia before performing the hydrolysis reaction. Neutralization may be performed on a liquid that still contains a solid content or a liquid fraction from which the solid content has been separated. An acid reagent used for neutralization is not particularly limited. Ammonia can also be removed by volatilization in the gaseous state by keeping the ammonia-treated product under the reduced pressure condition. In that case, ammonia that has been removed may be recovered and recycled.

The hydrothermal treatment may be performed by, for example, adding water so that the cellulose-containing biomass is 0.1 to 50 wt.%, and treating the resulting solution at a temperature of 100 to 400°C for one second to 60 minutes. The number of treatment is not particularly limited, and it may be performed at least once. When the treatment is performed twice or more, the first treatment and the second treatment may be performed under different conditions.

For diluted sulfuric acid treatment, for example, the concentration of sulfuric acid is preferably 0.1 to 15 wt.%, more preferably 0.5 to 5 wt.%. The reaction temperature can be set in a range of 100 to 300°C, preferably at 120 to 250°C. The reaction time can be set in a range of one second to 60 minutes. The number of treatment is not particularly limited, and it may be performed at least once. When the treatment is performed twice or more, the first treatment and the second treatment may be performed under different conditions. Because the hydrolysate obtained by the diluted sulfuric acid treatment contains an acid, it needs to be neutralized before using it in the hydrolysis reaction.

Regarding the condition of the enzymatic treatment of cellulose-containing biomass in the present invention, when an enzyme composition for saccharification of biomass containing a cellulase derived from filamentous fungi and the mutant glucosidase of the present invention is used, the treatment is preferably carried out at a temperature of 40°C to 60°C, pH of 3 to 7, and a cellulose-containing biomass solid content concentration of 0.1 to 30%. By setting the condition of the enzymatic treatment in the above range, the cellulose degradation efficiency of a cellulase derived from filamentous fungi and a glucosidase derived from a thermophile can be maximized. Some of the glucosidase derived from a thermophile naturally have an optimum reaction temperature of near 100°C; however, the glucosidase derived from a thermophile used in the present invention exhibits a sufficiently high specific activity even at 40°C to 60°C and can efficiently degrade cellulose-containing biomass in the co-presence of a cellulase derived from filamentous fungi. This enzymatic treatment may be carried out batch-wise or in a continuous manner.

Owing to a high β-glucosidase activity of the enzyme composition for saccharification of biomass containing the mutant glucosidase of the present invention, a sugar liquid obtained by hydrolysis of cellulosic biomass using this enzyme composition has characteristics of having small content of cellobiose but a large amount of glucose. Accordingly, a sugar liquid obtained by using the enzyme composition for saccharification of biomass according to the present invention can be favorably utilized as a carbon source for the growth of microorganisms or cultured cells or for fermentative production using these microorganisms or cultured cells. Examples of the microorganism or the cultured cell used here include yeast such as baker's yeast used in the fermentation industry, bacteria such as E. coli and the coryneform group of bacteria, filamentous fungi, actinomycetes, animal cells, and insect cells. The microorganisms and the cells to be used may be those isolated from natural environments or those having partially modified properties by mutation and genetic recombination. Also, because a sugar liquid derived from cellulose-containing biomass contains pentose such as xylose, a microorganism having an enhanced pentose metabolism pathway is preferably used. Also, using such a sugar liquid as the fermentation raw material, a chemical product can be produced. Specific examples of the chemical product include a substance that is mass-produced in the fermentation industry such as an alcohol, an organic acid, an amino acid, and a nucleic acid, for example, an alcohol such as ethanol, 1,3-propanediol, 1,4-butanediol, and glycerol, an organic acid such as acetic acid, lactic acid, pyruvic acid, succinic acid, malic acid, itaconic acid, and citric acid, a nucleoside such as inosine and guanosine, a nucleotide such as inosinic acid and guanylic acid, and an amine compound such as cadaverine. Further, the sugar liquid can also be applied to the production of an enzyme, an antibiotic, a recombinant protein, and so on.

The sugar liquid obtained by the hydrolysis of cellulosic biomass using the enzyme composition for saccharification of biomass according to the present invention can be used as a sugar liquid after removing the undegraded solid residues as needed, or be directly used as a sugar liquid with the solid residues still contained therein.

In the method for hydrolyzing biomass using the enzyme composition for saccharification of biomass according to the present invention, the used enzyme composition can be separated and recovered from the sugar liquid obtained by the enzymatic treatment of cellulose-containing biomass. Although the method of separation and recovery is not particularly limited, compared to a conventional unglycosylated glucosidase, the mutant glucosidase of the present invention has a characteristic of having a greatly reduced adsorptivity for cellulose-containing biomass, particularly for lignocellulose, and also for an ultrafiltration membrane. Therefore, for separation and recovery of the used enzyme composition, a method of subjecting the hydrolysate to solid-liquid separation as needed, filtering the sugar liquid thus obtained through an ultrafiltration membrane, and separating and recovering the enzyme composition as a non-permeable liquid is preferably used.

As the solid-liquid separation technique in the method for hydrolyzing biomass according to the present invention, either the filtration method or the centrifugation method can be used. Examples of the device for carrying out the solid-liquid separation include, but are not limited to, a belt filter, a screw decanter, a continuous centrifuge, a filter press, and a drum filter.

In the method for hydrolyzing biomass according to the present invention, as the ultrafiltration membrane used for separation and recovery of the enzyme composition, ones made of polyethersulfone (PES), polysulfone (PS), polyacrylonitrile (PAN), poly vinylidene difluoride (PVDF), regenerated cellulose, cellulose, a cellulose ester, sulfonated polysulfone, sulfonated polyethersulfone, polyolefin, polyvinyl alcohol, polymethyl methacrylate, polytetrafluoroethylene and the like can be used. Among them, from the viewpoint of long-term use, an ultrafiltration membrane made of a synthetic polymer other than cellulose is preferred. Generally, an ultrafiltration membrane made of a synthetic polymer has a problem that an enzyme (protein) has high adsorptivity for such a membrane. However, the enzyme composition separated and recovered in the present invention has reduced adsorptivity owing to the effect of glycosylation, and thus is preferably used. With regard to the molecular weight cutoff of the ultrafiltration membrane used in the present invention, an ultrafiltration membrane having a molecular weight cutoff of 500 Da to 100000 Da can be used. Among such ultrafiltration membranes, particularly, one with a molecular weight cutoff ranging from 10000 Da to 30000 Da, which can separate and recover both of the mutant glucosidase of the present invention and the cellulase component derived from filamentous fungi with good yield can be most preferably used.

The filtration method using an ultrafiltration membrane includes dead-end filtration and cross-flow filtration, and from the viewpoint of inhibition of membrane fouling, cross-flow filtration is preferred. Also, as the form of the membrane of ultrafiltration membrane to be used, an appropriately formed membrane such as a flat type membrane, a spiral type membrane, a tubular type membrane, and a hollow yarn type membrane can be used. Specific examples include G-5 type, G-10 type, G-20type, G-50 type, PW type, and HWS UF type, all of which are supplied by DESAL, HFM-180, HFM-183, HFM-251, HFM-300, HFM-116, HFM-183, HFM-300, HFK-131, HFK-328, MPT-U20, MPS-U20P, and MPS-U20S, all of which are supplied by Koch Membrane Systems Inc., SPE1, SPE3, SPE5, SPE10, SPE30, SPV5, SPV50, and SOW30, all of which are supplied by Synder Filtration, the product of microza(R) UF series manufactured by Asahi Kasei Corporation corresponding to a molecular weight cutoff of 3000 to 100000, and NTR7410 and NTR7450 manufactured by Nitto Denko Corporation.

## Examples

Hereinbelow, the present invention will be specifically described with reference to Examples. However, the present invention is not limited to these Examples.

[Comparative Example 1] Preparation of β-glucosidase derived from *Pyrococcus furiosus* (1)

A β-glucosidase derived from the hyperthermophilic archaea *Pyrococcus furiosus* (hereinbelow, referred to as "PfuBGL") is so heat resistant that it is still active at 100°C or higher, and it hydrolyzes various kinds of cellooligosaccharides to produce glucose; therefore, PfuBGL is anticipated for effective utilization of cellulosic biomass.

As to the PfuBGL gene, the gene shown in SEQ ID NO: 3 was entirely synthetized and linked to NcoI and BamHI of pET-lld using Ligation High (Toyobo Co., Ltd.), and the resulting vector was used for transformation of JM109 (Takara Bio Inc.). Screening was performed using an LB agar medium containing ampicillin as an antibiotic. From the transformed JM109 strain, the vector pET-PfuBGL thus prepared was isolated by the miniprep kit (QIAGEN) and subjected to a nucleotide sequence analysis. The pET-PfuBGL was used for transformation of the expression E. coli BL21(DE3)pLysS strain, whereby a BL21-PfuBGL strain was prepared. The BL21-PfuBGL strain was inoculated into 10 mL of an ampicillin-containing LB agar medium, followed by shaking culture (preculture) at 37°C overnight. As the main culture, the bacteria obtained by the preculture were inoculated into 1 L of an ampicillin-containing LB agar medium, and shaking culture was performed until OD 600, the absorbance at a wavelength of 600 nm, reached 0.8. Subsequently, isopropyl-1-thio-β-D-galactoside (IPTG) was added so that the final concentration was 0.4 mM, and shaking culture was further continued at 25°C overnight. After culturing, the bacteria were collected by centrifugation and resuspended in a 50 mM tris-HCl buffer (pH 8.0). The resulting solution was subjected to ultrasonic disintegration while ice-cooling, and the supernatant was collected by centrifugation as a cell-free extract. The cell-free extract thus obtained was kept warm at 85°C for 15 minutes, and coagulation sedimentation of E. coli-derived proteins other than the glucosidase took place. The sediment was removed by centrifugation and the supernatant was dialyzed against a 50 mM acetate buffer (pH 5.0) through a dialysis membrane made of regenerated cellulose with a molecular weight cutoff of 10000 (manufactured by Spectrum Laboratories, Inc.). The protein solution thus obtained was used as the wild-type PfuBGL.

[Example 1] Determination of the N-linked glycosylation sequence-introduction site in PfuBGL

First of all, the determination of the primary sequence and the tertiary structure was attempted to search for the glycosylation sequence-introduction site in PfuBGL.

To perform alignment with respect to a PfuBGL homologue, the homologue search server FUGUE was used. As a result, in terms of ZSCORE, which indicates the homology of the sequence, PfuBGL exhibited a maximum score of 71.65 with respect to Glycosyl hydrolase family 1 (ZSCORE $\geq$ 6.0 indicates 99% confidence). In order to form the alignment of the Glycosyl hydrolase family 1 thus obtained, the JOY server was used. As a result, the sequence of PfuBGL corresponding to the position of the N-linked glycosylation sequence that is present at three sites in the β-glucosidase derived from *Aspergillus niger* (AspNgBGL) and in the β-glucosidase derived from *Trichoderma reesei* (TriReBGL) was found not to be the N-linked glycosylation sequence (three sites: H60, L61, and Y62, N148, L149, and Y150, and N374, G375, and M376).

An incomplete x-ray crystal structure of PfuBGL is reported (Thijis K. et al., Biochem. vol. 39, No. 17 (2000)), which has degradation ability of as low as 3.3 Å. A complete structural model has not yet been constructed, and such a model has not yet been registered at Protein Data Bank (PDB) either. In light of the above, in order to determine the tertiary structure of PfuBGL, detailed X-ray crystallography was attempted using new crystal conditions. New crystallization conditions were searched and crystallization was successfully achieved using phosphoric acid as a precipitating agent. An X-ray diffraction experiment was performed in the large synchrotron radiation facility SPring-8, and the structure of PfuBGL was determined with degradation ability of 2.5 Å, whereby a complete model of PfuBGL was successfully constructed. For structural determination, the molecular replacement method was used, and as a model molecule, the β-glucosidase derived from *Themosphaera aggregans* shown in SEQ ID NO: 8 (ThAggBGY, PDB ID: 1QVB) was used.

From the tertiary structure of PfuBGL thus obtained, in the aforementioned three sites corresponding to the position of the N-linked glycosylation sequence, a site that is exposed to the enzyme surface and not located near the active site, thereby presumably having a little impact on the enzyme activity, was only found in H60, L61, and Y62. Accordingly, this site was determined as the N-linked glycosylation sequence-introduction site, and a mutation of H60N, L61R, and Y62T was introduced by substitution, whereby the amino acid sequence shown in SEQ ID NO: 6 was obtained as the glycosylation mutant PfuBGL.

[Example 2] Determination of the N-linked glycosylation sequence-introduction site in a glucosidase having a homologous amino acid sequence to PfuBGL

To perform alignment between SEQ ID NO: 6 obtained in Example 1 and SEQ ID NO: 8, SEQ ID NO: 10, SEQ ID NO: 12, SEQ ID NO: 14, SEQ ID NO: 16, SEQ ID NO: 18, and SEQ ID NO: 20, an alignment was formed using ClustalW and BOXSHADE, both of which are software that is well-known to those skilled in the art (Figure 2-1 and Figure 2-2). In SEQ ID NO: 8, SEQ ID NO: 10, SEQ ID NO: 12, SEQ ID NO: 14, SEQ ID NO: 16, SEQ ID NO: 18, or SEQ ID NO: 20, a site corresponding to the glycosylation sequence Asn-Arg-Thr (N-R-T) in SEQ ID NO: 6 was determined as the glycosylation sequence-introduction site in each sequence.

[Example 3] Preparation of the glycosylated mutant PfuBGL (1)

The glycosylated mutant PfuBGL shown in SEQ ID NO: 6 was prepared.

First of all, using the mutation-introducing primers 5'-CCACATATTGGCACCTCTATAAGCAAGATCATG-3' (SEQ ID NO: 21) and 5'-CATGATCTTGCTTAGTGCGGTTCCAATATGCTGG-3' (SEQ ID NO: 22) for introducing the glycosylation sequence determined in Example 1 into PfuBGL shown in SEQ ID NO: 4, a N-linked glycosylation sequence mutation was introduced by site-directed mutagenesis. After confirming the sequence of the gene thus obtained, it was inserted between the EcoRI and NotI sites in the yeast expression vector pPIC9, which originally possesses the $\alpha$ factor secretion signal sequence. The mutation-introduced gene was used for transformation of E. coli, and the colony having the gene having the desired mutation was confirmed by a sequencer.

Yeast competent cells produced by a general technique were mixed with the mutant plasmids, whereby transformation was performed using GENE PULSERII (Bio-Rad Laboratories, Inc.) under the conditions of 1.7 kV, 25 $\mu$F, and 200 $\Omega$. The transformed yeast was streaked onto a RDB plate. Three days later, from the colonies that appeared on the plate, 10 colonies were selected and checked for the expression. A clone in which the expression of the desired protein was confirmed by polyacrylamide gel electrophoresis was selected.

As the seed culture, the yeast colony on the plate was inoculated into 2 mL of a BMGY medium and cultured for two days. Subsequently, as the main culture, 2 mL of the yeast seed culture liquid was added to 1 L of a BMGY medium and cultured for two days so as to allow yeast to sufficiently proliferate. Then, 1 L of the culture liquid was subjected to centrifugation once to precipitate yeast, and the BMGY medium was exchanged for a 2% methanol-containing BMMY medium. The yeast was then resuspended in the medium, followed by culturing for 48 hours. The medium containing the expressed protein was collected by centrifugation and filtered through a filter, followed by ammonium sulfate precipitation using 70% (w/v) ammonium sulfate. The precipitate was collected by centrifugation and dissolved in a buffer, and dialysis was performed and the desired protein was obtained.

The enzyme thus obtained was treated with EndoH and subjected to polyacrylamide gel electrophoresis (Figure 3). The preparation of the N-linked glycosylated mutant PfuBGL was confirmed by a band shift before and after the EndoH treatment.

[Example 4] Enzyme activity of the glycosylated mutant PfuBGL (2)

The glycosylated mutant PfuBGL and PfuBGL were compared for the β-glucosidase activity. Using a 10 mM cellobiose / 50 mM acetate buffer solution as a substrate, the enzymes prepared in Example 2 and Comparative Example 1 were each added at a final concentration of 0.23 mg/mL and the enzymatic reaction was carried out at 50°C. For quantitation of the product, Glucose Test Wako II (Wako Pure Chemical Industries, Inc.) was used.

One unit (U) of the β-glucosidase activity was calculated in accordance with the following formula.

**One unit (U) of the β-glucosidase activity = the concentration of glucose produced upon completion of the reaction (g/L) × 1000 / 180 / 30**.

Further, a specific β-glucosidase activity per the amount of β-glucosidase (mg) was calculated by the following formula.

**Specific β-glucosidase activity (U/mg protein) = β-glucosidase activity (U) / the amount of β-glucosidase added for the activity measurement**

PfuBGL produced 1.58 g/L, while the glycosylated mutant PfuBGL produced 1.34 g/L glucose upon completion of the reaction. Also, the specific activity of the glycosylated mutant PfuBGL was 85% relative to the specific activity of PfuBGL, clearing indicating that the introduction of the glycosylation sequence mutation into the site determined in Examine 1 did not cause a loss of the enzyme activity. From this, it was confirmed that the glycosylated mutant PfuBGL is utilizable in place of PfuBGL.

[Example 5] Heat stability of the glycosylated mutant PfuBGL

The changes in the amount of glucose produced up to 24 hours when the product was kept warm at 50°C, 60°C, 70°C, 80°C, and 90°C were measured.

The enzymes prepared in Example 2 and Comparative Example 1 (protein concentration of 1.0 mg/mL) were each kept warm at 50°C, 60°C, 70°C, 80°C, or 90°C. At each heat retention time, a substrate, which was a 10 mM cellobiose / 50 mM acetate buffer solution, was added, and the enzymatic reaction was carried out for 30 minutes at the heat retention temperature. The solution after the reaction was collected and the product was quantitated using Glucose Test Wako II (Wako Pure Chemical Industries, Inc.) (Figure 4 and Figure 5).

By heating up to 24 hours, compared to PfuBGL, the glycosylated mutant PfuBGL exhibited more reduced enzyme inactivation at a high temperature. This finding confirmed that the glycosylated mutant PfuBGL achieved improved enzyme stability by acquiring a higher heat resistance than PfuBGL.

[Reference Example 1] Preparation of lignocellulose

Lignocelluloses 1 to 3 to be used for the hydrolysis using an enzyme composition containing the glycosylated mutant glucosidase were prepared as follows.

1. Preparation of lignocellulose 1 (ammonia treatment)

As cellulose, rice straw was used. The above cellulose was placed in a small reactor (manufactured by Taiatsu Techno, TVS-N2 30ml) and cooled with liquid nitrogen. To this reactor, ammonia gas was infused and the sample was completely immersed in liquid ammonia. The reactor was capped and left at room temperature for about 15 minutes. Further, it was treated in an oil bath of 150°C for one hour. After the treatment, the reactor was taken out of the oil bath and ammonia gas was immediately leaked in a draft chamber. Subsequently, the inside of the reactor was dried by vacuuming down to 10 Pa by a vacuum pump. The resulting product was used in the following Examples as lignocellulose 1.

2. Preparation of lignocellulose 2 (diluted sulfuric acid treatment)

As cellulose, rice straw was used. The cellulose was immersed in a 1% aqueous solution of sulfuric acid, followed by autoclave treatment (manufactured by Nitto koatsu K.K.) at 150°C for 30 minutes. After the treatment, solid-liquid separation was performed to separate an aqueous solution of sulfuric acid (hereinbelow, diluted sulfuric acid treatment liquid) from sulfuric acid-treated cellulose. Subsequently, sulfuric acid-treated cellulose and the diluted sulfuric acid treatment liquid were mixed by stirring so that the solid content concentration was 10 wt.%. Subsequently, pH was adjusted to around 5 with sodium hydroxide. The resulting product was used in the following Examples as lignocellulose 2.

3. Preparation of lignocellulose 3 (hydrothermal treatment)

As cellulose, rice straw was used. The above cellulose was immersed in water and subjected to autoclave treatment (manufactured by Nitto koatsu K.K.) at 180°C for 20 minutes while stirring. In this treatment, the pressure was 10 MPa. After the treatment, the resulting product was subjected to solid-liquid separation by centrifugation (3000G) to separate the solution component (hereinbelow, the hydrothermal treatment solution) from the treated biomass component. The resulting treated biomass component was used in the following Examples as lignocellulose 3.

[Example 6] Hydrolysis of lignocellulose using the enzyme composition composed of a mixture of cellulases derived from filamentous fungi and the glycosylated mutant PfuBGL (1)

The changes in the amount of glucose produced by allowing the enzyme composition to act on the lignocellulose substrate were compared. A 50 mM acetate buffer (pH 5.0) in which 5 wt.% lignocellulose 1 (prepared in Reference Example 1) was suspended was used as a substrate. The reaction was allowed to proceed up to 28 hours at 50°C, and the reaction product was sampled as appropriate and measured for the glucose concentration (Figure 6). As the mixture of cellulases derived from filamentous fungi, commercially available cellulase derived from *Trichoderma reesei* (Celluclast, Sigma) was used. As glucosidase, the glycosylated mutant PfuBGL prepared in Example 2 and PfuBGL prepared in Comparative Example 1 were each used. Cellulase was added at 0.5 mg/mL and glucosidase was added at 0.005 mg/mL (1/100 of the amount of cellulase).

Comparing the use of PfuBGL and the use of the glycosylated mutant PfuBGL, the amount of glucose produced by the glycosylated mutant PfuBGL after 28 hours of reaction was greatly increased, and it was 1.6 times as much as the amount produced by PfuBGL. It was revealed that the addition of the glycosylated mutant PfuBGL only in an amount of 1/100 the mount of cellulase was tremendously effective for increasing the production amount of glucose.

[Reference Example 2] Preparation of a cellulase derived from *Trichoderma*

A cellulase derived from *Trichoderma* was prepared by the following method.

1. Preculture

The following substances were added to distilled water in the amounts indicated below; corn steep liquor 5% (w/vol), glucose 2% (w/vol), ammonium tartrate 0.37% (w/vol), ammonium sulfate 0.14% (w/vol), potassium dihydrogen phosphate 0.2% (w/vol), calcium chloride dihydrate 0.03% (w/vol), magnesium sulfate heptahydrate 0.03% (w/vol), zinc chloride 0.02% (w/vol), iron (III) chloride hexahydrate 0.01% (w/vol), copper(II) sulfate pentahydrate 0.004% (w/vol), manganese chloride tetrahydrate 0.0008% (w/vol), boric acid 0.0006% (w/vol), and hexaammonium heptamolybdate tetrahydrate 0.0026% (w/vol). Then, 100 mL of the resulting solution was poured into a 500 mL triangle flask with a baffle and sterilized by autoclaving at 121°C for 15 minutes. After naturally cooling, PE-M and Tween 80, which were each separately sterilized by autoclaving at 121°C for 15 minutes, were each added at 0.1%. To the resulting preculture medium, *Tricoderma reesei* ATCC66589 was inoculated at $1 \times 10^5$/mL, and preculture was performed by shaking at 28°C for 72 hours at 180 rpm (shaker: BIO-SHAKER BR-40LF manufactured by Taitec Corporation).

2. Main culture

The following substances were added to distilled water in the amounts indicated below; corn steep liquor 5% (w/vol), glucose 2% (w/vol), cellulose (Avicel) 10% (w/vol), ammonium tartrate 0.37% (w/vol), ammonium sulfate 0.14% (w/vol), potassium dihydrogen phosphate 0.2% (w/vol), calcium chloride dihydrate 0.03% (w/vol), magnesium sulfate heptahydrate 0.03% (w/vol), zinc chloride 0.02% (w/vol), iron (III) chloride hexahydrate 0.01% (w/vol), copper(II) sulfate pentahydrate 0.004% (w/vol), manganese chloride tetrahydrate 0.0008% (w/vol), boric acid 0.0006% (w/vol), and hexaammonium heptamolybdate tetrahydrate 0.0026% (w/vol). Then, 2.5 L of the resulting solution was poured into a 5 L stirring jar (DPC-2A manufactured by ABLE & Biott Co., Ltd.) and sterilized by autoclaving at 121°C for 15 minutes. After naturally cooling, PE-M and Tween 80, which were each separately sterilized by autoclaving at 121°C for 15 minutes, were each added at 0.1%. In this, 250 mL of *Tricoderma reesei* ATCC66589 that had been precultured in the liquid culture medium by the aforementioned method was inoculated. Thereafter, the fungi were cultured at 28°C for 87 hours at 300 rpm with an aeration rate of 1 vvm, followed by centrifugation, and the supernatant was filtered through a membrane (STERICUP-GV, material: PVDF, manufactured by Millipore Corporation).

[Example 7] Hydrolysis of lignocellulose using the enzyme composition composed of a mixture of cellulases derived from filamentous fungi and the glycosylated mutant PfuBGL (2)

Hydrolysis of lignocelluloses 1 to 3 was performed in the same manner as Example 6 except for using lignocelluloses 1 to 3 prepared in Reference Example 1 as the substrate, the culture liquid of *Trichoderma reesei* prepared in Reference Example 2 as the mixture of cellulases derived from filamentous fungi, and adding cellulase at 1.0 mg/mL and glucosidase at 0.005 mg/mL (1/100 of the amount of cellulase).

In Table 2, the concentrations of glucose (g/L) produced after 28 hours of reaction were compared.

[Table 2]

| Substrate | Concentration of glucose produced (g/L) | |
|---|---|---|
| | Wild-type PfuBGL | Glycosylated mutant PfuBGL |
| Lignocellulose 1 (Ammonia treatment) | 5g/L | 15g/L |
| Lignocellulose 2 (Diluted sulfuric acid treatment) | 3g/L | 16g/L |
| lignocellulose 3 (Hydrothermal treatment) | 4g/L | 18g/L |

Comparing the use of PfuBGL and the use of the glycosylated mutant PfuBGL, the amount of glucose produced by the glycosylated mutant PfuBGL after 28 hours of reaction was greatly increased, and it was 1.8 times as much as the amount produced by PfuBGL. It was revealed that the addition of the glycosylated mutant PfuBGL only in an amount of 1/200 the mount of cellulase produced an effect of greatly increasing the production amount of glucose. It was revealed that not only commercially available cellulase such as the one used in Example 6 but also the culture liquid of *Trichoderma reesei* exerted an effect in use with a glycosylated mutant.

[Example 8] Hydrolysis of lignocellulose using the enzyme composition composed of a mixture of cellulases derived from filamentous fungi and the glycosylated mutant PfuBGL (3)

Using the enzyme composition composed of a cellulase derived from filamentous fungi and the glycosylated mutant PfuBGL, the amounts of glucose produced were comparatively studied by varying the reaction temperature conditions of hydrolysis. By setting the reaction temperature at 30°C, 40°C, 50°C (Example 7), or 60°C, the hydrolysis was performed by a similar procedure to Example 7, and the amount of glucose produced after 28 hours was measured. As the substrate, lignocellulose 1 was used.

[Table 3]

| Reaction temperature of hydrolysis (°C) | Concentration of glucose produced (g/L) |
|---|---|
| 30°C | 5g/L |
| 40°C | 13g/L |
| 50°C (Example 7) | 15g/L |
| 60°C | 7g/L |

As shown in Table 3, it was revealed that it was preferable to set the reaction temperature in a range of 40°C to 50°C when a cellulase derived from filamentous fungi, particularly a cellulase derived from *Trichoderma* was used. This result reflects the fact that the optimum reaction temperature for a cellulase derived from *Trichoderma* is 40°C to 50°C. That is, it was revealed that although the glucosidase derived from a thermophile according to the present invention was still highly active at 50°C or higher, when it is used as an enzyme composition for saccharification of biomass containing a cellulase derived from filamentous fungi, the reaction was preferably carried out within a range of the optimum reaction temperature for the cellulase derived from filamentous fungi.

[Example 9] Hydrolysis of lignocellulose using the enzyme composition composed of a mixture of cellulases derived from filamentous fungi and the glycosylated mutant PfuBGL (4)

Using the enzyme composition composed of a cellulase derived from filamentous fungi and the glycosylated mutant PfuBGL, the amounts of glucose produced were comparatively studied by varying the pH conditions of the hydrolysis reaction. By setting the pH of the hydrolysis reaction to 1.2, 3.5, 5.0 (Example 7), 7.0, or 8.2 by the addition of diluted sulfuric acid, the hydrolysis was performed by a similar procedure to Example 7, and the amount of glucose produced after 28 hours was measured. As the substrate, lignocellulose 1 was used.

[Table 4]

| Reaction pH of hydrolysis | Concentration of glucose produced (g/L) |
|---|---|
| 1.2 | 2g/L |
| 3.5 | 12g/L |
| 5 (Example 7) | 15g/L |
| 7 | 10g/L |
| 8.2 | 5g/L |

As shown in Table 4, it was revealed that it was preferable to carry out the hydrolysis in a range of pH 3.5 to pH 7 when a cellulase derived from filamentous fungi, particularly a cellulase derived from *Trichoderma* is used.

[Comparative Example 2] Preparation of a glucosidase homologous to PfuBGL (2)

Seven kinds of glucosidases having homologous amino acid sequences to PfuBGL prepared in Comparative Example 1 were prepared as follows.

DNA sequences of SEQ ID NOs: 23, 25, 27, 29, 31, 33, and 35 were each entirely synthesized and incorporated into the cloning site of pET-11d (between NcoI and BamHI), whereby the expression vector for each of the above DNA was constructed. Subsequently, in a similar manner to Comparative Example 1, the wild-type glucosidases represented by SEQ ID NOs: 24, 26, 28, 30, 32, 34, and 36 (ThAggBGY, CmGHFP, SaBGAL, SsoBGAL, PtBGAL, TvBGAL, and FnGHFP) were obtained.

[Example 10] Preparation of a glycosylated mutant glucosidase homologous to PfuBGL

Based on the information of glycosylation sequence-introduction site in PfuBGL determined in Example 2, the sugar chain-introduction site in the wild-type glucosidases obtained in Comparative Example 2 (ThAggBGY, CmGHFP, SaBGAL, SsoBGAL, PtBGAL, TvBGAL, and FnGHFP) were determined, whereby the glycosylated mutant glucosidases were each prepared.

DNA sequences of SEQ ID NOs: 37, 39, 41, 43, 45, 47, and 49 encoding glycosylated mutant glucosidase were each entirely synthesized and incorporated between EcoRI and NotI of the pCU9 vector, whereby the expression vector for each of the above DNA was constructed. Subsequently, in a similar manner to Example 3, glycosylated mutant glucosidases represented by the amino acid sequences SEQ ID NOs: 38, 40, 42, 44, 46, 48, and 50 were obtained.

[Example 11] Enzyme activity of the glycosylated mutant glucosidase

The enzyme activity of the glycosylated mutant glucosidases obtained in Example 10 was measured in a similar manner to Example 4 and compared with the enzyme activity of each wild-type glucosidase obtained in Comparative Example 2. Setting the enzyme activity of each wild-type glucosidase at 100, the enzyme activity of the glycosylated mutant was shown as relative activity (%) in Table 5.

[Table 5]

| Entry No. | SEQ ID NO | Glucosidase | Relative activity (%) of glycosylated mutant glucosidase to wild-type |
|---|---|---|---|
| 1 (Example 4) | 6 | PfuBGL | 85% |
| 2 | 38 | ThAggBGY | 90% |
| 3 | 40 | CmGHFP | 83% |
| 4 | 42 | SaBGAL | 87% |
| 5 | 44 | SsoBGAL | 81% |
| 6 | 46 | PtBGAL | 85% |
| 7 | 48 | TvBGAL | 93% |
| 8 | 50 | FnGHFP | 78% |

In Table 5, the glycosylated mutant glucosidases were found to retain the enzyme activity in comparison with the wild-type glucosidase before glycosylation.

[Example 12] Heat stability of the glycosylated mutant glucosidase

In a similar manner to Example 5, a heat stability test was performed on each wild-type glucosidase obtained in Comparative Example 2 and on each glycosylated mutant glucosidase obtained in Example 10. Regarding the heat stability, by setting the enzyme activity before applying heat at 100, the residual activity at each a heat retention time was shown as relative activity in Figures 8 to 20. It was revealed that in all glucosidases, the glycosylated mutants (Figure 9, Figure 11, Figure 13, Figure 15, Figure 17, Figure 19, and Figure 21) exhibited improved heat stability compared to the wild-type (Figure 8, Figure 10, Figure 12, Figure 14, Figure 16, Figure 18, and Figure 20).

[Example 13] Hydrolysis of lignocellulose using the enzyme composition composed of a mixture of cellulases derived from filamentous fungi and the glycosylated mutant glucosidase (5)

In a similar manner to Example 6, using each wild-type glucosidase obtained in Comparative Example 2 and each glycosylated mutant glucosidase obtained in Example 10, hydrolysis of lignocellulose 1 (ammonia treatment) prepared in Reference Example 1 was carried out. The amount of glucose produced by each glucosidase after 28 hours of reaction (g/L) was shown in Table 6.

[Table 6]

| Entry No. | Glucosidase | Concentration of glucose produced (g/L) | |
|---|---|---|---|
| | | Wild-type glucosidase (Comparative Example 2) | Glycosylated mutant glucosidase (Example 10) |
| 2 | ThAggBGY | 5g/L | 10g/L |
| 3 | CmGHFP | 4g/L | 9g/L |
| 4 | SaBGAL | 5g/L | 11g/L |
| 5 | SsoBGAL | 6g/L | 12g/L |

(continued)

| Entry No. | Glucosidase | Concentration of glucose produced (g/L) | |
|---|---|---|---|
| | | Wild-type glucosidase (Comparative Example 2) | Glycosylated mutant glucosidase (Example 10) |
| 6 | PtBGAL | 5g/L | 9g/L |
| 7 | TvBGAL | 4g/L | 10g/L |
| 8 | FnGHFP | 6g/L | 13g/L |

As shown in Table 6, in the hydrolysis of ammonia-treated lignocellulose 1, the sugar production by the glycosylated mutant glucosidases per unit time was greatly increased compared to the wild-type, confirming that the glycosylated mutant glucosidases had excellent cellulose degradation efficiency.

[Example 14] Hydrolysis of lignocellulose using the enzyme composition composed of a mixture of cellulases derived from filamentous fungi and the glycosylated mutant glucosidase (6)

In a similar manner to Example 6, using each wild-type glucosidase obtained in Comparative Example 2 and each glycosylated mutant glucosidase obtained in Example 10, hydrolysis of lignocellulose 2 (diluted sulfuric acid treatment) prepared in Reference Example 1 was carried out. The amount of glucose produced by each glucosidase after 28 hours of reaction (g/L) was shown in Table 7.

[Table 7]

| Entry No. | Glucosidase | Concentration of glucose produced (g/L) | |
|---|---|---|---|
| | | Wild-type glucosidase (Comparative Example 2) | Glycosylated mutant glucosidase (Example 10) |
| 2 | ThAggBGY | 5g/L | 10g/L |
| 3 | CmGHFP | 4g/L | 9g/L |
| 4 | SaBGAL | 5g/L | 11g/L |
| 5 | SsoBGAL | 6g/L | 12g/L |
| 6 | PtBGAL | 5g/L | 9g/L |
| 7 | TvBGAL | 4g/L | 10g/L |
| 8 | FnGHFP | 6g/L | 13g/L |

As shown in Table 7, in the hydrolysis of diluted sulfuric acid-treated lignocellulose 2, the sugar production by the glycosylated mutant glucosidases per unit time was greatly increased compared to the wild-type, confirming that the glycosylated mutant glucosidases had excellent cellulose degradation efficiency.

[Example 15] Hydrolysis of lignocellulose using the enzyme composition composed of a mixture of cellulases derived from filamentous fungi and the glycosylated mutant glucosidase (7)

In a similar manner to Example 6, using each wild-type glucosidase obtained in Comparative Example 1 and Comparative Example 2 and each glycosylated mutant - glucosidase obtained in Example 1 and Example 10, hydrolysis of lignocellulose 3 (hydrothermal treatment) prepared in Reference Example 1 was carried out. The amount of glucose produced by each glucosidase after 28 hours of reaction (g/L) was shown in Table 8.

[Table 8]

| Entry No. | Glucosidase | Concentration of glucose produced (g/L) | |
|---|---|---|---|
| | | Wild-type glucosidase (Comparative Example 1,2) | Glycosylated mutant glucosidase (Example 1,10) |
| 1 | PfuBGL | 4g/L | 11g/L |

(continued)

| Entry No. | Glucosidase | Concentration of glucose produced (g/L) | |
|---|---|---|---|
| | | Wild-type glucosidase (Comparative Example 1,2) | Glycosylated mutant glucosidase (Example 1,10) |
| 2 | ThAggBGY | 4g/L | 12g/L |
| 3 | CmGHFP | 5g/L | 14g/L |
| 4 | SaBGAL | 5g/L | 14g/L |
| 5 | SsoBGAL | 4g/L | 12g/L |
| 6 | PtBGAL | 5g/L | 15g/L |
| 7 | TvBGAL | 4g/L | 13g/L |
| 8 | FnGHFP | 6g/L | 15g/L |

As shown in Table 8, in the hydrolysis of hydrothermally treated lignocellulose 3, the sugar production by the glycosylated mutant glucosidases per unit time was greatly increased compared to the wild-type, confirming that the glycosylated mutant glucosidases had excellent cellulose degradation efficiency.

[Example 16] Hydrolysis of industrial pulp using the enzyme composition composed of a mixture of cellulases derived from filamentous fungi and the glycosylated mutant glucosidase

In the hydrolysis of industrial pulp, the amount of glucose produced by the action of each wild-type glucosidase obtained in Comparative Example 1 and Comparative Example 2 and that produced by the action of each glycosylated mutant glucosidase obtained in Example 1 and Example 10 were comparatively studied. Hydrolysis was performed in the same manner as Example 6 except for using a 50 mM acetate buffer (pH 5.0) in which 5 wt.% industrial pulp (manufactured by Toa Kasei Co., Ltd.) was suspended as a substrate. The amount of glucose produced by each glucosidase after 28 hours of reaction (g/L) was shown in Table 9.

[Table 9]

| Entry No. | Glucosidase | Concentration of glucose produced (g/L) | |
|---|---|---|---|
| | | Wild-type glucosidase (Comparative Example 1,2) | Glycosylated mutant glucosidase (Example 1,10) |
| 1 | PfuBGL | 9g/L | 12g/L |
| 2 | ThAggBGY | 8g/L | 11g/L |
| 3 | CmGHFP | 8g/L | 11g/L |
| 4 | SaBGAL | 10g/L | 13g/L |
| 5 | SsoBGAL | 10g/L | 12g/L |
| 6 | PtBGAL | 9g/L | 11g/L |
| 7 | TvBGAL | 8g/L | 10g/L |
| 8 | FnGHFP | 11g/L | 13g/L |

As shown in Table 9, also in the hydrolysis of industrial pulp, the sugar production by the glycosylated mutant glucosidases per unit time was greatly increased compared to the wild-type, confirming that the glycosylated mutant glucosidases had excellent cellulose degradation efficiency.

[Example 17] Selection of the glycosylation mutation-induction site

Based on the information of the tertiary structure of PfuBGL obtained in Example 1, a further search for glycosylation site was conducted. Protection of the catalytic site by glycosylation was attempted by introducing the glycosylation sequence around the cleft where the enzymatic reaction takes place. First of all, the potential glycosylation mutation-introduction site was limited to, in the part other than the sugar chain mutation-introduction site in PfuBGL described in Example 1, the part that is exposed to the surface of the enzyme. Subsequently, such a position that would not cause the steric hindrance and/or structural deformation by introduction of mutation of the glycosylation sequence Asn-Xaa-Thr (N-X-T) was searched. Also, in the part where the active site of glucosidase was avoided, three mutation-introduction sites were selected.

As a result, in addition to the glycosylation site selected in Example 1, three mutation-introduction sites were newly selected, which were H37-D38-K39 (mutation A), S230-F231-E232 (mutation C), and A364-Y365-E366 (mutation E).

[Example 18] Preparation of the glycosylated mutant PfuBGL (2)

In order to introduce the glycosylation sequence into the mutation-introduction site determined in Example 17 in the glycosylated mutant PfuBGL shown in SEQ ID NO: 6, using primers for introducing mutation A shown in SEQ ID NO: 57 and SEQ ID NO: 58, N-linked glycosylation sequence mutation was introduced by site-directed mutagenesis. As a result, the glycosylated mutant PfuBGL2A gene of SEQ ID NO: 51 was obtained. Also, as to mutation C and mutation E, using primers for introducing mutation C shown in SEQ ID NO: 59 and SEQ ID NO: 60 and primers for introducing mutation E shown in SEQ ID NO: 61 and SEQ ID NO: 62, respectively, N-linked glycosylation sequence mutation was introduced by site-directed mutagenesis in a similar manner, whereby the glycosylated mutant PfuBGL2C gene shown in SEQ ID NO: 53 and the glycosylated mutant PfuBGL2E gene shown in SEQ ID NO: 55 were each obtained. Using the glycosylated mutant genes prepared as above, the glycosylated mutant PfuBGL2A represented by SEQ ID NO: 52, the glycosylated mutant PfuBGL2C represented SEQ ID NO: 54, and the glycosylated mutant PfuBGL2E represented by SEQ ID NO:56 were each obtained by following the same steps as in Example 3.

[Example 19] Enzyme activity of the glycosylated mutant PfuBGL (2)

In a similar manner to Example 4, the enzyme activity of the glycosylated mutant glucosidase PfuBGL2A (SEQ ID NO: 52), the glycosylated mutant PfuBGL2C (SEQ ID NO: 54), and the glycosylated mutant PfuBGL2E (SEQ ID NO: 56) obtained in Example 18 was measured in comparison with the wild-type PfuBGL. Setting the enzyme activity of the wild-type PfuBGL at 100, the enzyme activity of the glycosylated mutant was shown as relative activity (%) in Table 10.

[Table 10]

| Entry No. | SEQ ID NO | Glucosidase | Relative activity (%) of glycosylated mutant glucosidase to wild-type |
|---|---|---|---|
| 9 | 52 | PfuBGL2A | 84 |
| 10 | 54 | PfuBGL2C | 81 |
| 11 | 56 | PfuBGL2E | 85 |

As shown in Table 10, it was found that the mutant glucosidase having two or more sugar chains attached thereto retained the enzyme activity in comparison with the wild-type glucosidase before glycosylation.

[Example 20] Hydrolysis of lignocellulose using the enzyme composition composed of a mixture of cellulases derived from filamentous fungi and various kinds of glycosylated mutant glucosidases (8)

In a similar manner to Example 6, using the glycosylated mutant glucosidases (PfuBGL2A, PfuBGL2C, and PfuBGL2E) obtained in Example 18, hydrolysis of lignocellulose 2 (diluted sulfuric acid treatment) prepared in Reference Example 1 was carried out. The amount of glucose produced after 28 hours of reaction (g/L) was shown in Table 11.
[Table 11]

| Entry No. | Glucosidase | Concentration of glucose produced (g/L) | |
|---|---|---|---|
| | | Wild-type glucosidase (Comparative Example 1) | Glycosylated mutant glucosidase (Example 18) |
| 1 | PfuBGL | 3g/L | - |
| 9 | PfuBGL2A | - | 18g/L |
| 10 | PfuBGL2C | - | 18g/L |

(continued)

| Entry No. | Glucosidase | Concentration of glucose produced (g/L) | |
|---|---|---|---|
| | | Wild-type glucosidase (Comparative Example 1) | Glycosylated mutant glucosidase (Example 18) |
| 11 | PfuBGL2E | - | 19g/L |

As shown in Table 11, it was revealed that the mutant glucosidases having two or more sugar chains attached thereto (SEQ ID NO: 52, SEQ ID NO: 54, and SEQ ID NO: 56) also had greatly improved lignocellulose degradation efficiency compared to an unglycosylated wild-type glucosidase (Comparative Example 1; the amount of glucose produced: 3 g/L).

[Example 21] Evaluation of the adsorptivity of the glycosylated mutant glucosidase to lignocellulose

The adsorptivity of the glycosylated mutant glucosidase to crystalline cellulose (Avicel) and to lignocelluloses 1 to 3 prepared in Reference Example 1 was evaluated. As the glucosidase, the glycosylated mutant PfuBGL prepared in Example 1 and the wild-type PfuBGL prepared in Comparative Example 1 were used, and the enzyme solutions were adjusted so as to have equal specific activities, and further prepared at a final concentration of 100 mM by the addition of an acetate buffer (pH 5). The resulting enzyme solutions were each added to the crystalline cellulose and to lignocelluloses 1 to 3 at a final concentration (solid concentration) of 7.5 wt.%, and the resulting mixtures were kept warm and stirred at 50°C for one hour. Subsequently, the mixtures after reaction were centrifuged at 15000 rpm for 10 minutes, and the supernatant thus obtained was measured for the cellobiose degradation activity. Setting the enzyme activity of an enzyme solution without crystalline cellulose and lignocelluloses 1 to 3 at 100, the cellobiose degradation activity was evaluated in terms of relative activity (%).

[Table 12]

| Cellulose | Relative activity (%) of cellobiose degradation activity (%) | |
|---|---|---|
| | Wild-type PfuBGL | Glycosylated mutant PfuBGL |
| Crystalline cellulose (Avicel) | 85% | 95% |
| Lignocellulose 1 (Diluted sulfuric acid treatment) | 23% | 84% |
| Lignocellulose 2 (Ammonia treatment) | 62% | 84% |
| Lignocellulose 3 (Hydrothermal treatment) | 34% | 81% |

As shown in Table 12, it was revealed that the cellobiose degradation activity in the supernatant of the glycosylated mutant glucosidase was increased in the presence of any of the lignocelluloses 1 to 3, compared to the wild-type glucosidase. Also, the cellobiose degradation activity in the supernatant was slightly increased also for Avicel, which was crystalline cellulose. From the above results, it was revealed that the glycosylated mutant glucosidase had low adsorptivity particularly for lignocellulose (pre-treatment cellulose product), and thus would be recovered with good yield from the supernatant after solid-liquid separation.

[Example 22] Evaluation of the adsorptivity of the glycosylated mutant glucosidase to an ultrafiltration membrane

The adsorptivity of the glycosylated mutant glucosidase to an ultrafiltration membrane was evaluated. As the ultrafiltration membrane, VIVASPIN20 (SARTORIUS K.K., made of polyethersulfone, a molecular weight cut off of 10000 Da) was used. As the glucosidase, the glycosylated mutant PfuBGL prepared in Example 1 and the PfuBGL prepared in Comparative Example 1 were used, and the enzyme solutions were adjusted so as to have equal specific activities, and further prepared at a final concentration of 50 mM by the addition of an acetate buffer (pH 5). From these enzyme solutions, 5 mL was transferred to the ultrafiltration membrane VIVASPIN20, followed by centrifugation at 4000 G for 10 minutes. After centrifugation, to the solution remained on the non-permeable side of the ultrafiltration membrane (about 100 μL or less), 3 mL of a 50 mM acetate buffer was added, and the residual products left on the surface of the ultrafiltration membrane and on the inner wall of the container were collected by pipetting. The residual product thus collected was made up to 5 mL, and from this, 10 μL was sampled for the cellobiose degradation activity. The above series of operations was repeated seven times, and setting the initial activity at 100(%), the cellobiose degradation activity was calculated as relative activity (%) at each operation. The results thus obtained are shown in Table 13.

[Table 13]

| Number of repetition of operation | Relative activity (%) of cellobiose degradation activity (%) | |
|---|---|---|
| | Wild-type PfuBGL | Glycosylated mutant PfuBGL |
| 0 (initial) | 100% | 100% |
| 1 | 21% | 114% |
| 2 | 5.4% | 115% |
| 3 | 1.8% | 112% |
| 4 | ND | 116% |
| 5 | ND | 110% |
| 6 | ND | 101% |
| 7 | ND | 98% |

As shown in Table 13, it was revealed that the activity of the unglycosylated wild-type PfuBGL was decreased as the number of repetition of the operation was increased (the activity disappeared at the fourth and subsequent operations). Meanwhile, the glycosylated mutant PfuBGL remained active even after seven repeated operations. That is, it was revealed that the glycosylated mutant glucosidase had low adsorptivity for the ultrafiltration membrane, and thus was recovered with good yield when it was recovered using an ultrafiltration membrane after the hydrolysis reaction.

[Example 23] Recovery of the enzyme composition in the hydrolysate using an ultrafiltration membrane

From the product of hydrolysis of lignocelluloses 1 to 3 in Example 7, the enzyme composition was recovered as follows. First of all, 10 mL of the hydrolysate was centrifuged and 5 mL of the supernatant was obtained. Subsequently, the supernatant was filtered through a precise membrane filter having an average pore diameter of 0.2 μm (PVDF membrane, manufactured by Millipore Corporation), and the filtrate was collected. The total volume of the filtrate thus obtained was transferred to the ultrafiltration membrane VIVASPIN20 (made of polyethersulfone, a molecular weight cutoff of 10000 Da), followed by centrifugation. The residual product remained on the non-permeable side of the ultrafiltration membrane was collected and measured for the cellobiose degradation activity. By setting the activity of the introduced enzyme at the initial enzyme activity of 100%, the enzyme activity of the enzyme composition recovered as the residual product was calculated in terms of relative value (%).

[Table 14]

| Cellulose | Relative activity (%) of cellobiose degradation activity of the recovered enzyme | |
|---|---|---|
| | Wild-type PfuBGL | Glycosylated mutant PfuBGL |
| Lignocellulose 1 (Diluted sulfuric acid treatment) | 21% | 69% |
| Lignocellulose 2 (Ammonia treatment) | 11% | 51% |
| Lignocellulose 3 (Hydrothermal treatment) | 7.7% | 48% |

As shown in Table 14, it was revealed that the activity of the recovered glycosylated mutant glucosidase was greatly increased compared to the mutant glucosidase. This was assumed to be attributable to decreased adsorptivity of the glycosylated mutant glucosidase for lignocellulose as demonstrated in Example 21, and also to decreased adsorptivity of the glycosylated mutant glucosidase for the ultrafiltration membrane as demonstrated in Example 22.

[Example 24] Analysis of the sugar chain component of the glycosylated mutant glucosidase

The sugar chain structure of the glycosylated PfuBGL described in Example 1 was analyzed. To 1.33 mg of a sample, which was freeze-dried (-80°C) glycosylated PfuBGL, 1.33 mL of purified water was added to prepare a 1 mg/mL sample solution. The neutral sugar and amino sugar in this sample solution were quantitated by the following procedure.

1. Neutral sugar

Into a test tube, 100 μL of the 1 mg/mL sample solution was transferred, which was dried under reduced pressure, to which 200 μL of 2 M trifluoroacetic acid was added. The resulting test tube was subjected to nitrogen substitution and then sealed under reduced pressure. Subsequently, the hydrolysis was performed at 100°C for six hours, and the resulting product was dried again under reduced pressure. To the residue thus obtained, 200 μL of purified water was added, in which the residue was dissolved. The resulting solution was filtered through a filter of 0.22 μm. A sample obtained by diluting the filtrate 10-fold with purified water was analyzed under the following conditions.

As the analytical instrument, the HPLC system LC20A system (Shimadzu Corporation) and the spectrofluorometer RF-10AXL (Shimadzu Corporation) were used.

The analytical conditions were as follows; TSK-gel Sugar AXG 4.6 mm I.D. x 15 cm (Tosoh Corporation), the column temperature, 70°C, the mobile phase, a 0.6 M potassium borate buffer (pH 8.7), and the mobile phase flow rate, 0.4 mL/min.

Using 1 wt.% arginine and 3 wt% boric acid as the reaction reagent, post-column labeling was performed at a reaction reagent flow rate of 0.5 mL/min and a reaction temperature of 150°C. Also, as to the detection wavelength, the excitation and the detection were performed at 320 nm and 430 nm, respectively. The neutral sugar was quantitated in comparison with each standard product of neutral sugar.

2. Amino sugar

Into a test tube, 100 μL of the 1 mg/mL sample solution was transferred, which was dried under reduced pressure, to which 200 μL of 4 M trihydrochloric acid was added. The resulting test tube was subjected to nitrogen substitution and then sealed under reduced pressure. Subsequently, the hydrolysis was performed at 100°C for six hours, and then the resulting product was dried again under reduced pressure. To the residue thus obtained, 200 μL of purified water was added, in which the residue was dissolved. The resulting solution was filtered through a filter of 0.22 μm.

As the analytical instrument, the HPLC system LC20A system (Shimadzu Corporation) and the spectrofluorometer RF-10AXL (Shimadzu Corporation) were used.

The analytical conditions were as follows; TSK-gel SCX 6 mm I.D. x 15 cm (Tosoh Corporation), the column temperature, 60°C, the mobile phase, a 0.16 M boric acid potassium borate buffer (pH 7.6), and the mobile phase flow rate, 0.3 mL/min.

Using 1 wt.% arginine and 3 wt.% boric acid as the reaction reagent, post-column labeling was performed at a reaction reagent flow rate of 0.5 mL/min and a reaction temperature of 150°C. Also, as to the detection wavelength, the excitation and the detection were performed at 320 nm and 430 nm, respectively. The neutral sugar was quantitated in comparison with each standard product of neutral sugar.

[Table 15] c

| Sugar | | Concentration | Composition ratio |
|---|---|---|---|
| | | (nmol/mg) | (*1) |
| Neutral sugar | Rhamnose | 30 | 1 |
| | Ribose | ND | - |
| | Mannose | 576 | 18 |
| | Arabinose | 24 | 1 |
| | Galactose | 75 | 2 |
| | Xylose | ND | - |
| | Glucose | 54 | 2 |
| Amino sugar | Glucosamine | 62 | 2 |
| | Galactosamine | 37 | 1 |
| (*1) By setting the value of the concentration B of glucosamine at "2" as the standard, the concentrations B of other sugars were calculated as relative ratio. In calculating, the digits after the decimal point were rounded to an integer. | | | |

As shown in Table 15, it was revealed that the main component of the neutral sugar composing the sugar chain of the sugar chain mutant PfuBGL obtained in Example 1 was mannose. Also, from the composition ratio of mannose to N-glucosamine, the sugar chain mutant PfuBGL was found to have a high mannose type sugar chain.

Industrial Applicability

The glycosylated mutant glucosidase derived from a thermophile according to the present invention can be used for the production of a sugar liquid by cellulose degradation. Because the glycosylated mutant glucosidase derived from a thermophile has an effect of greatly increasing the cellulose degradation efficiency, it is capable of considerably reducing the cost of enzyme. In light of the above, the glycosylated mutant glucosidase derived from a thermophile according to the present invention is extremely industrially beneficial.

## Claims

1. A method for producing a mutant β-glucosidase derived from a thermophile that has selectively attached thereto a sugar chain and also has a β-glucosidase activity, comprising:

   (i) preparing DNA encoding a mutant β-glucosidase derived from a thermophile by introducing a DNA sequence encoding Asn-X-Ser or Asn-X-Thr (wherein, X is any amino acid except proline) into DNA encoding a β-glucosidase derived from a thermophile that is originally devoid of a glycosylation sequence, and further, adding a DNA sequence encoding a secretion signal sequence to the DNA encoding a mutant β-glucosidase,
   (ii) introducing the DNA encoding a mutant β-glucosidase to which the DNA sequence encoding the secretion signal sequence has been added into an eukaryotic microorganism so that a mutant β-glucosidase encoded by the DNA of the mutant β-glucosidase is expressed as a secretory protein, and
   (iii) isolating and purifying the mutant β-glucosidase thus expressed as a secretory protein.

2. The method for producing a mutant β-glucosidase according to Claim 1, wherein the sugar chain is a high mannose type sugar chain.

3. The method for producing a mutant β-glucosidase according to Claim 1 or 2, wherein the β-glucosidase derived from a thermophile is a β-glucosidase derived from a thermophile selected from the group consisting of the genus Sulfolobus, the genus Thermoplasma, the genus Caldivirga, the genus Thermosphaera, the genus Pyrococcus, the genus Picrophilus, and the genus Fervidobacterium.

4. The method for producing a mutant β-glucosidase according to any one of Claim 1 to 3, wherein the β-glucosidase derived from a thermophile is a protein comprising:

   (i) a same amino acid sequence as any of amino acid sequences shown in SEQ ID NO: 4, SEQ ID NO: 8, SEQ ID NO: 10, SEQ ID NO: 12, SEQ ID NO: 14, SEQ ID NO: 16, SEQ ID NO: 18, and SEQ ID NO: 20, or
   (ii) an amino acid sequence having 85% or more identity with any of amino acid sequences shown in SEQ ID NO: 4, SEQ ID NO: 8, SEQ ID NO: 10, SEQ ID NO: 12, SEQ ID NO: 14, SEQ ID NO: 16, SEQ ID NO: 18, and SEQ ID NO: 20,and also β-glucosidase activity.

5. The method for producing a mutant β-glucosidase according to any one of Claim 1 to 4, wherein the eukaryotic microorganism is Pichia pastoris.

6. The method for producing a mutant β-glucosidase according to any one of Claims 1 to 5, wherein the secretion signal sequence is an α-factor secretion signal sequence.

7. The method for producing a mutant β-glucosidase according to any one of Claims 1 to 6, wherein the mutant β-glucosidase derived from a thermophile comprises an amino acid sequence shown in any of SEQ ID NO: 6, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, and SEQ ID NO: 56.

8. An enzyme composition for saccharification of biomass comprising cellulase and the mutant β-glucosidase derived from a thermophile obtained by the production method according to any one of Claims 1 to 7.

9. The enzyme composition for saccharification of biomass according to Claim 8, wherein the cellulase is a mixture of cellulases derived from filamentous fungi.

10. The enzyme composition for saccharification of biomass according to Claim 8 or 9, wherein the mixture of cellulases derived from filamentous fungi is a mixture of cellulases derived from the genus Trichoderma.

11. A method for hydrolyzing biomass, comprising using the enzyme composition according to any one of Claims 8 to 10.

12. The method for hydrolyzing biomass according to Claim 11, comprising filtering a hydrolysate obtained by the enzyme composition through an ultrafiltration membrane, and separating and recovering the used enzyme composition.

## Patentansprüche

1. Verfahren zur Herstellung einer mutierten β-Glukosidase, die aus einem Thermophilen stammt, an die selektiv eine Zuckerkette gebunden ist und die auch eine β-Glukosidaseaktivität hat, umfassend:

   (i) Herstellen von DNA, die für eine mutierte β-Glukosidase, die aus einem Thermophilen stammt, kodiert, indem eine DNA-Sequenz, die für Asn-X-Ser oder Asn-X-Thr kodiert (wobei X irgendeine Aminosäure außer Prolin ist), in die DNA, die für eine β-Glukosidase, die aus einem Thermophilen stammt und welche ursprünglich keine Glykosylierungssequenz hat, eingeführt wird und indem ferner eine DNA-Sequenz, die für eine Sekretionssignalsequenz kodiert, zu der DNA, die für eine mutierte β-Glukosidase kodiert, hinzugefügt wird,
   (ii) Einführen der DNA, die für eine mutierte β-Glukosidase kodiert und zu der die DNA-Sequenz, die für die Sekretionssignalsequenz kodiert, hinzugefügt worden ist, in einen eukaryotischen Mikroorganismus, so dass eine mutierte β-Glukosidase, die von der DNA der mutierten β-Glukosidase kodiert wird, als ein sekretorisches Protein exprimiert wird, und
   (iii) Isolieren und Aufreinigen der mutierten β-Glukosidase, die auf diese Weise als ein sekretorisches Protein exprimiert wird.

2. Verfahren zur Herstellung einer mutierten β-Glukosidase nach Anspruch 1, wobei die Zuckerkette eine Zuckerkette des mannosereichen Typs ist.

3. Verfahren zur Herstellung einer mutierten β-Glukosidase nach Anspruch 1 oder 2, wobei die β-Glukosidase, die aus einem Thermophilen stammt, eine β-Glukosidase ist, die aus einem Thermophilen stammt, der aus der Gruppe, bestehend aus der Gattung Sulfolobus, der Gattung Thermoplasma, der Gattung Caldivirga, der Gattung Thermosphaera, der Gattung Pyrococcus, der Gattung Picrophilus und der Gattung Fervidobacterium, ausgewählt ist.

4. Verfahren zur Herstellung einer mutierten β-Glukosidase nach einem der Ansprüche 1 bis 3, wobei die β-Glukosidase, die aus einem Thermophilen stammt, ein Protein ist, umfassend:

   (i) eine selbe Aminosäuresequenz, wie irgendeine von den Aminosäuresequenzen, die in SEQ ID NO: 4, SEQ ID NO: 8, SEQ ID NO: 10, SEQ ID NO: 12, SEQ ID NO: 14, SEQ ID NO: 16, SEQ ID NO: 18 und SEQ ID NO: 20 gezeigt sind, oder
   (ii) eine Aminosäuresequenz, die eine Identität von 85 % oder mehr mit irgendeiner von den Aminosäuresequenzen hat, die in SEQ ID NO: 4, SEQ ID NO: 8, SEQ ID NO: 10, SEQ ID NO: 12, SEQ ID NO: 14, SEQ ID NO: 16, SEQ ID NO: 18 und SEQ ID NO: 20 gezeigt sind, und auch eine β-Glukosidaseaktivität.

5. Verfahren zur Herstellung einer mutierten β-Glukosidase nach einem der Ansprüche 1 bis 4, wobei der eukaryotische Mikroorganismus Pichia pastoris ist.

6. Verfahren zur Herstellung einer mutierten β-Glukosidase nach einem der Ansprüche 1 bis 5, wobei die Sekretionssignalsequenz eine α-Faktor-Sekretionssignalsequenz ist.

7. Verfahren zur Herstellung einer mutierten β-Glukosidase nach einem der Ansprüche 1 bis 6, wobei die mutierte β-Glukosidase, die aus einem Thermophilen stammt, eine Aminosäuresequenz umfasst, die in irgendeiner von SEQ ID NO: 6, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54 und SEQ ID NO: 56 gezeigt ist.

8. Enzymzusammensetzung zur Verzuckerung von Biomasse, umfassend Cellulase und die mutierte β-Glukosidase, die aus einem Thermophilen stammt und durch das Herstellungsverfahren nach einem der Ansprüche 1 bis 7 erhalten wird.

9. Enzymzusammensetzung zur Verzuckerung von Biomasse nach Anspruch 8, wobei die Cellulase eine Mischung aus Cellulasen ist, die aus filamentösen Pilzen stammen.

10. Enzymzusammensetzung zur Verzuckerung von Biomasse nach Anspruch 8 oder 9, wobei die Mischung aus Cellulasen, die aus filamentösen Pilzen stammen, eine Mischung aus Cellulasen ist, die aus der Gattung Trichoderma stammen.

11. Verfahren zum Hydrolysieren von Biomasse, umfassend die Verwendung der Enzymzusammensetzung nach einem der Ansprüche 8 bis 10.

12. Verfahren zum Hydrolysieren von Biomasse nach Anspruch 11, umfassend das Filtern eines Hydrolysats, das durch die Enzymzusammensetzung erhalten wurde, durch eine Ultrafiltrationsmembran und das Trennen und Rückgewinnen der verwendeten Enzymzusammensetzung.

**Revendications**

1. Procédé de production d'une β-glucosidase mutante dérivée d'un thermophile sur laquelle est sélectivement fixée une chaîne glucidique et qui présente également une activité β-glucosidase, comprenant :

   (i) la préparation d'un ADN codant pour une β-glucosidase mutante dérivée d'un thermophile par l'introduction d'une séquence d'ADN codant pour Asn-X-Ser ou Asn-X-Thr (formules dans lesquelles X représente tout acide aminé à l'exception de la proline) dans un ADN codant pour une β-glucosidase dérivée d'un thermophile qui est à l'origine exempt d'une séquence de glycosylation, et en outre l'addition d'une séquence d'ADN codant pour une séquence signal de sécrétion à l'ADN codant pour une β-glucosidase mutante,
   (ii) l'introduction de l'ADN codant pour une β-glucosidase mutante auquel la séquence d'ADN codant pour la séquence signal de sécrétion a été ajoutée dans un microorganisme eucaryote de sorte qu'une β-glucosidase mutante codée par l'ADN de la β-glucosidase mutante soit exprimée sous la forme d'une protéine sécrétoire, et
   (iii) l'isolement et la purification de la β-glucosidase mutante ainsi exprimée sous la forme d'une protéine sécrétoire.

2. Procédé de production d'une β-glucosidase mutante selon la revendication 1, dans lequel la chaîne glucidique est une chaîne glucidique de type riche en mannose.

3. Procédé de production d'une β-glucosidase mutante selon la revendication 1 ou 2, dans lequel la β-glucosidase dérivée d'un thermophile est une β-glucosidase dérivée d'un thermophile choisi dans le groupe constitué du genre Sulfolobus, du genre Thermoplasma, du genre Caldivirga, du genre Thermosphaera, du genre Pyrococcus, du genre Picrophilus, et du genre Fervidobacterium.

4. Procédé de production d'une β-glucosidase mutante selon l'une quelconque des revendications 1 à 3, dans lequel la β-glucosidase dérivée d'un thermophile est une protéine comprenant :

   (i) une même séquence d'acides aminés que l'une quelconque des séquences d'acides aminés illustrées par SEQ ID NO : 4, SEQ ID NO : 8, SEQ ID NO : 10, SEQ ID NO : 12, SEQ ID NO : 14, SEQ ID NO : 16, SEQ ID NO : 18 et SEQ ID NO : 20, ou
   (ii) une séquence d'acides aminés présentant 85 % d'identité ou plus avec l'une quelconque des séquences d'acides aminés illustrées par SEQ ID NO : 4, SEQ ID NO : 8, SEQ ID NO : 10, SEQ ID NO : 12, SEQ ID NO : 14, SEQ ID NO : 16, SEQ ID NO : 18 et SEQ ID NO : 20, et également une activité β-glucosidase.

5. Procédé de production d'une β-glucosidase mutante selon l'une quelconque des revendications 1 à 4, dans lequel le microorganisme eucaryote est Pichia pastoris.

6. Procédé de production d'une β-glucosidase mutante selon l'une quelconque des revendications 1 à 5, dans lequel la séquence signal de sécrétion est une séquence signal de sécrétion du facteur α.

7. Procédé de production d'une β-glucosidase mutante selon l'une quelconque des revendications 1 à 6, dans lequel la β-glucosidase mutante dérivée d'un thermophile comprend une séquence d'acides aminés illustrée par l'une quelconque de SEQ ID NO : 6, SEQ ID NO : 38, SEQ ID NO : 40, SEQ ID NO : 42, SEQ ID NO : 44, SEQ ID NO : 46, SEQ ID NO : 48, SEQ ID NO : 50, SEQ ID NO : 52, SEQ ID NO : 54 et SEQ ID NO : 56.

8. Composition enzymatique pour la saccharification de biomasse comprenant une cellulase et la β-glucosidase mutante dérivée d'un thermophile obtenue par le procédé de production selon l'une quelconque des revendications 1 à 7.

9. Composition enzymatique pour la saccharification de biomasse selon la revendication 8, dans laquelle la cellulase est un mélange de cellulases dérivées de champignons filamenteux.

**10.** Composition enzymatique pour la saccharification de biomasse selon la revendication 8 ou 9, dans laquelle le mélange de cellulases dérivées de champignons filamenteux est un mélange de cellulases dérivées du genre Trichoderma.

**11.** Procédé d'hydrolyse de biomasse, comprenant l'utilisation de la composition enzymatique selon l'une quelconque des revendications 8 à 10.

**12.** Procédé d'hydrolyse de biomasse selon la revendication 11, comprenant la filtration d'un hydrolysat obtenu par la composition enzymatique à travers une membrane d'ultrafiltration, et la séparation et la récupération de la composition enzymatique utilisée.

# Fig. 1

```
TriReBGL    1 --MLPKDFQWGFATAAYQIEGAVDQDGRGPSIWDTFCAQPGKIADGSSGVTACDS---YN
AspNiBGL    1 --MLPKDLQWGFAKAAYQIEGAVDQDGRGPSIWDTFCAQPGKIADGSSGVTACDS---YN
PfuBGL      1 MAKFPKNFMFGYSWSGFQFEMGLPGSEVESDWWVWVHDKENIASGLVSGDLPENGPAYWH

TriReBGL   56 RTAEDIALLKSLGAKSYRFSISWSRIIPEGGRGDAVNQAG--------IDHYVKFVDDLL
AspNiBGL   56 RTAEDIALLKSLGAKSYRFSISSR--IPEGGRGDAVNQAG--------IDHYVKFVDDLL
PfuBGL     61 LYKQDHDIAEKLGMDCIRGGIEWARIFPKPTFDVKVDVEKDEEGNIISVDVPESTIKELE

TriReBGL  108 DAGITPFITLFHWDLPEGLHQRYGGLLNRTEFPLDFENYARVMFRALPKVRNWITFNEPL
AspNiBGL  106 DAGITPFITLFHWDL---LHQRYGGLLNRTEFPLDFENYARVMFRALPKVR---NWNEPL
PfuBGL    121 KIANMEALEHYRKIYSDWKERGKTFILNLYHWPLPLWIHDPIAVRKLGPDRAPAGWLDEK

TriReBGL  168 CSAIPGYGSGTFAPGRQSTSEPWTVGHNILVAHGRAVKAYRDDFKPASGDGQIGIVLNGD
AspNiBGL  160 CSAIPGYGSGSFAPGRQSTSEPWTVGHNILVAHGRAVKAYRDDFKPASGDGQIGIVLNGD
PfuBGL    181 TVVEFVKFAAFVAYHLDDLVDMWSTMNEPNVVYNQGYINLRSGFPPGYLSFEAAEKAKFN

TriReBGL  228 FTYPWDAADPADKEAAERRLEFFTAWFADPIYLGDYPASMRKQLGDRLPTFTPEERALVH
AspNiBGL  220 FTYPWDAADPADKE----RLEFFTAWFADPIYLGDYPASMRKQLGDRLPTFTPEERALVH
PfuBGL    241 LIQAHIGAYDAIKE--------YSEKSVGVIYAFAWHDPLAEEYKDEVEEIRKKDYEFVT

TriReBGL  288 GSNDFYGMNHYTSNYIRhrSSPASADDTVGNVDVLFTNKQGNCIG---PETQSPWLRPCA
AspNiBGL  276 GSNDFYGMNHYTSNYIRhrSSPASADDTVGNVDVLFTNKQGNCIG---PETQSPWLRPCA
PfuBGL    293 ILHSKGKLDWIGVNYYSRLVYGAKDGHLVPLPGYGFMSERGGFAKSGRPASDFGWEMYPE

TriReBGL  345 AGFRDFLVWISKRYGYPPIYVTENGTSIKGESDLPKEKILEDDFRVKYYNEYIRAMVTAV
AspNiBGL  333 AGFRDFLVWTSKRYGSPPIYVTENGTSIKGESDLPNEKILEDDFRVKYYNEYIRAMVTAV
PfuBGL    353 G-LENLLKYLNNAYELP-MIITENG------------MADAADRYRPHYLVSHLKAVYNA

TriReBGL  405 ELDGVNVKGYFAWSLMDNFEWADGYVTRFGVTYVDYENGQKRFP------------KKSA
AspNiBGL  393 ELDGVNVR-------------------FGVTYVDYENGQKRFP------------KKSA
PfuBGL    399 MKEGADVRGYLHWSLTDNYEWAQGFRMRFGLVYVDFETKKRYLRPSALVFREIATQKEIP

TriReBGL  453 KSLKPLFDELIAAA-
AspNiBGL  421 KSLKPLFDELIAAA-
PfuBGL    459 EELAHLADLKFVTRK
```

# Fig. 2-1

```
gPfuBGL     1 --MAKFPKNFMFGYSWSGFQFEMG---LPGSE-VESDWWVWVHDKENIASGLVSGDLPEN
ThAggBGY    1 ---MKFPKDFMIGYSSSPFQFEAG---IPGSEDPNSDWWVWVHDPENTAAGLVSGDFPEN
CmGHFP      1 --MIKFPSDFRFGFSTVGTQHEMG---TPGSE-FVSDWYVWLHDPENIASGLVSGDLPEH
SaBGAL      1 --MLSFPKGFKFGWSQSGFQSEMG---TPGSEDPNSDWHVWVHDRENIVSQVVSGDLPEN
SsoBGAL     1 --MYSFPNSFRFGWSQAGFQSEMG---TPGSEDPNTDWYKWVHDPENMAAGLVSGDLPEN
TvBGAL      1 MVENNFPEDFKFGWSQSGFQSEMG---YDNAMDDKSDWYVWVHDKENIQSGLVSGDMPEN
PtBGAL      1 -----MLPKNFLLGFSLAGFQSEMG---ISD-PDSNSDWWLWVHDPVNIRTGLVSGDLPEN
FnGHFP      1 ---MMFPKDFLFGVSMSGFQFEMGNPQDAEEVDLNTDWYVWVRDIGNIVNGVVSGDLPEN

gPfuBGL    55 GPAYWNRTKQDHDIAEKLGMDCIRGGIEWARIFPKPTFDVKVDVEKDEEG-NIISVDVPE
ThAggBGY   55 GPGYWNLNQNDHDLAEKLGVNTIRVGVEWSRIFPKPTFNVKVPVERDENG-SIVHVDVDD
CmGHFP     55 GPGYWDLYKQDHSIARDLGLDAAWITIEWARVFPKPTFDVKVKVDEDDGG-NVVDVEVNE
SaBGAL     56 GPGYWGNYKRFHDEAEKIGLNAVRINVEWSRIFPRPLPKPEMQTGTDKENSPVISVDLNE
SsoBGAL    56 GPGYWGNYKTFHDNAQKMGLKIARLNVEWSRIFPNPLPRP---QNFDESKQDVTEVEINE
TvBGAL     58 GPGYWNNYKSFHEAAQNMGLKMARIGVEWSRLFPEPFPEKIMADAKNN-------SLEINN
PtBGAL     53 GIGYWDLYKKYNGLAVQTGMNAARLGVEWSRIFPKSTEEVKVMEDYKDD--DLISVDVNE
FnGHFP     58 GSWYWKQYGKVHQLAADFGMDVIRIGTEWSRIFPVSTQSVEY----------------GSP

gPfuBGL   114 STIKELEKIANMEALEHYRKIYSDWKERGKTFILNLYHWPLPLWIHDPIAVRKLGPDRAP
ThAggBGY  114 KAVERLDELANKEAVNHYVEMYKDWVERGRKLILNLYHWPLPLWLHNPIMVRRMGPDRAP
CmGHFP    114 SALEELRRLADLNAVNHYRGILSDWKERGGLLVINLYHWAMPTWLHDPIAVRKNGPDRAP
SaBGAL    116 SKLREMDNYANHEALSHYRQILEDLRNRGFHIVLNMYHWTLPIWLHDPIRVRR-GDFTGP
SsoBGAL   113 NELKRLDEYANKDALNHYREIFKDLKSRGLYFILNMYHWPLPLWLHDPIRVRR-GDFTGP
TvBGAL    112 NILSELDKYVNKDALNHYIEIFNDIKNRNIDLIINMYHWPLPVWLSDPVSVRK-GIKTER
PtBGAL    111 GSLEKLDRLANQKAINRYMEIFNNIKENNMTLIVNVYHWPIPIYLHDPIEARNSGLSNKR
FnGHFP    103 DMLEKLDKLANQKAVSHYRKIMEDIKAKGLKLFVNLYHFTLPIWLHDPIAVHK-GEKTDK

gPfuBGL   174 AGWLDEKTVVEFVKFAAFVAYHLDDLVDMWSTMNEPNVVYNQGYINLRSGFPPGYLSFEA
ThAggBGY  174 SGWLNEESVVEFAKYAAYIAWKMGELPVMWSTMNEPNVVYEQGYMFVKGGFPPGYLSLEA
CmGHFP    174 SGWLDKRSVIEFTKFAAFIAHELGDLADMWYTMNEPGVVITEGYLYVKSGFPPGYLDLNS
SaBGAL    175 TGWLNSRTVYEFARFSAYVAWKLDDLASEYATMNEPNVVWGAGYAFPRAGFPPNYLSFRL
SsoBGAL   172 SGWLSTRTVYEFARFSAYIAWKFDDLVDEYSTMNEPNVVGGLGYVGVKSGFPPGYLSFEL
TvBGAL    171 SGWLNDRIVQLFALFSSYIVYKMEDLAVAFSTMNEPNVVYGNGFINIKSGFPPSYLSSEF
PtBGAL    171 NGWLNHKTVVEFVKYAKYLAWKFSDVADMFSIMNEPNVVFGNGYFNVKSGFPPAFPSVHG
FnGHFP    162 IGWISDATPIEFAKYAEYMAWKFADIVDMWASMNEPHVVSQLGYFAINAGFPPSYFNPSW

gPfuBGL   234 AEKAKFNLIQAHIGAYDAIKEYSEKSVGVIYAFAWHDPLAE--EYKDEVEEIRKKDYEFV
ThAggBGY  234 ADKARRNMIQAHARAYDNIKRFSKKPVGLIYAFQWFELLEG--PAEVFDKFKSSKLYYFT
CmGHFP    234 LATAGKHLIEAHARAYDAIKAYSRKPVGLVYSFADYQPLRQ--GDEEAVKEAKGLDYSFF
SaBGAL    235 SEIAKWNIIQAHARAYDAIKSVSKKSVGIIYANTSYYPLRP-QDNEAVEIAERLNRWSFF
SsoBGAL   232 SRRAMYNIIQAHARAYDGIKSVSKKPVGIIYANSSFQPLTD-KDMEAVEMAENDNRWWFF
TvBGAL    231 ASKVKNNILKAHSLAYDSMKKITDKPVGIIYANTYFTPLDPEKDNDAIAKADSDAKWSFF
PtBGAL    231 GLLAKKHEIEAIARSYDAMKEITKKPVGLIMANSDVQPLTD-EDKEAAEMATYNDRYSFI
FnGHFP    222 YIKSLENEAKAHNLSYDAIKKYTNNPVGVIYSFTWYDTVNK-DDKESFENAMDLTNWRFI
```

# Fig. 2-2

```
gPfuBGL   292 TILHS--------------------------KGKLDWIGVNYYSRLVYGAKDGH------
ThAggBGY  292 DIVSKGSSIINVEY-------------RRDLANRLDWLGVNYYSRLVYKIVDDK------
CmGHFP    292 DAPIKGELMG--VT-------------RDDLKGRLDWIGVNYYTRAVLRRRQDAGRA---
SaBGAL    294 DSIIKGEITSEGQ------------NVREDLRNRLDWIGVNYYTRTVVTKAESG------
SsoBGAL   291 DAIIRGEITRGNEK-----------IVRDDLKGRLDWIGVNYYTRTVVKRTEKG------
TvBGAL    291 DPLIKGDKSLGIN------------------GNKLDWIGINYYTRTMLRKDGDG------
PtBGAL    290 DPLRVGEMKWADEVTAGNPIGEKSNIDRSDLKNKLDWIGVNYYTRAVVKKSGNG------
FnGHFP    281 DMVKD----------------------------KTDYIGVNYYTRAVIDRLPTTIDFGEF

gPfuBGL   320 ---LVPLPGYGFMSERGGFAKSGRPASDFGWEMYPEGLENLLKYLNNAYELPMIITENGM
ThAggBGY  333 ---PIILHGYGFLCTPGGISPAENPCSDFGWEVYPEGLYLLLKELYNRYGVDLIVTENGV
CmGHFP    334 --SVAVVDGFGYSCEPGGVSNDRRPCSDFGWEIYPEGVYNVLMDLWRRYRMPMYITENGI
SaBGAL    336 ---YLTLPGYGDRCERNSLSLANLPTSDFGWEFFPEGLYDVLLKYWNRYGLPLYVMENGI
SsoBGAL   334 ---YVSLGGYGHGCERNSVSLAGLPTSDFGWEFFPEGLYDVLTKYWNRYHLYMYVTENGI
TvBGAL    327 ---YISLKGYGHSGSPNTVTNDKRPTSDIGWEFYPEGLEYVIMNYWNRYKLPMYVTENGI
PtBGAL    344 ---YTTLKGYGHSATAGMPSRAGRDVSDFGWEFYPEGLVNVLSSYWKRYHIPMIVTENGV
FnGHFP    313 KMNWYTLRGYGYSCEEGGFSLSGRPASEFGWEIYPEGLYNILIHVYNRYKKDIYVTENGI

gPfuBGL   377 ADAADRYRPHYLVSHLKAVYNAMKEGADVRGYLHWSLTDNYEWAQGFRMRFGLVYVDFET
ThAggBGY  390 SDSRDALRPAYLVSHVYSVWKAANEGIPVKGYLHWSLTDNYEWAQGFRQKFGLVMVDFKT
CmGHFP    392 ADEHDKWRSWFIVSHLYQIhrAMEEGVDVRGYFHWNLIDNLEWAAGYRMRFGLVYVDYAT
SaBGAL    393 ADDADYQRPYYLVSHIYQVhrALNEGVDVRGYLHWSLADNYEWSSGFSMRFGLLKVDYLT
SsoBGAL   391 ADDADYQRPYYLVSHVYQVhrAINSGADVRGYLHWSLADNYEWASGFSMRFGLLKVDYNT
TvBGAL    384 ADNGDYQRPYYLVSHIASVLRAINKGANVKGYLHWSLVDNYEWALGFSPKFGLIGYDENK
PtBGAL    401 ADSIDRLRPRYLVSHIKSVEKALSMGMDIRGYLHWSLIDNYEWASGFSMKFGLYGIDLNN
FnGHFP    373 ADSKDKYRSLFIISHLYAIEKALNEGIPIKGYLHWSIIDNFEWAKGYSKRFGLAYTDLST

gPfuBGL   437 KKRYLRPSALVFREIATQKEIPEELAHLADLKFVTRK--
ThAggBGY  450 KKRYLRPSALVFREIATHNGIPDELQHLTLIQ-------
CmGHFP    452 KRRYFRPSALVMREVAKQKAIPDYLEHYIKPPRIE----
SaBGAL    453 KRLYWRPSALVYREITRSNGIPEELEHLNRVPPIKPLRH
SsoBGAL   451 KRLYWRPSALVYREIATNGAITDEIEHLNSVPPVKPLRH
TvBGAL    444 K-LYWRPSALVYKEIATKNCISPELKHLDSIPPINGLRK
PtBGAL    461 KKIQhrPSALVFKEIANANGVPEEFEWMADQHQNS----
FnGHFP    433 KKYIPRPSMYIFREIIKDKSIDKFKGYDPYNLMKF----
```

Fig. 3

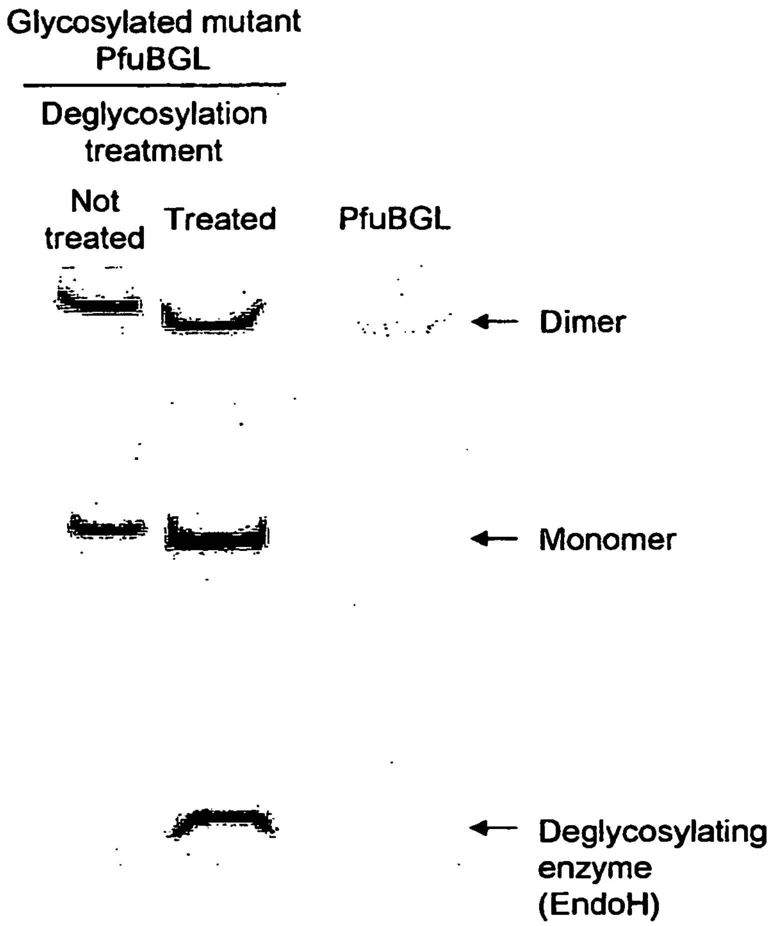
Glycosylated mutant PfuBGL
Deglycosylation treatment
Not treated
Treated
PfuBGL
Dimer
Monomer
Deglycosylating enzyme (EndoH)

Fig. 4

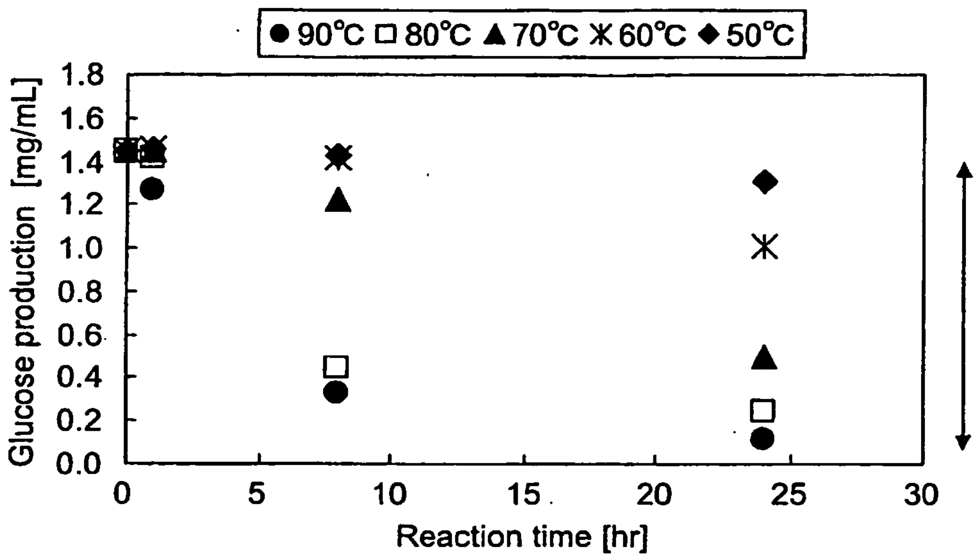
● 90°C □ 80°C ▲ 70°C ✳ 60°C ◆ 50°C
Glucose production [mg/mL]
1.8
1.6
1.4
1.2
1.0
0.8
0.6
0.4
0.2
0.0
0
5
10
15
20
25
30
Reaction time [hr]

Fig. 5

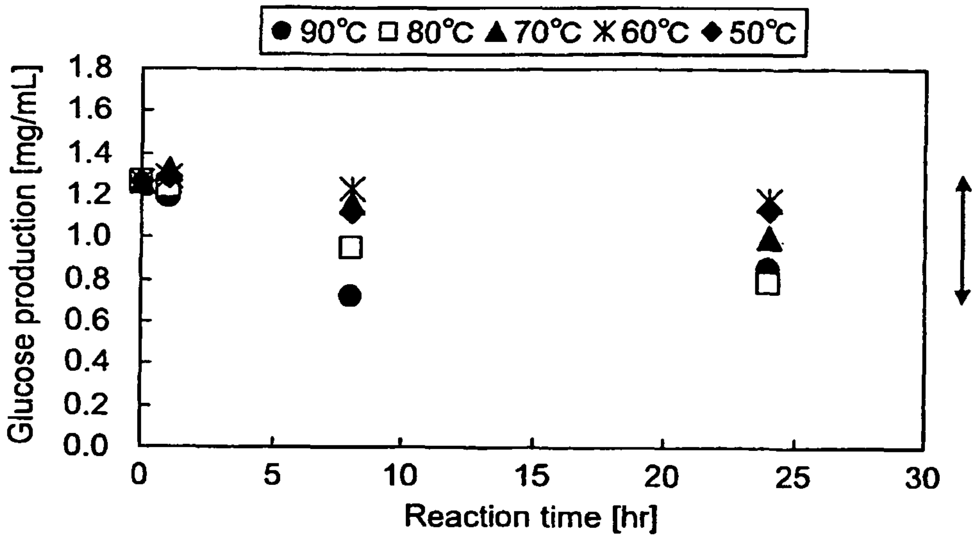
● 90°C □ 80°C ▲ 70°C ✳ 60°C ◆ 50°C
Glucose production [mg/mL]
1.8
1.6
1.4
1.2
1.0
0.8
0.6
0.4
0.2
0.0
0
5
10
15
20
25
30
Reaction time [hr]

Fig. 6

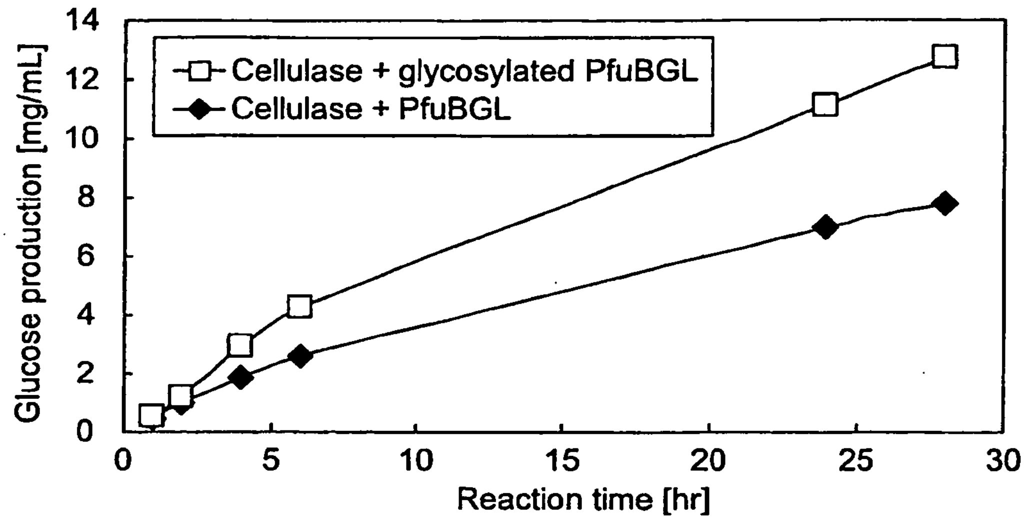
Glucose production [mg/mL]
14
12
10
8
6
4
2
0
0
5
10
15
20
25
30
Reaction time [hr]
Cellulase + glycosylated PfuBGL
Cellulase + PfuBGL

Fig. 7

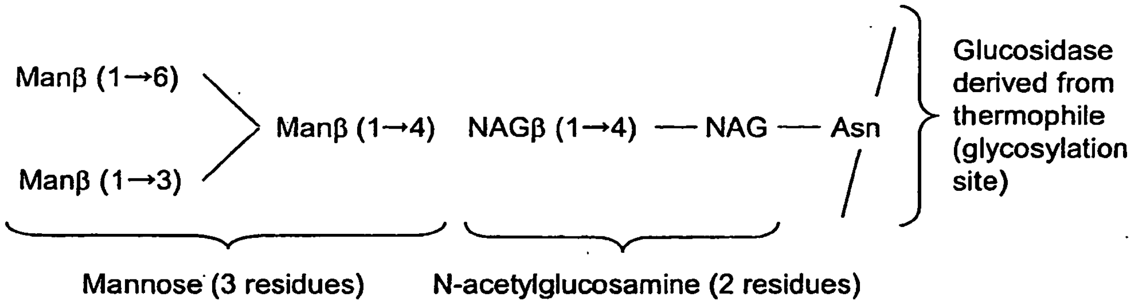
Manβ (1→6)
Manβ (1→3)
Manβ (1→4)
NAGβ (1→4)
NAG
Asn
Glucosidase derived from thermophile (glycosylation site)
Mannose (3 residues)
N-acetylglucosamine (2 residues)

Fig. 8

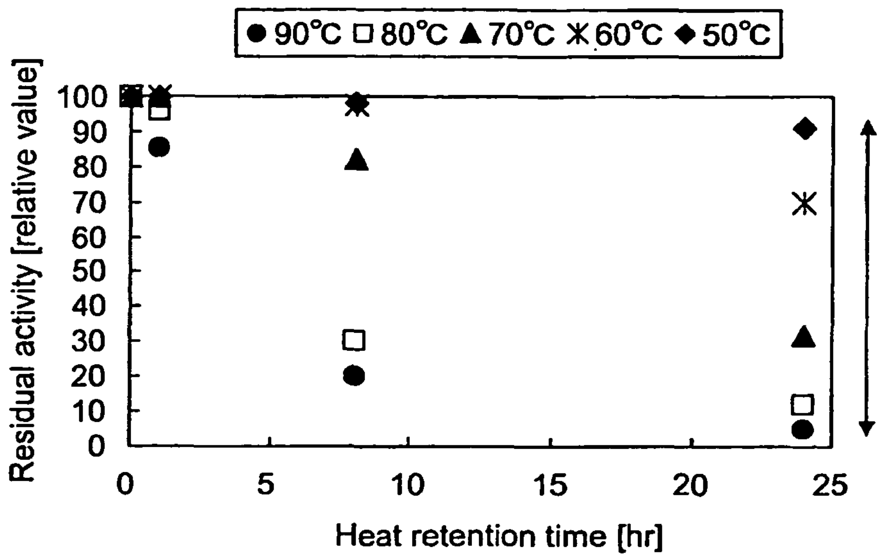
● 90°C □ 80°C ▲ 70°C ✳ 60°C ◆ 50°C
Residual activity [relative value]
100
90
80
70
60
50
40
30
20
10
0
0
5
10
15
20
25
Heat retention time [hr]

Fig. 9

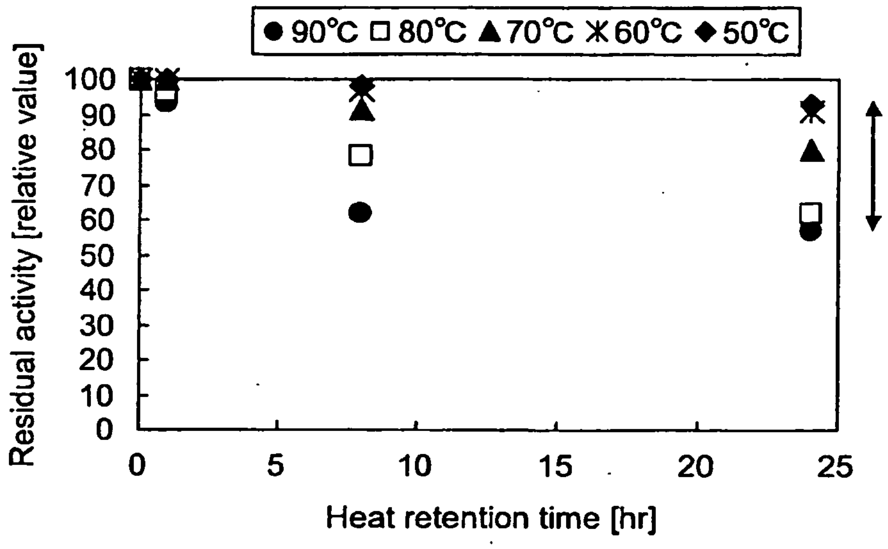
● 90℃ □ 80℃ ▲ 70℃ ✳ 60℃ ◆ 50℃
Residual activity [relative value]
100
90
80
70
60
50
40
30
20
10
0
0
5
10
15
20
25
Heat retention time [hr]

Fig. 10

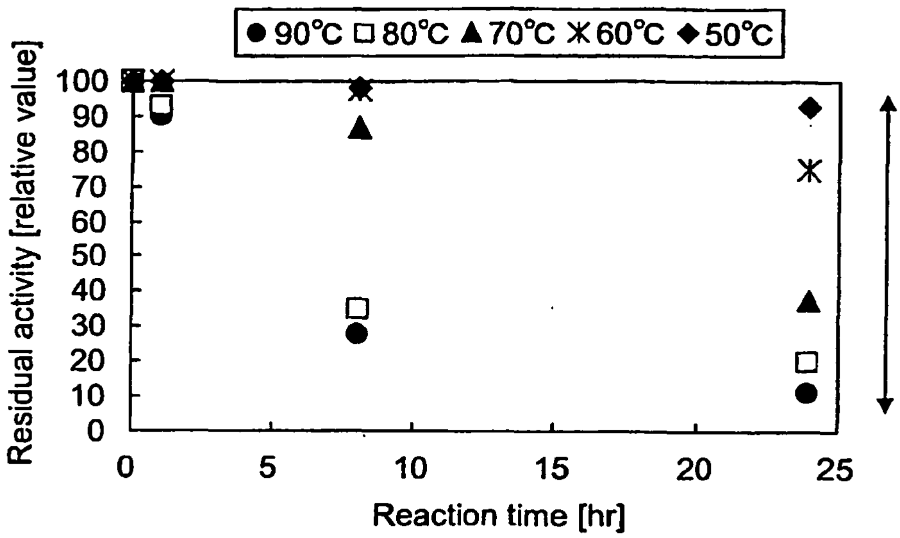
● 90°C □ 80°C ▲ 70°C ✳ 60°C ◆ 50°C
Residual activity [relative value]
100
90
80
70
60
50
40
30
20
10
0
0
5
10
15
20
25
Reaction time [hr]

Fig. 11

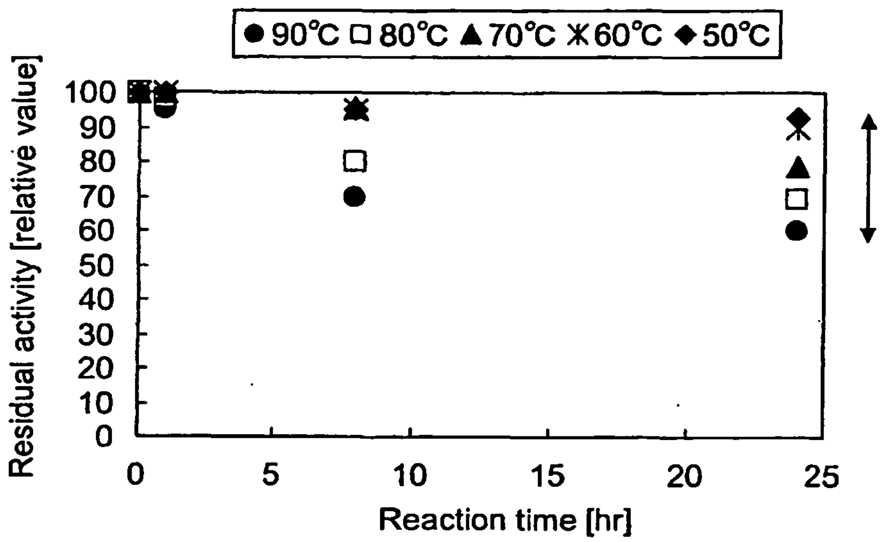
● 90℃ □ 80℃ ▲ 70℃ ✻ 60℃ ◆ 50℃
Residual activity [relative value]
100
90
80
70
60
50
40
30
20
10
0
0
5
10
15
20
25
Reaction time [hr]

Fig. 12

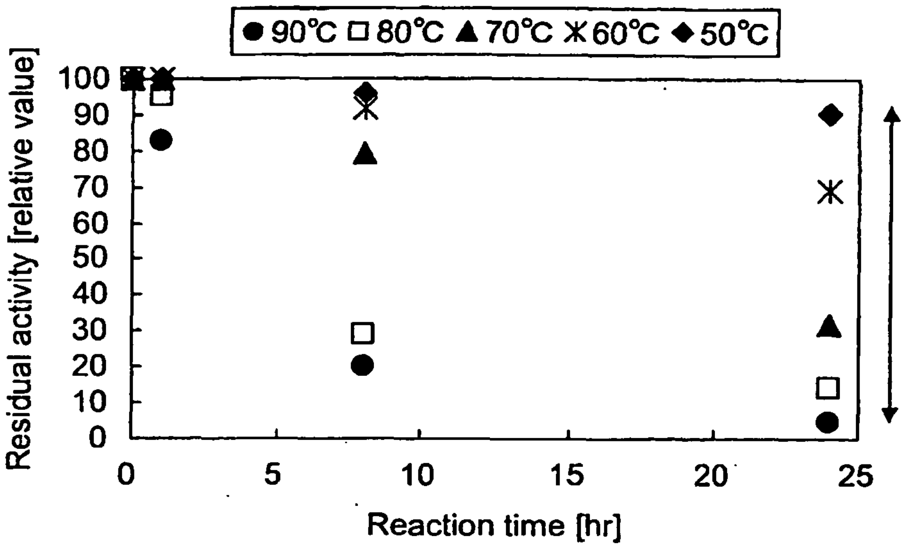
90°C 80°C 70°C 60°C 50°C
Residual activity [relative value]
100
90
80
70
60
50
40
30
20
10
0
0
5
10
15
20
25
Reaction time [hr]

Fig. 13

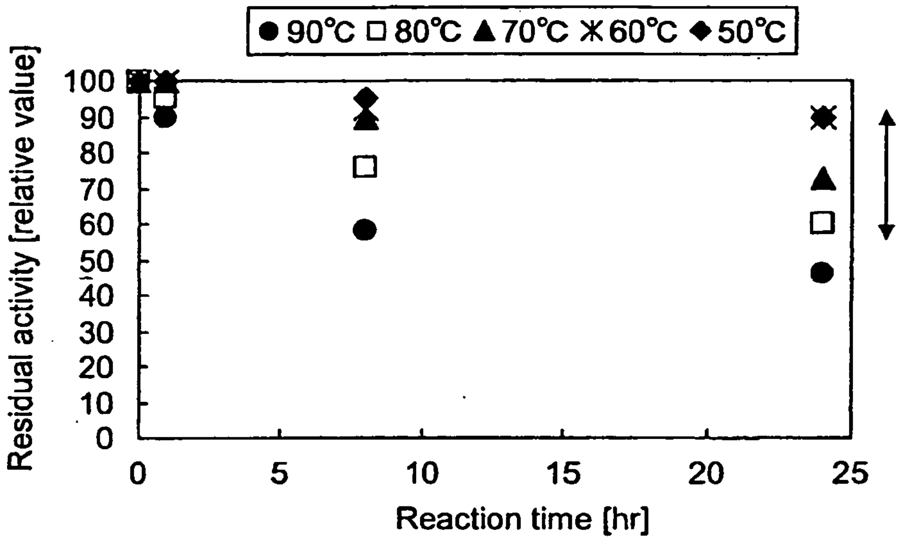
● 90℃ □ 80℃ ▲ 70℃ ✳ 60℃ ◆ 50℃
Residual activity [relative value]
100
90
80
70
60
50
40
30
20
10
0
0
5
10
15
20
25
Reaction time [hr]

Fig. 14

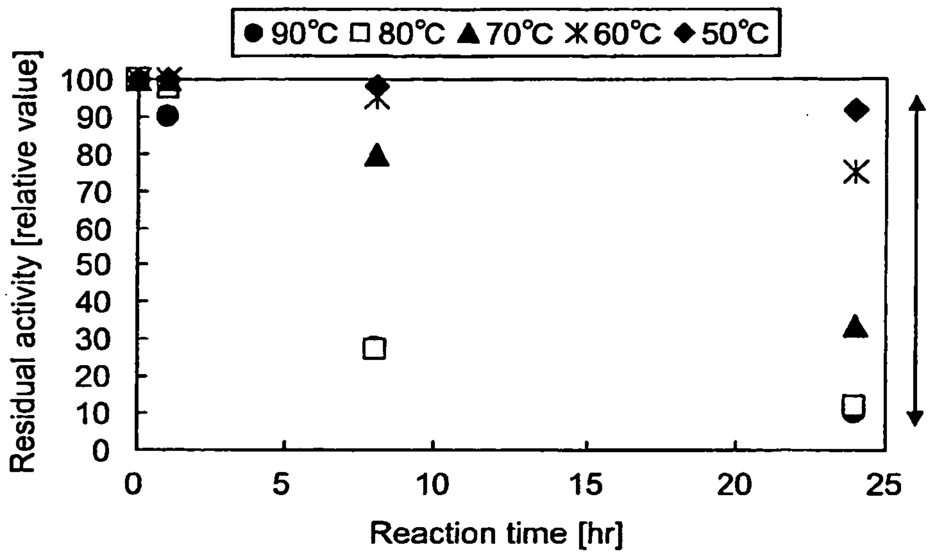
● 90℃ □ 80℃ ▲ 70℃ ✱ 60℃ ◆ 50℃
Residual activity [relative value]
100
90
80
70
60
50
40
30
20
10
0
0
5
10
15
20
25
Reaction time [hr]

Fig. 15

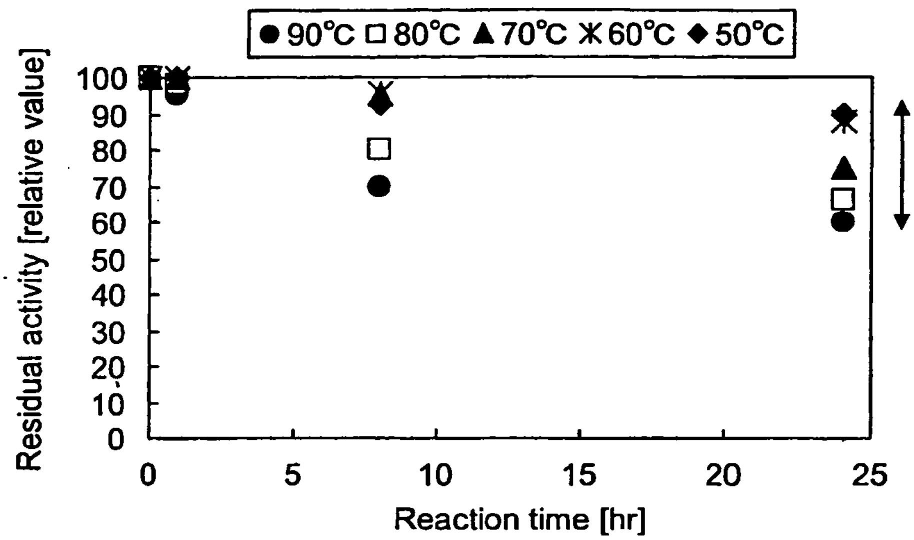
● 90℃ □ 80℃ ▲ 70℃ ✱ 60℃ ◆ 50℃
Residual activity [relative value]
100
90
80
70
60
50
40
30
20
10
0
0
5
10
15
20
25
Reaction time [hr]

Fig. 16

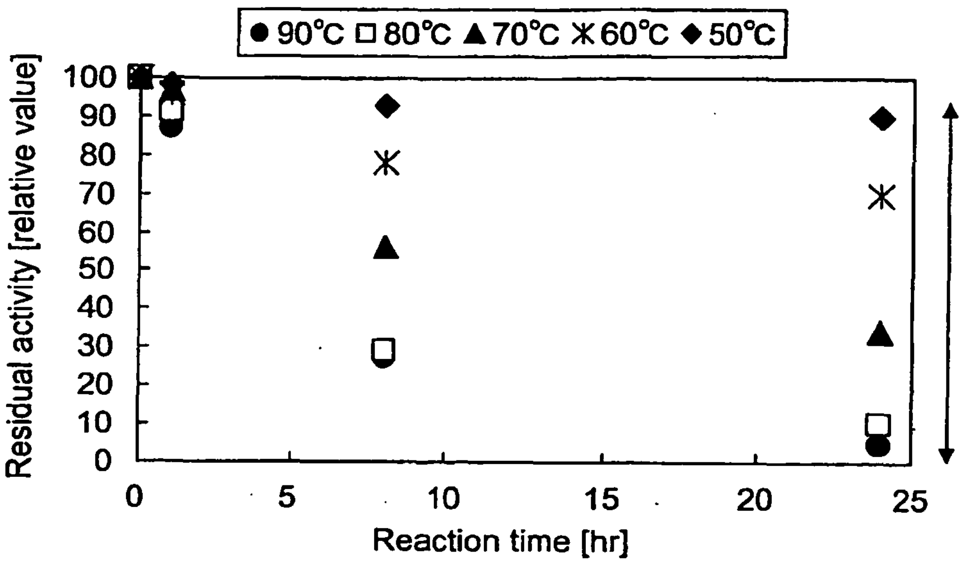
● 90℃ □ 80℃ ▲ 70℃ ✳ 60℃ ◆ 50℃
Residual activity [relative value]
100
90
80
70
60
50
40
30
20
10
0
0
5
10
15
20
25
Reaction time [hr]

Fig. 17

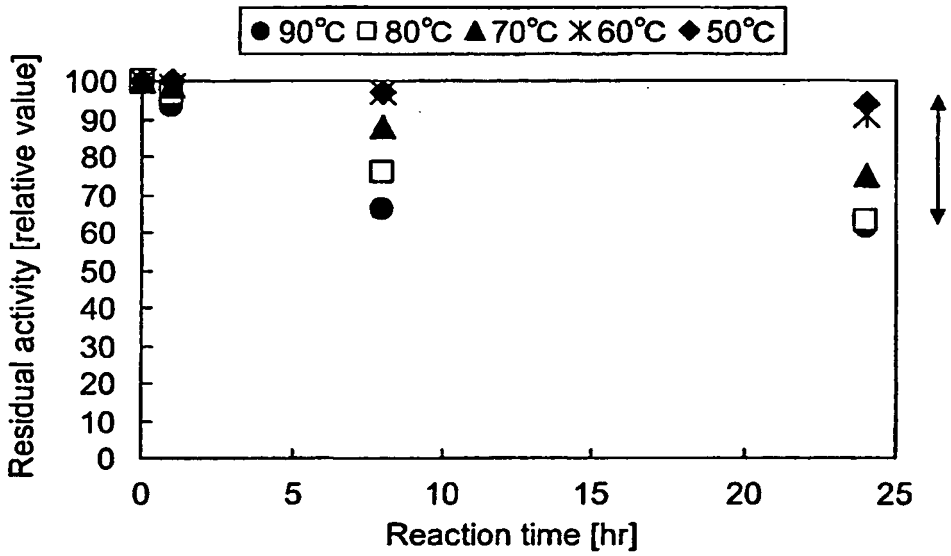
● 90°C □ 80°C ▲ 70°C ✕ 60°C ◆ 50°C
Residual activity [relative value]
100
90
80
70
60
50
40
30
20
10
0
0
5
10
15
20
25
Reaction time [hr]

Fig. 18

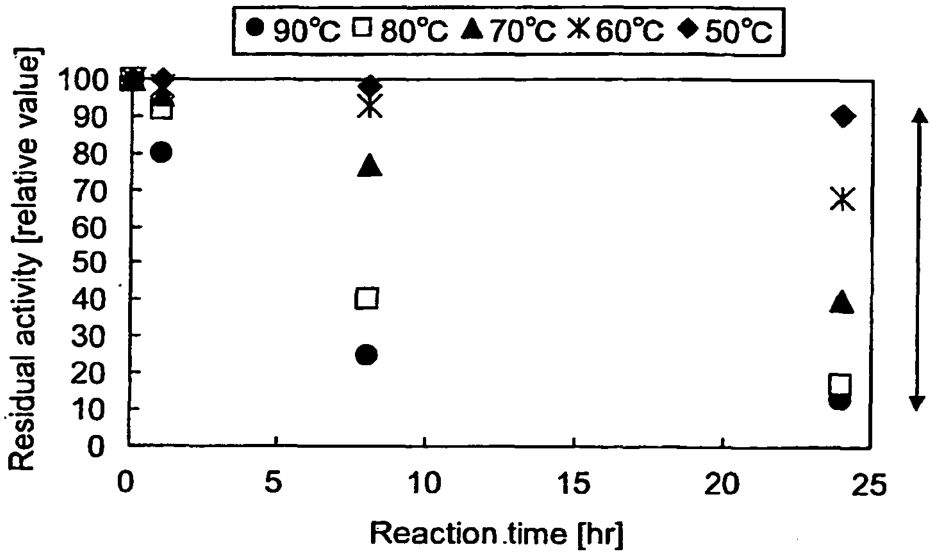
● 90℃ □ 80℃ ▲ 70℃ ✳ 60℃ ◆ 50℃
Residual activity [relative value]
100
90
80
70
60
50
40
30
20
10
0
0
5
10
15
20
25
Reaction time [hr]

Fig. 19

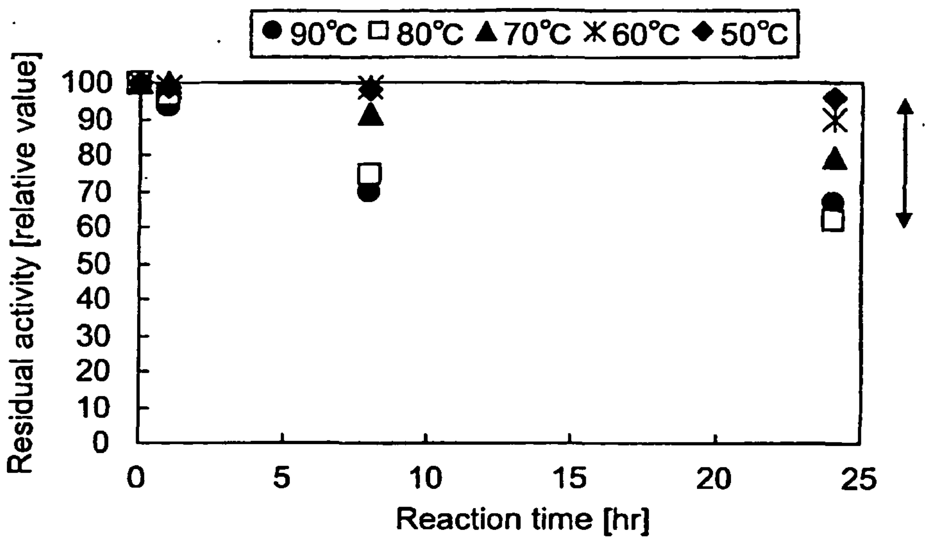
● 90°C □ 80°C ▲ 70°C ✳ 60°C ◆ 50°C
Residual activity [relative value]
100
90
80
70
60
50
40
30
20
10
0
0
5
10
15
20
25
Reaction time [hr]

Fig. 20

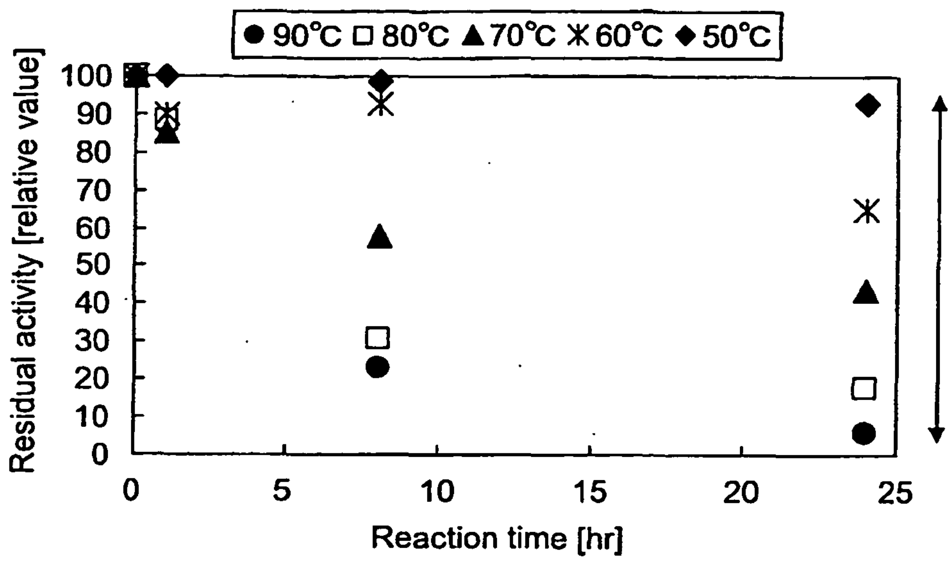
● 90°C □ 80°C ▲ 70°C ✳ 60°C ◆ 50°C
Residual activity [relative value]
100
90
80
70
60
50
40
30
20
10
0
0
5
10
15
20
25
Reaction time [hr]

Fig. 21

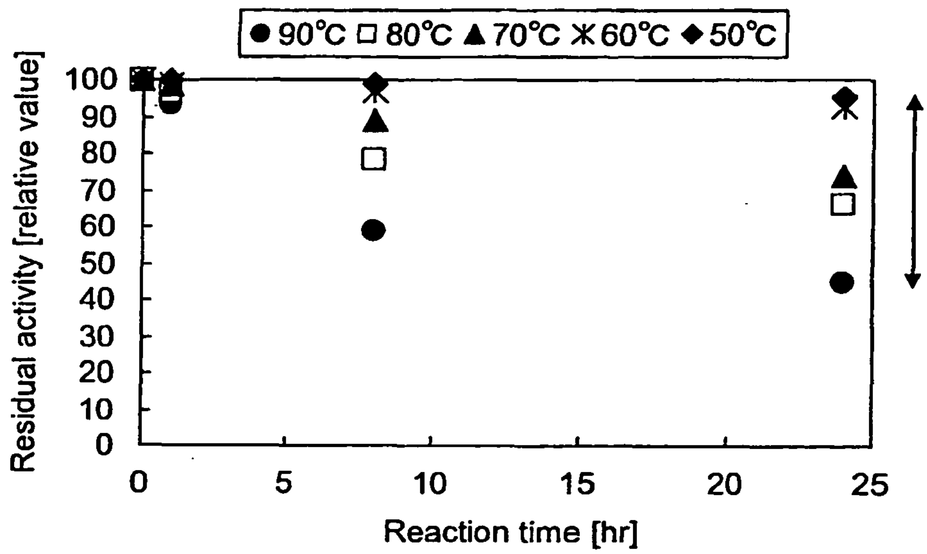
● 90°C □ 80°C ▲ 70°C ✳ 60°C ◆ 50°C
Residual activity [relative value]
100
90
80
70
60
50
40
30
20
10
0
0
5
10
15
20
25
Reaction time [hr]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005093072 A
- JP 2010044242 A
- JP 2008161125 A
- JP 2008535664 A


### Non-patent literature cited in the description


- **HETTI P et al.** *Journal of Biotechnology,* 2002, vol. 107, 65-72
- **CHRISTIAN P et al.** Trichoderma and Gliocladium: Basic Biology. *Taxonomy and Genetics.,* 1998, vol. 1, 121-138
- **H. OHBA et al.** *Biosci. Biotech. Biochem.,* 1995, vol. 59, 1581-1583
- **SAMBROOK et al.** Molecular Cloning, A Laboratory Manual. Cold Spring Harbor Laboratory Press, 1989
- **THIJIS K et al.** *Biochem.,* 2000, vol. 39 (17